(19) Europäisches Patentamt
European Patent Office
Office européen des brevets

(11) Publication number: 0 281 118 B1

(12) EUROPEAN PATENT SPECIFICATION

(45) Date of publication of patent specification: 10.04.91 Bulletin 91/15

(51) Int. Cl.$^5$: G03C 7/26, G03C 7/32, // C07D257/04

(21) Application number: 88103208.0

(22) Date of filing: 02.03.88

(54) **Silver halide photographic material.**

(30) Priority: 03.03.87 JP 48381/87

(43) Date of publication of application: 07.09.88 Bulletin 88/36

(45) Publication of the grant of the patent: 10.04.91 Bulletin 91/15

(84) Designated Contracting States: DE FR GB NL

(56) References cited:
EP-A- 0 204 175
PATENT ABSTRACTS OF JAPAN, vol. 11, no. 72 (P-554)[2519], 5th March 1987; & JP-A-61 233 741 (FUJI PHOTO FILM CO. LTD) 18.10.1986

(73) Proprietor: FUJI PHOTO FILM CO., LTD.
210 Nakanuma Minami Ashigara-shi
Kanagawa 250-01 (JP)

(72) Inventor: Ichijima, Seiji
c/o Fuji Photo Film Co., Ltd. No. 210, Nakanuma
Minami Ashigara-Shi Kanagawa (JP)
Inventor: Mihayashi, Keiji
c/o Fuji Photo Film Co., Ltd. No. 210, Nakanuma
Minami Ashigara-Shi Kanagawa (JP)

(74) Representative: Patentanwälte Grünecker, Kinkeldey, Stockmair & Partner
Maximilianstrasse 58
W-8000 München 22 (DE)

EP 0 281 118 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

The present invention relates to a silver halide photographic material which is excellent in image quality such as graininess, and more particularly, to a silver halide photographic material containing a compound capable of releasing a development inhibitor upon oxidation in a development processing step.

### BACKGROUND OF THE INVENTION

A series of compounds which are capable of releasing a photographically useful group via an oxidation reduction reaction have hitherto been known.

For instance, as hydroquinone derivatives capable of releasing a development inhibitor, compounds as described in U.S. Patents 3,379,529, 3,620,746, 4,144,071, 4,377,634, 4,332,878 and 3,930,863 are exemplified. Also, as catechol derivatives, compounds as described in British Patent 1,400,149 are known.

EP-A-204175 and JP-A-61-233741 both disclose silver halide photographic material comprising compounds which contain groups at the position of $R_3$ of formula (I) of the present invention. These groups are released upon the reaction with an oxidation product of a developing agent.

As described in the above-mentioned patents, these hitherto known compounds are employed for various purposes depending on the kind of photographic light-sensitive material in which they are present. Among others, in color photographic light-sensitive materials, hydroquinones capable of releasing a development inhibitor exhibit effective properties for the purpose of improving sharpness, graininess and color reproducibility. However, these known compounds are still insufficient to meet stringent requirements and to improve various kinds of photographic properties. Therefore, further improvents have been desired.

More specifically, the above described known compounds are apt to be oxidized and immediately release a development inhibitor at the time of development. However, they have the fatal defect that they are extremly unstable when incorporated into photographic light-sensitive materials and are readily oxidized by air during storage of the photographic light-sensitive materials.

### SUMMARY OF THE INVENTION

Therefore, an object of the present invention is to provide a photographic light-sensitive material excellent instability during storage, sharpness, graininess and color reproducibility.

Other objects of the present invention will become apparent from the following detailed description and examples.

These objects of the present invention can be accomplished by a silver halide photographic material comprising a support having thereon at least one silver halide emulsion layer, characterized in that the silver halide photographic material contains at least one compound represented by the following general formula (I) :

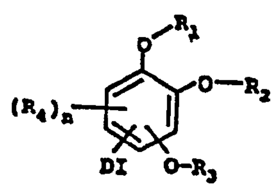

(I)

wherein $R_1$ and $R_2$ each represents a hydrogen atom or a group capable of being cleaved with an alkali ; $R_3$ represents an aromatic group or a heterocyclic group, the group represented by $R_3$ is unable to be released upon the reaction with an oxidation product of a developing agent ; $R_4$ represents a group capable of substituting on the benzene ring ; n represents an integer from 0 to 2 ; and DI when released represents a development inhibitor or a precursor thereof, when n represents 2, two $R_4$'s may be the same or different, an when any two substituents of $R_1$, $R_2$ and $R_4$ are present on the adjacent positions, they may be divalent groups and connected with each other to form a cyclic structure(e.g., 5- or 6-membered cyclic structure).

### DETAILED DESCRIPTION OF THE INVENTION

The compounds represented by general formula (I) are described in detail below.

In the following description, $R_5$ and $R_6$ each represents an aliphatic group, an aromatic group or a heterocyc-

lic group, and $R_7$ and $R_8$ each represents a hydrogen atom, an aliphatic group, an aromatic group or a heterocyclic group.

In general formula (I), $R_1$ and $R_2$ are preferably hydrogen atoms. When $R_1$ and $R_2$ each represents a group capable of being cleaved with alkali, representative examples thereof include the group $R_5$-CO – (e.g., an acetyl group or benzoyl group) and the group of $R_5$O-CO – (e.g., an ethoxycarbonyl group or a phenoxycarbonyl group).

When $R_1$ and $R_2$ each represents a divalent group to form a cyclic structure, representative examples are represented by the following general formula (Ia) or (Ib) :

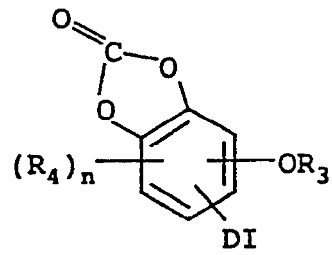

(Ia)

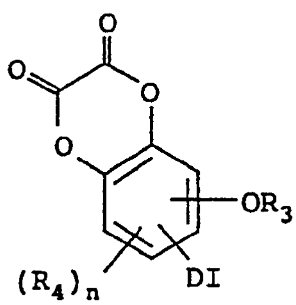

(Ib)

wherein $R_3$, $R_4$, n and DI each has the same meaning as defined in general formula (I).

In general formula (I), examples of the group represented by $R_4$ include the group $R_6$O-CO –, the group

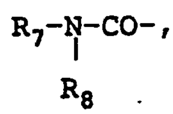

the group $R_6$ –, the group $R_7$-CO –, the group $R_6$-S –, a halogen atom, the group $R_6$-$SO_2$ –, the group

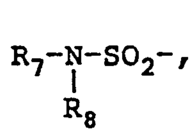

a nitro group, etc.

The aromatic group represented by $R_3$, $R_5$, $R_6$, $R_7$ or $R_8$ includes a substituted or unsubstituted aromatic group having from 6 to 20 carbon atoms and is preferably a substituted or unsubstituted phenyl group or a substituted or unsubstituted naphthyl group.

The aliphatic group represented by $R_5$, $R_5$, $R_7$ or $R_8$ includes a saturated or unsaturated, straight chain, branched chain or cyclic, substituted or unsubstituted aliphatic hydrocarbon group having from 1 to 40 carbon atom, preferably from 1 to 18 carbon atoms. Suitable examples of the aliphatic groups include a methyl group, an ethyl group, a propyl group, a butyl group, a tert-butyl group, a pentyl group, a tert-pentyl group, an octyl group, a cyclohexyl group, a dodecyl group, a hexadecyl group, an octadecyl group, etc.

The heterocyclic group represented by $R_3$, $R_5$, $R_6$, $R_7$ or $R_8$ includes a substituted or unsubstituted

heterocyclic group containing at least one (preferably 1 to 4) hetero atom selected from a nitrogen atom, an oxygen atom and a sulfur atom. A 3-membered to 8-membered hetero cyclic group having from 1 to 7 carbon atoms is preferred. Representative examples of the heterocyclic group include a 2-pyridyl group, a thiadiazolyl group, a furyl group, etc.

Representative examples of the substituents for the aliphatic group, aromatic group or heterocyclic group described above include a halogen atom (e.g., a fluorine atom, a chlorine atom), an aromatic group, an aliphatic oxycarbonyl group, a carbonamido group, a sulfonamido group, a carbamoyl group, an aromatic oxy group, an aliphatic oxy group, an imido group, a nitro group, an aliphatic thio group, a hydroxy group, an aromatic oxycarbonyl group, an amino group, an aliphatic group, a heterocyclic group, and a cyano group, etc. The number of carbon atoms included in each of these substituents is usually from 0 to 30.

Examples of the developement inhibitors represented by DI in the general formula (I) include a tetrazolylthio group, a thiazolylthio group, a benzothiadiazolylthio group, a benzoxazolylthio group, a benzotriazolyl group, an indazolyl group, a benzimidazolylthio group, a triazolylthio group, a thiadiazolylthio group, a triazolyl group substituted with a thioether group (for example, development inhibitors as described in U.S. Patent 4,579,816), an oxadiazolyl group, etc.

The development inhibitor may be substituted at an appropriate position. Suitable substituents are selected from the groups illustrated as the representative substituents for the above described aliphatic, aromatic or heterocyclic group. The total number of carbon atoms included in the substituents is preferably up to 15.

The development inhibitor released from the compound represented by the general formula (I) exhibits a development inhibiting function and a part thereof is introduced into a color developing solution. The development inhibitor introduced into the color developing solution may have the property that it decomposes or charges into a compound which does not have any photographic function. Suitable examples of such development inhibitors are described, for example, in U.S. Patent 4,477,563, Japanese Patent Application (OPI) Nos. 218644/85, 221750/85, 233650/85 and 11743/86, etc. (the term "OPI" as used herein refers to a "published unexamined application").

Specific examples of development inhibitors used in the present invention are set forth below, but the present invention should not be construed as being limited thereto.

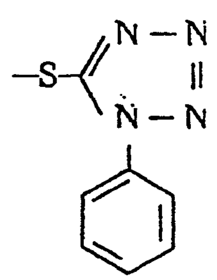
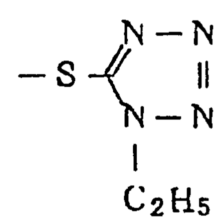
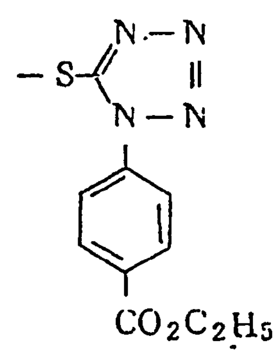

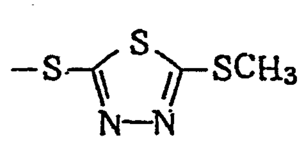
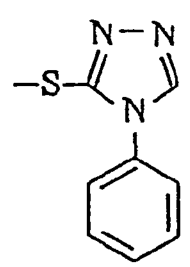
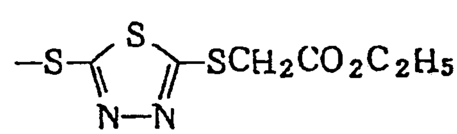

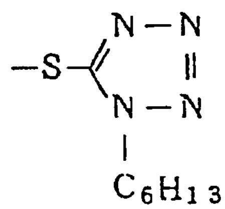
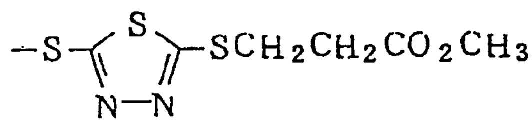

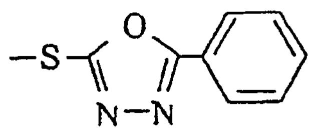
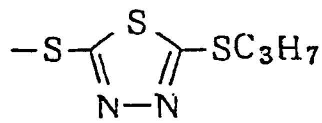
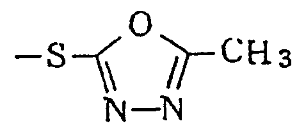

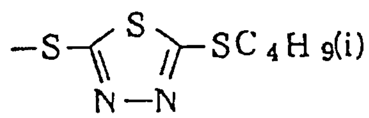
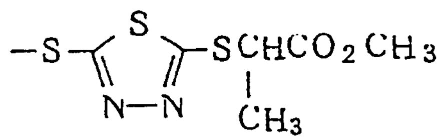

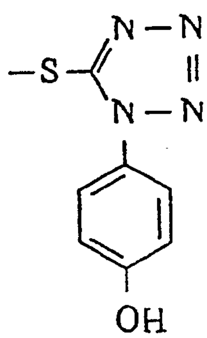
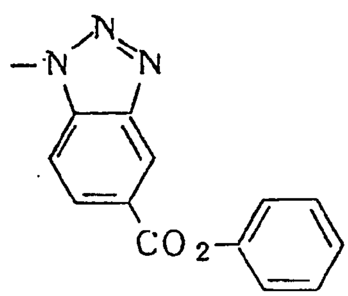
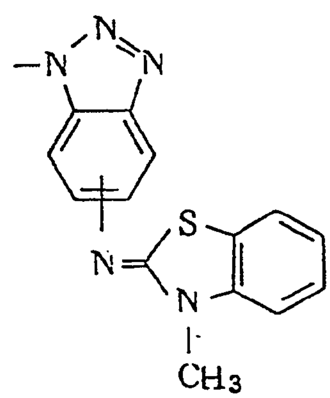

In general formula (I), the group represented by DI is preferably a development inhibitor. However, it may be a precursor of a development inhibitor. Examples of the precursor include a group represented by the following formula :

$$* - \text{TIME} - \text{DI}'$$

wherein the symbol * denotes the position at which the group is connected to the balance of the compound represented by general formula (I) ; TIME represents a group capable of releasing DI′ after being released as TIME-DI′ ; and DI′ represents a development inhibitor which is the same as the development inhibitor as defined for DI above.

Suitable examples of TIME include a group utilizing a cleavage reaction of hemiacetal as described in U.S. Patent 4,146,396, Japanese Patent Application (OPI) Nos. 249148/85, 249149/85 and 218645/85, etc., a group causing a cleavage reaction utilizing an intramolecular nucleophilic displacement reaction as described in U.S. Patent 4,248,962, etc., and a group causing a cleavage reaction utilizing electron transfer via a conjugated system as described in U.S. Patent 4,409,323, etc.

The effect of the compound(s) according to the present invention is particularly remarkably exhibited in the following embodiment.

The compound(s) represented by general formula (I) according to the present invention can be applied to a multilayer multi-color photographic material having at least two layers sensitive to different spectral wavelength ranges from each other on a support for the purpose mainly of improving graininess, sharpness

and/or color reproducibility. A multi-layer natural color photographic material ordinarily possesses at least one red-sensitive emulsion layer, at least one green-sensitive emulsion layer and at least one blue-sensitive emulsion layer on a support. The order of these layers can be appropriately selected, as desired. Further, the compound according to Claim 1 can be employed in any of a high-sensitive layer, a low-sensitive layer and a middle-sensitive layer. Moreover, the compound can be employed in a layer adjacent to a light-sensitive silver halide emulsion layer.

Of the compounds used in the present invention, particularly preferred are those represented by the following general formula (II) :

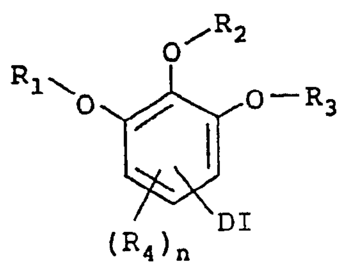

(II)

wherein $R_1$, $R_2$, $R_3$, $R_4$, n and DI each has the same meaning as earlier given.

The amount of the compound according to Claim 1 added may be varied depending on the structure and use of the compound. However, it is preferred to employ the compound(s) in a range from $1 \times 10^{-7}$ mol to 0.5 mol, particularly from $1 \times 10^{-6}$ mol to $1 \times 10^{-1}$ mol, per mol of silver present in the layer which contains the compound(s) or in a layer adjacent to the layer which contains the compound when the layer which contains the compound does not contain silver.

The compound according to Claim 1 can be employed individually or together with known couplers in a layer. In the case of using the compound according to Claim 1 together with other color image forming conplers, the ratio of them (the compound according to Claim 1/other color image forming coupler) is from 0.1/99.9 to 90/10, preferably from 1/99 to 50/50 by mol.

Specific examples of the compounds represented by the general formula (I) according to the present invention are set forth below, but the present invention should not be construed as being limited thereto.

(1)

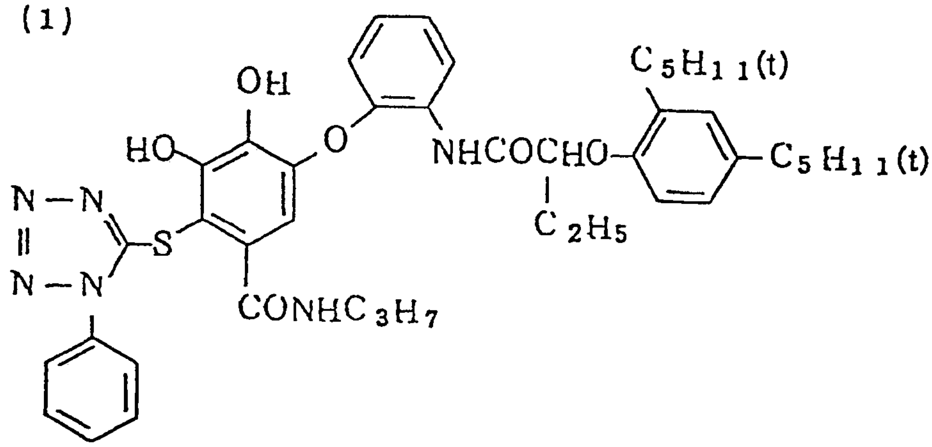

(2)

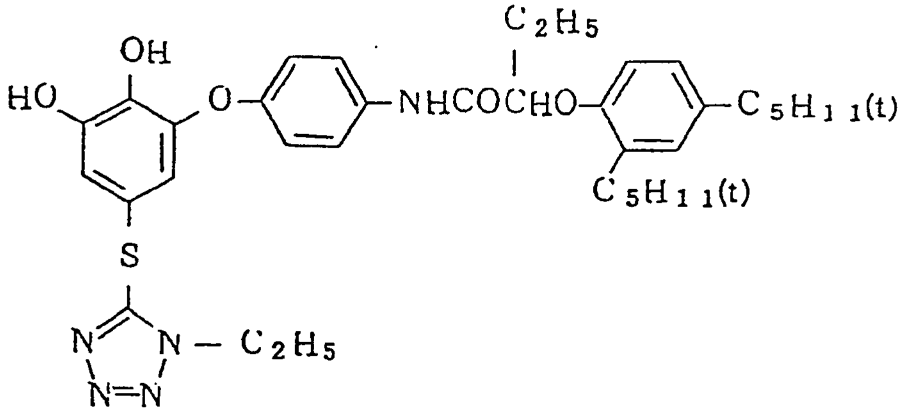

(3)

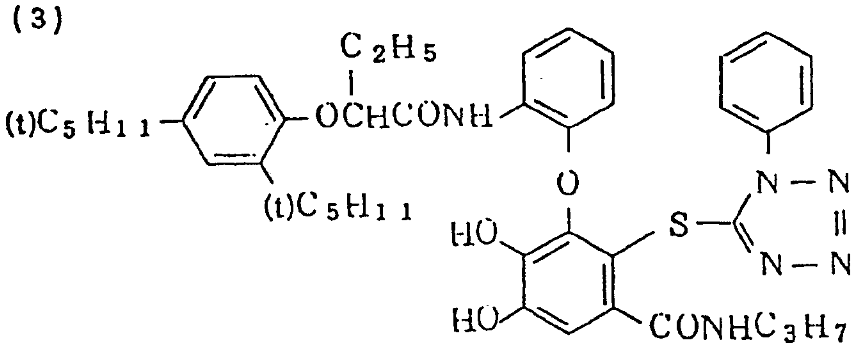

(4)

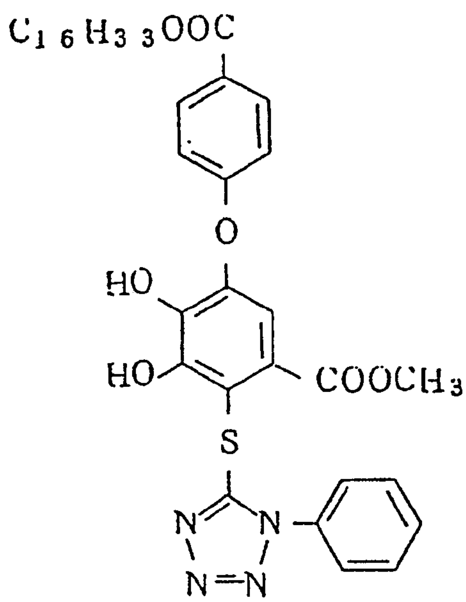

(5)

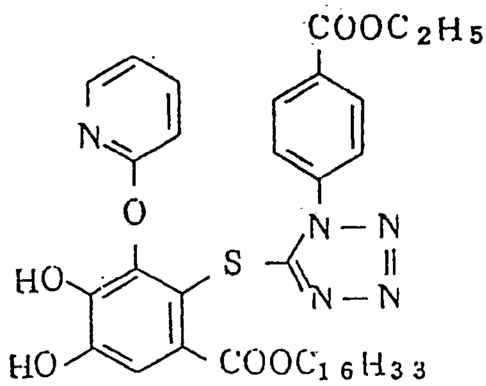

(6)

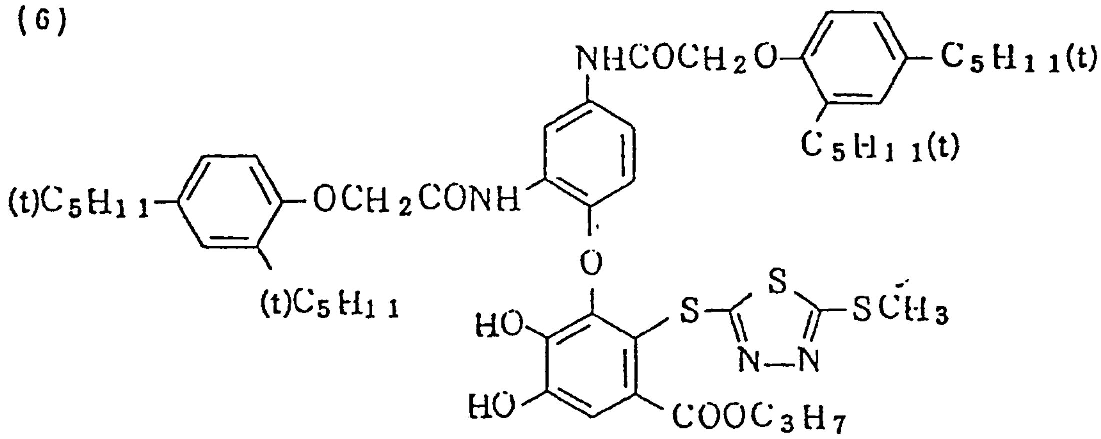

(7)

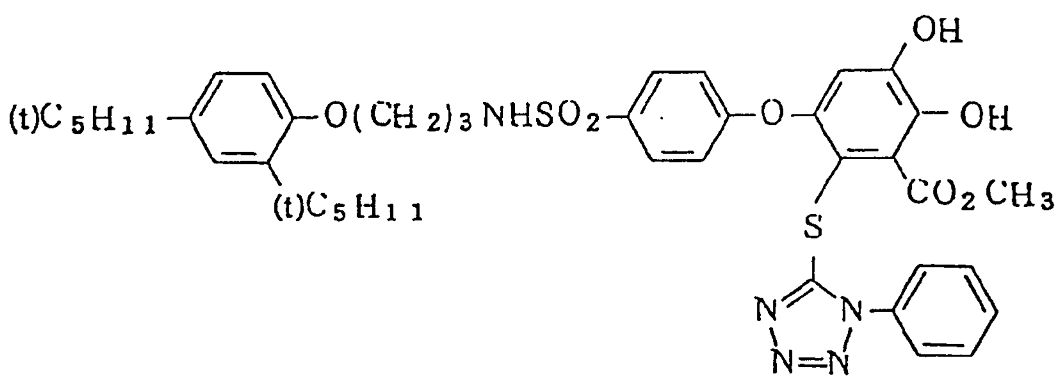

(8)

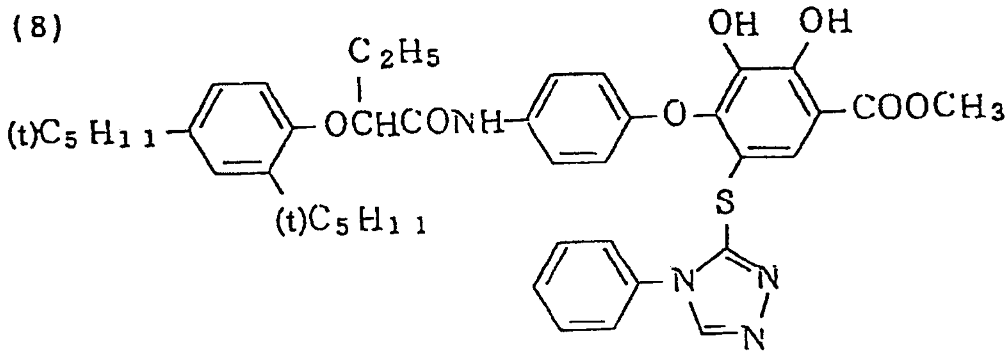

(9)

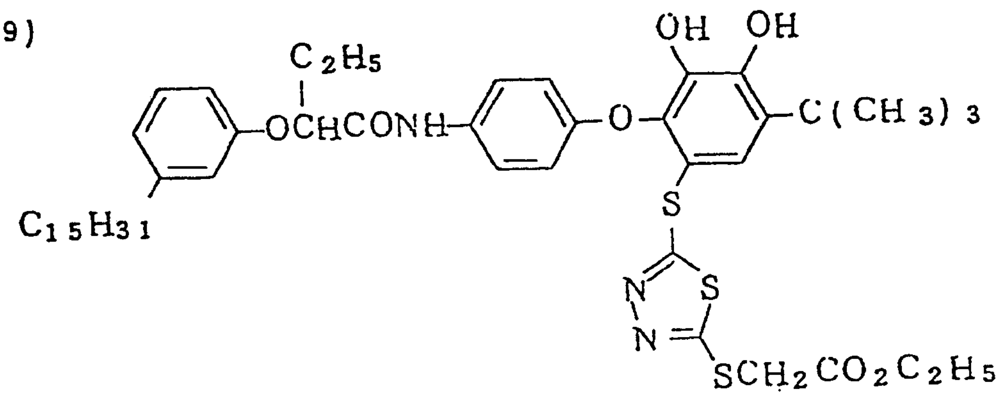

(10)

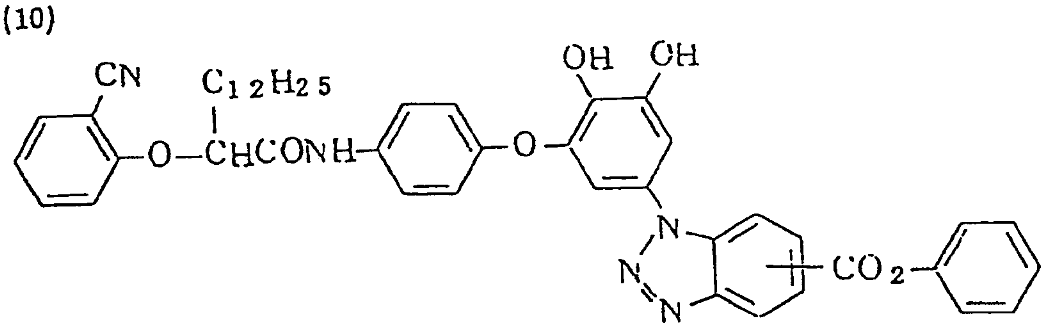

(The mixture of isomers containing 5-positioned and 6-positioned $-CO_2-$ 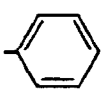 groups)

(11)

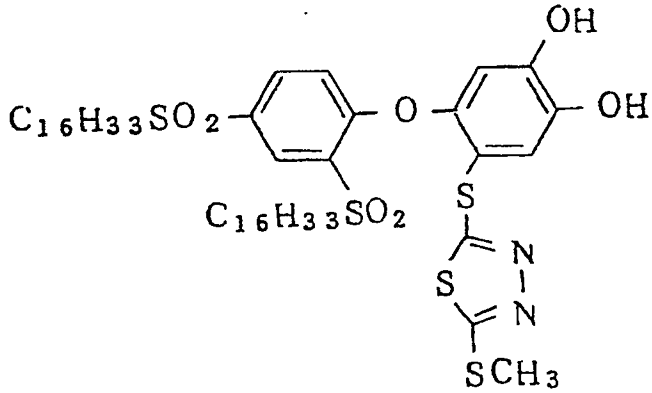

(12)

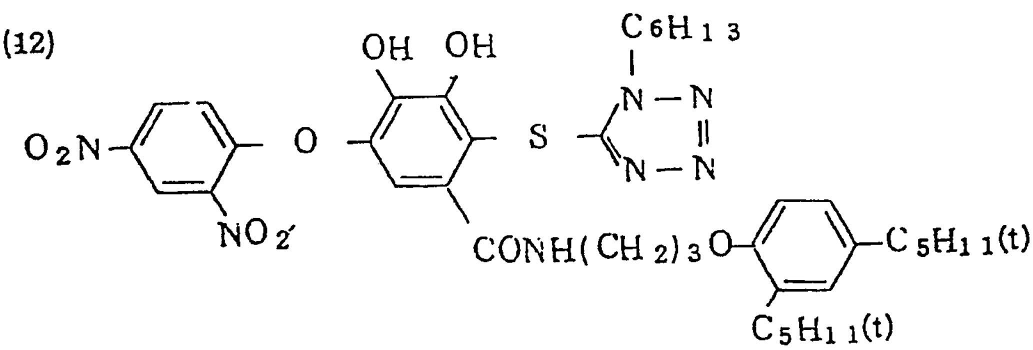

(13)

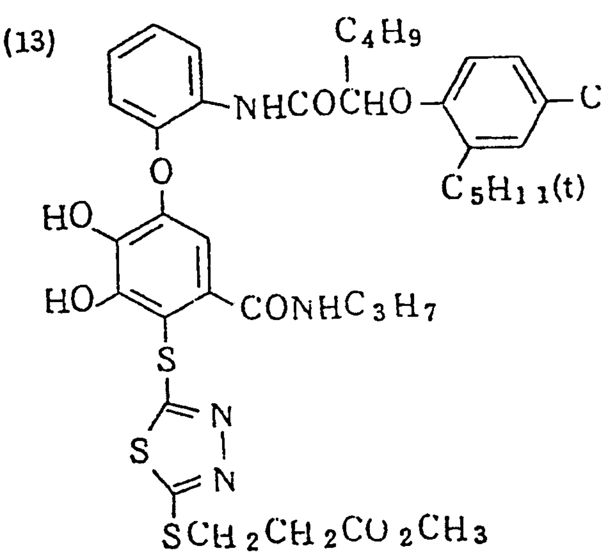

(14)

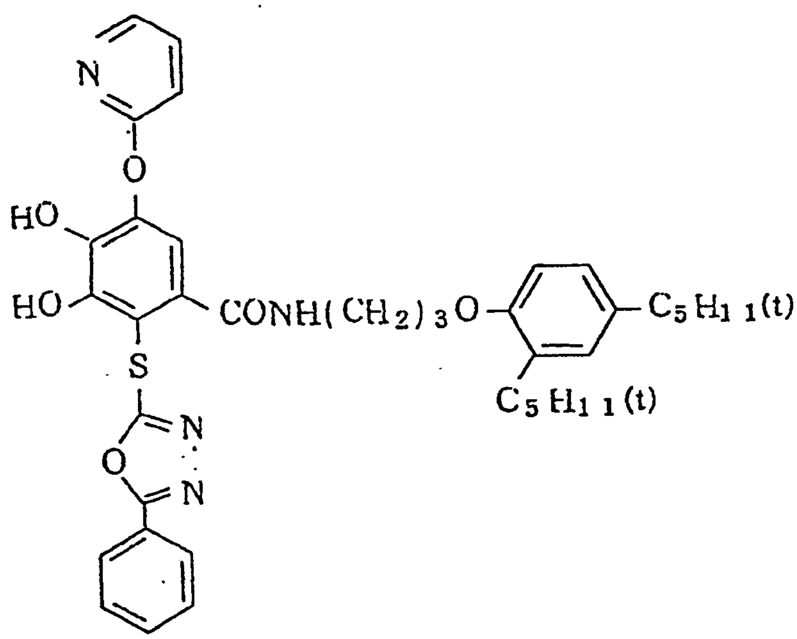

(15)

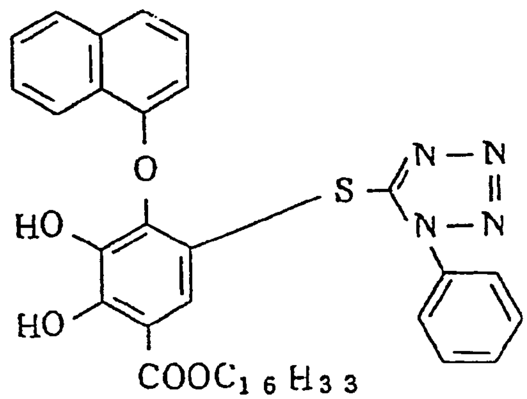

(16)

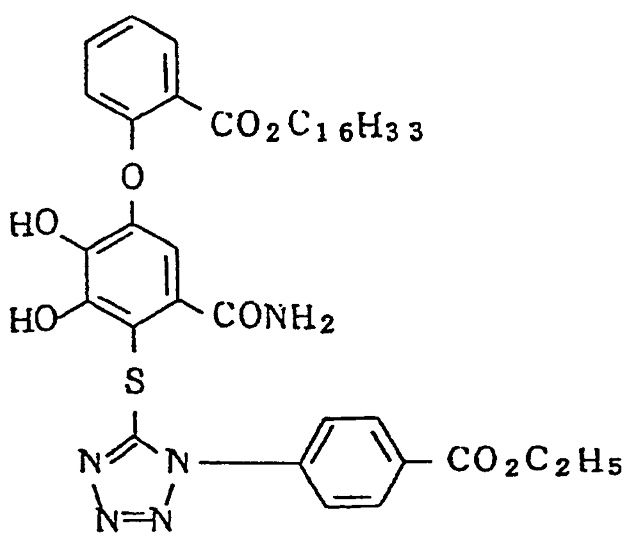

(17)

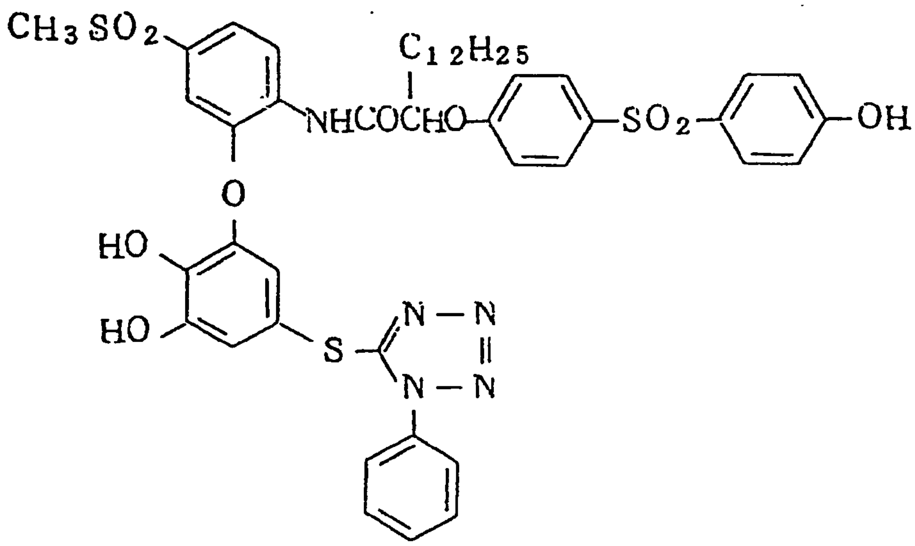

(18)

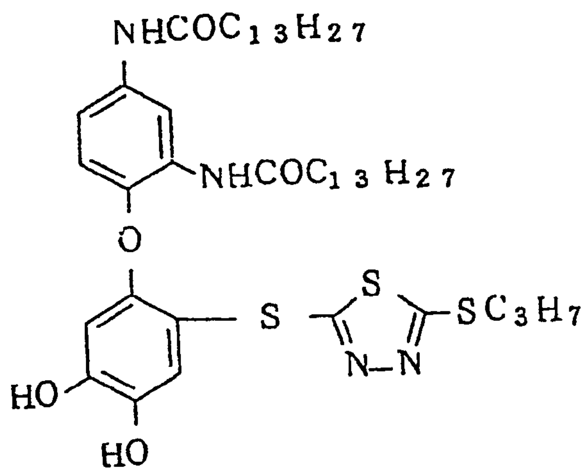

(19)

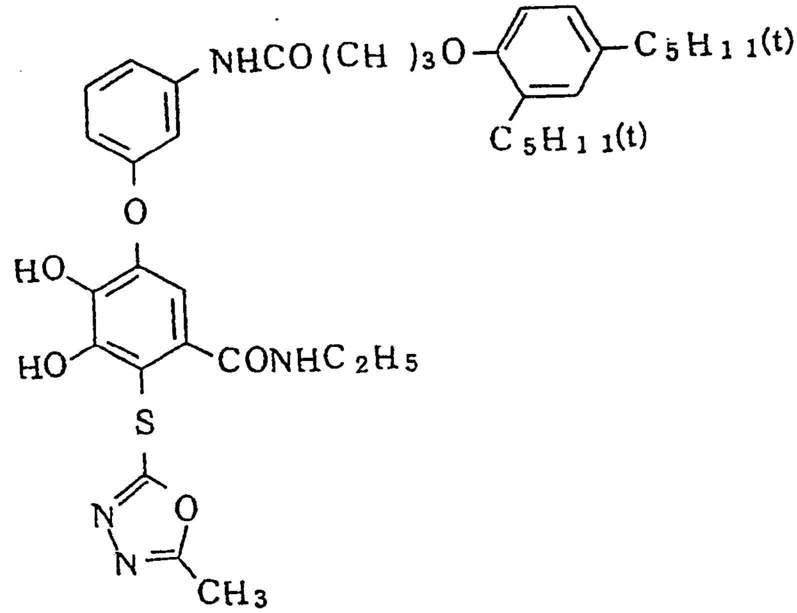

(20)

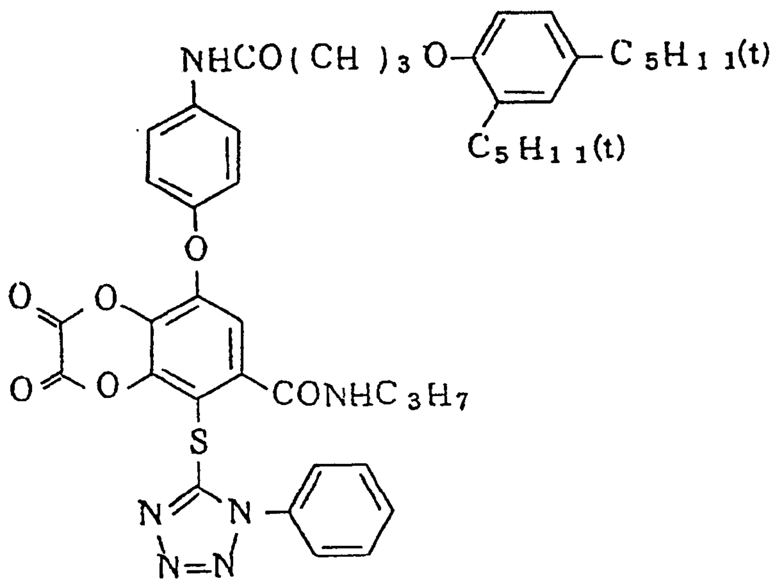

(21)

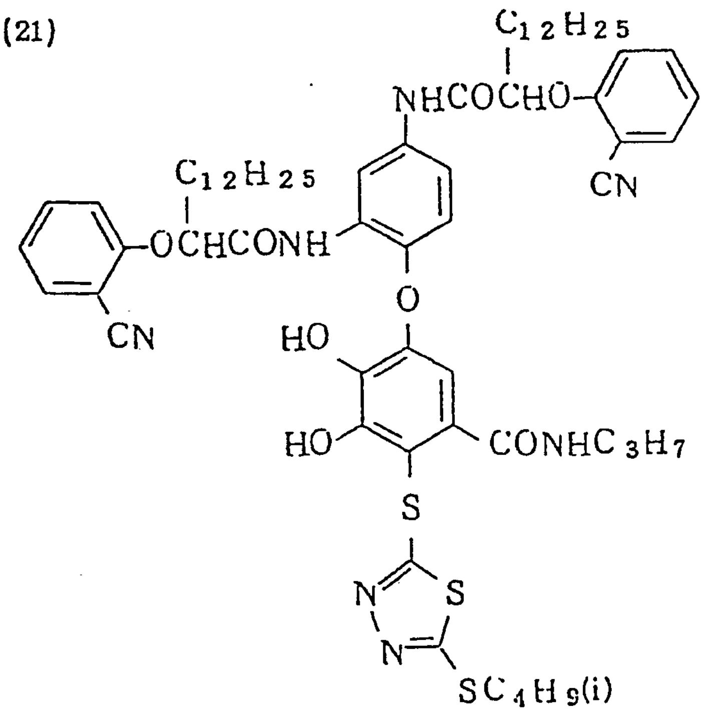

(22)

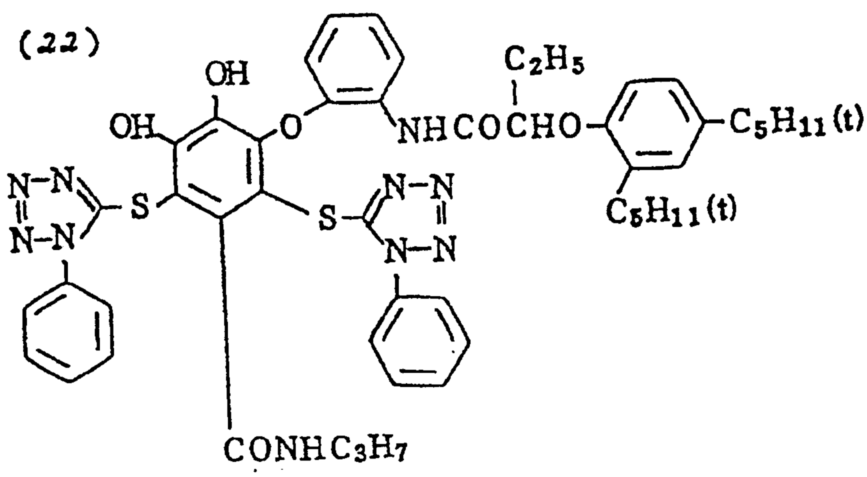

(23)

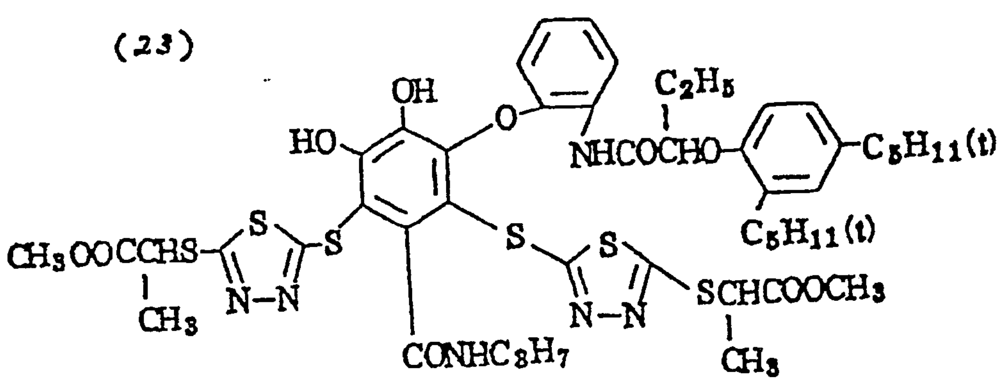

The synthesis of various compound represented by general formula (I) is specifically illustrated below.

SYNTHESIS EXAMPLE

Synthesis of Compound (I)

Compound (I) was synthesized according to the route schematically shown below.

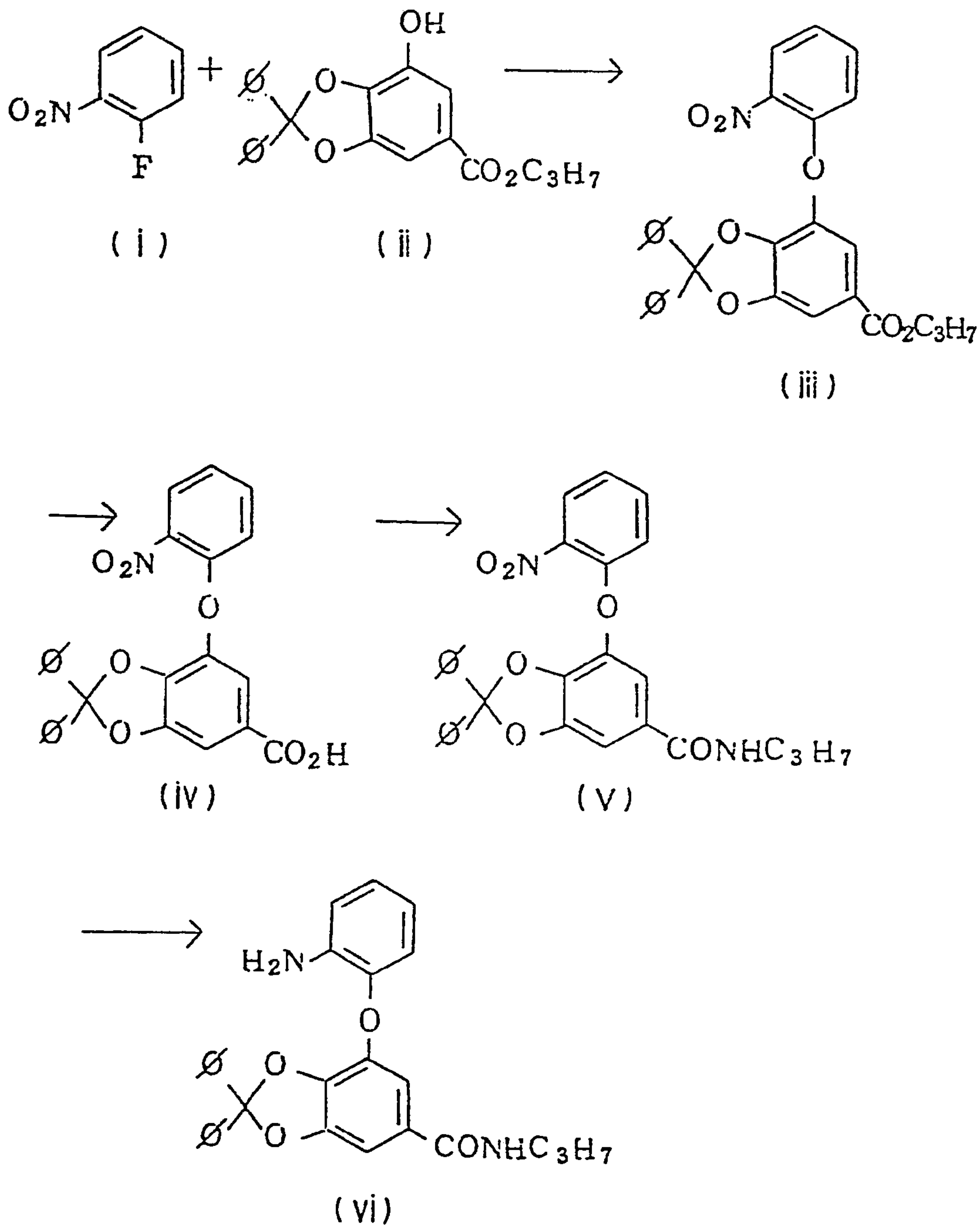

( i ) ( ii ) ( iii )

( iv ) ( v )

( vi )

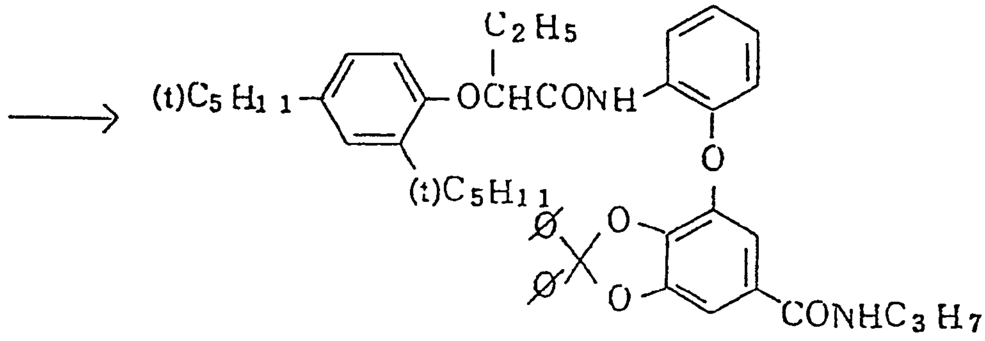

(vii)

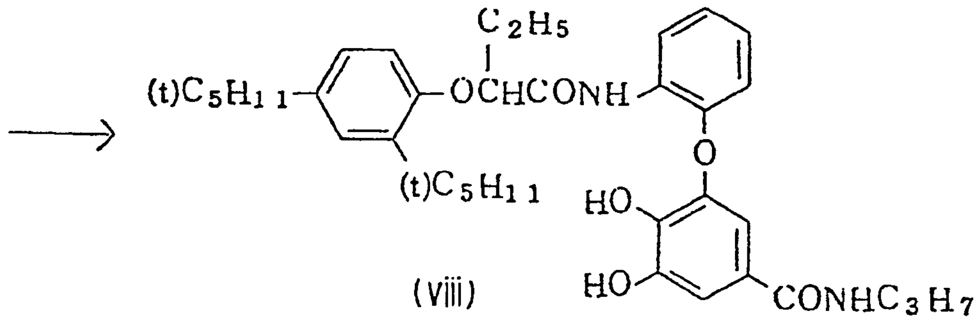

(viii)

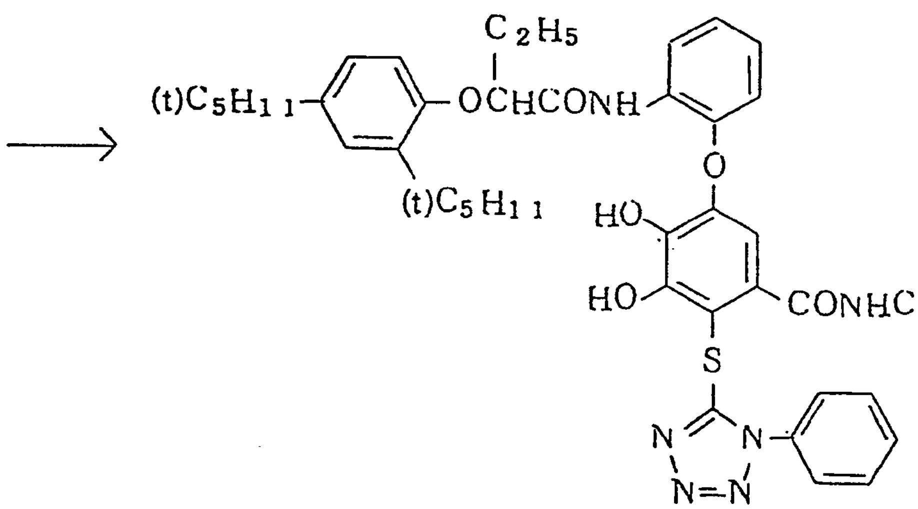

Compound (I)

In the above formulae, the symbol Ø means a phenyl group.

Step 1 : Synthesis of Intermediate Compound (iii)

75 g of Compound (ii), 28 g of Compound (i) and 25 g of potassium tert-butoxide were dissolved in 250 ml of N,N-dimethylformamide and the resulting solution was subjected to reaction for 2 hours at 100°C. After-treatment was conducted in a conventional manner to obtain 105 g of Compound (iii) as an oil.

Step 2 : Synthesis of Intermediate Compound (iv)

105 g of Compound (iii) obtained in Step (1) and 66 g of potassium hydroxide were mixed with 600 ml of a 10 vol% aqueous ethanol solution and the mixture was subjected to reaction at 50°C for 2 hours. After-treatment was conducted in a conventional manner to obtain 79 g of Compound (iv). The compound was washed with acetonitrile and employed in the following step.

Step (3) : Synthesis of Intermediate Compound (v)

53 g of Compound (iv) was dissolved in a solvent mixture composed of 150 ml of acetonitrile and 600 ml of N,N-dimethylacetamide and to the solution at room temperature was added 24 g of thionyl. After reaction at room temperature for 1 hour, the mixture was cooled to – 5°C and 35 g of propylamine was dropwise added thereto. After reaction for 2 hours, the mixture was subjected to after-treatment in a conventional manner. The resulting condensate was purified by column chromatography to obtain 48 g of Compound (v). Silica gel was used as the packing material and a mixture of ethyl acetate and hexane (1 : 2) were used as an eluate.

Step (4) : Synthesis of Intermediate Compound (vi)

48 g of Compound (v) was dissolved in a solvent mixture composed of 300 ml of ethanol and 150 ml of water, to the solution was added 70 g of sodium thiosulfate and the mixture was subjected to reaction at room temperature for 1 hour. After-treatment was conducted in a conventional manner to obtain 30 g of Compound (vi).

Step (5) : Synthesis of Intermediate Compound (vii)

30 g of Compound (vi) and 34 g of 2-(2,4-di-tert-amylphenoxy)butanoyl chloride were reacted in 300 ml of acetonitrile at 50°C for 1 hour. After-treatment was conducted in a conventional manner to obtain 61 g of Compound (vii) as an oil. The oil was employed in the following step without further purification.

Step (6) : Synthesis of Intermediate Compound (viii)

61 g of Compound (vii) was added to 500 ml of methanol containing 10 wt% hydrochloric acid and the mixture was refluxed for 1 hour to obtain 32 g of Compound (viii).

Step (7) : Synthesis of Compound (1)

29 g of 5-mercapto-1-phenyltetrazole was added to 150 ml of dichloromethane and to the mixture was added dropwise 22 g of sulfuryl chloride under cooling with ice. After 1 hour, the solvent was distilled off and to the residue was added 800 ml of tetrahydrofuran containing 32 g of compound (viii) dissolved therein. Under cooling with ice, 8.9 g of aluminum chloride was added thereto and the mixture was subjected to reaction for 2 hours. Water was gradually added and the mixture was extracted with ethyl acetate. The ethyl acetate component was washed with water using a separatory funnel until the water indicated neutral. The oil layer was separated and dried with anhydrous sodium sulfate. After distilling off the solvent, the residue was recrystallized from a solvent mixture of hexane and chloroform to obtain 14.1 g of Compound (1).

The methods described in Journal of Organic Chemistry, Vol. 29, pages 588 and 594 (1964), U.S. Patents 3,364,022, 3,379,529, and 3,639,417 can also be used for the sysnthesis of the compounds represented by general formula (I).

Couplers which can be employed together with the compound(s) represented by general formula (I) according to the present invention are now described in detail.

More specifically, image forming couplers, DIR couplers (for example, couplers as described in U.S. Patents 3,227,554, 4,146,396, 4,248,962, 4,409,323, 4,421,845, 4,477,563 and 3,148,062, etc.), couplers capable of forming a dye having a desired diffusibility (for example, couplers as described in U.S. Patents 4,522,915 and 4,420,556, etc.), couplers capable of releasing a development accelerator or a fogging agent (for example, couplers as described in U.S. Patent 4,390,618, etc.), colored couplers (for example, couplers as described in U.S. Patents 4,004,929, 4,138,258 and 4,070,191, etc.), completing couplers (for example, couplers as described in U.S. Patent 4,130,427, etc.), poly-equivalent couplers (for example, couplers as described in U.S. Patents 4,283,472, 4,338,393 and 4,310,618, etc.), DIR redox compound releasing couplers (for example, couplers as described in Japanese Patent Application (OPI) No. 185950/85, etc.), couplers capable of releasing a dye which turns to a colored form after being released (for example, couplers as described in European Patent Application (OPI) No. 173,302, etc.), and various polymer couplers (for example, couplers as described in U.S. Patents 3,767,412, 3,623,871, 4,367,282 and 4,474,870, etc.), etc., may be employed in the silver halide photographic material of the present invention.

The dyes formed from the couplers may be yellow, magenta and cyan dyes. As yellow couplers, for example, acylacetamide type couplers and malondiamide type couplers are exemplified. As magenta couplers, for example, 5-pyrazolone type couplers, pyrazoloimidazole type couplers and pyrazolotriazole type couplers are exemplified. As cyan couplers, for example, phenol type couplers and naphthol type couplers are exemplified. These couplers may be either four-equivalent or two-equivalent couplers. Further, couplers which do not substantially form dyes may be employed. Examples of such couplers are those as described in U.S. Patents 3,958,993, 3,961,959, 4,315,070, 4,183,752 and 4,171,223, etc.

Couplers preferably employed in the present invention are those represented by the following general formula (Cp-1), (Cp-2), (Cp-3), (Cp-4), (Cp-5), (Cp-6), (Cp-7) or (Cp-8) :

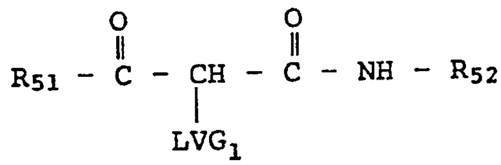

(Cp-1)

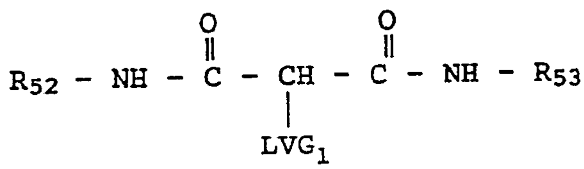

(Cp-2)

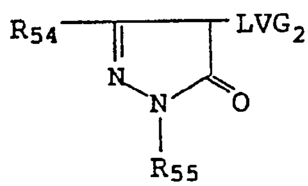

(Cp-3)

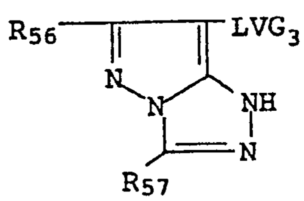

(Cp-4)

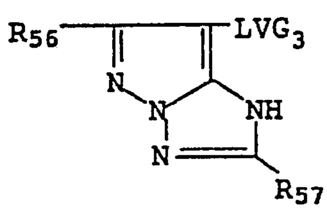

(Cp-5)

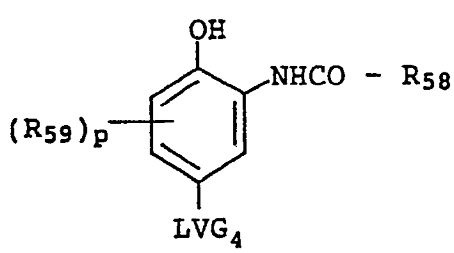

(Cp-6)

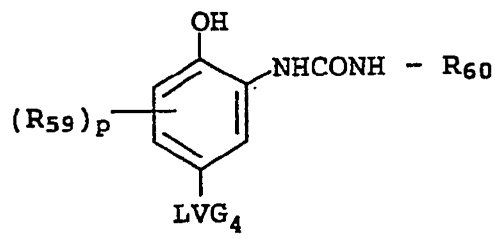

(Cp-7)

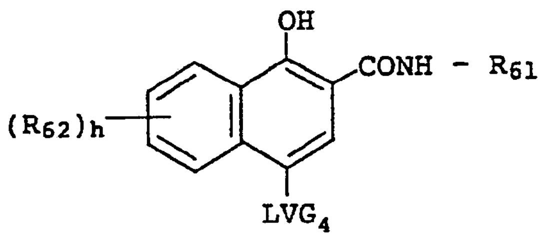

(Cp-8)

$R_{51}$ to $R_{62}$, $LVG_1$ to $LVG_4$, p and h in the above general formulae (Cp-1) to (Cp-8) are explained in detail.

In the above-described general formulae, when $R_{61}$, $R_{52}$, $R_{53}$, $R_{54}$, $R_{65}$, $R_{56}$, $R_{57}$, $R_{58}$, $R_{69}$, $R_{60}$, $R_{61}$, $R_{62}$, $LVG_1$, $LVG_2$, $LVG_3$, or $LVG_4$ contains a diffusion resistant group (a ballast group), this group is selected so that the total number of carbon atoms included therein is from 8 to 40 and preferably from 12 to 32. In other cases, the total number of carbon atoms included therein is preferably not more than 15. In the case of a bis, telomer or polymer type coupler, any of the above-described substituents forms a divalent group and may connect to a repeating unit, etc. In such cases, the total number of carbon atoms can be outside of the above-described range.

In the following, $R_{41}$ represents an aliphatic group, an aromatic group or a heterocyclic group ; $R_{42}$ represents an aromatic group or a heterocyclic group ; and $R_{43}$, $R_{44}$ and $R_{45}$ each represents a hydrogen atom, an aliphatic group, an aromatic group or a heterocyclic group.

$R_{51}$ represents a group as defined for $R_{41}$.

$R_{52}$ and $R_{53}$ each represents a group as defined for $R_{42}$.

$R_{54}$ represents a group as defined for $R_{41}$, the group $R_{41}CO\underset{R_{43}}{\underset{|}{N}}-$, the group $R_{41}\underset{R_{43}}{\underset{|}{N}}-$, the group $R_{41}SO_2\underset{R_{43}}{\underset{|}{N}}-$, the group $R_{41}S-$, the group $R_{43}O-$, the group $R_{45}\underset{R_{43}}{\underset{|}{N}}CO\underset{R_{44}}{\underset{|}{N}}-$, the group $R_{41}OOC-$, the group $R_{44}\underset{R_{43}}{\underset{|}{N}}CO-$ or the group $N{\equiv}C-$.

$R_{55}$ represents a group as defined for $R_{41}$.

$R_{56}$ and $R_{57}$ each represents a group as defined for $R_{43}$, the group $R_{41}S-$, the group $R_{43}O-$, the group $R_{41}CO\underset{R_{43}}{N}-$, the group $R_{41}\underset{R_{43}}{N}-$, the group $R_{41}OCO\underset{R_{43}}{N}-$, the group $R_{41}O-$, the group $R_{43}\underset{R_{44}}{N}CO\underset{R_{45}}{N}-$ or the group $R_{41}SO_2\underset{R_{43}}{N}-$.

$R_{58}$ represents a group as defined for $R_{41}$.

$R_{59}$ represents a group as defined for $R_{41}$, the group $R_{41}CO\underset{R_{43}}{N}-$, the group $R_{41}OCO\underset{R_{43}}{N}-$, the group $R_{43}SO_2\underset{R_{43}}{N}-$, the group $R_{43}\underset{R_{44}}{N}CO\underset{R_{45}}{N}-$, the group $R_{43}\underset{R_{44}}{N}SO_2\underset{R_{45}}{N}-$, the group $R_{41}O-$, the group $R_{41}S-$, a halogen atom (e.g., a fluorine atom, a chlorine atom) or the group $R_{41}\underset{R_{43}}{N}-$.

p represents an integer from 0 to 3. When p represents 2 or more, two or more $R_{59}$'s may be the same or different. Further, each of two $R_{59}$'s may be a divalent group and they can be connected with each other to form a cyclic structure.

Examples of such divalent groups for forming a cyclic structure include the group

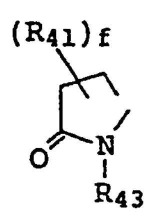

, the group

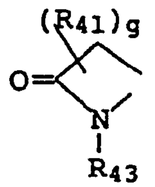 or the group 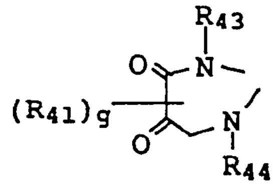 , wherein f represents an integer from 0 to 4 ; and g represents an integer from 0 to 2.

$R_{60}$ represents a group as defined for $R_{41}$.

$R_{61}$ represents a group as defined for $R_{41}$.

$R_{62}$ represents a group as defined for $R_{41}$, the group $R_{41}CONH-$, the group $R_{41}OCONH-$, the group $R_{41}SO_2NH-$, the group $R_{43}\underset{R_{44}}{\underset{|}{N}}CO\underset{R_{45}}{\underset{|}{N}}-$, the group $R_{43}\underset{R_{44}}{\underset{|}{N}}SO_2\underset{R_{45}}{\underset{|}{N}}-$, the group $R_{43}O-$, the group $R_{41}S-$, a halogen atom (e.g., a fluorine atom, a chlorine atom) or the group $R_{41}\underset{R_{43}}{\underset{|}{N}}-$.

h represents an integer from 0 to 4. When h represents 2 or more, two or more $R_{62}$'s may be the same or different. Further, each two $R_{62}$'s may be divalent group, and when two $R_{62}$'s are present respectively on two carbon atoms adjacent to each other, two $R_{62}$'s can be connected with each other to form a cyclic structure.

The aliphatic group described above for $R_{41}$, $R_{43}$, $R_{44}$ and $R_{45}$ is an aliphatic hydrocarbon group having from 1 to 40 carbon atoms, preferably from 1 to 22 carbon atoms and may be saturated or unsaturated, straight chain, branched chain or cyclic, substituted or unsubstituted. Representative examples of the unsubstituted aliphatic group include a methyl group, an ethyl group, a propyl group, an isopropyl group, a butyl group, a tert-butyl group, an isobutyl group, a tert-amyl group, a hexyl group, a cyclohexyl group, a 2-ethylhexyl group, an octyl group, a, 1,1,3,3-tetramethylbutyl group, a decyl group, a dodecyl group, a hexadecyl group, or an octadecyl group, etc.

The aromatic group described above for $R_{41}$, $R_{42}$, $R_{43}$, $R_{44}$ and $R_{45}$ is an aromatic group having from 6 to 20 carbon atoms, and preferably an unsubstituted or substituted phenyl group or an unsubstituted or substituted naphthyl group.

The heterocyclic group described above for $R_{41}$, $R_{42}$, $R_{43}$, $R_{44}$ and $R_{45}$ is a heterocyclic group having from 1 to 20 carbon atoms, preferably from 1 to 7 carbon atoms and contains at least one of a nitrogen atom, an oxygen atom and a sulfur atom, as a hetero atom, and preferably is a three-membered to eight-membered, substituted or unsubstituted heterocyclic group. The numbers of hetero atoms in the heterocyclic group is preferably 1 to 4. Representative examples of the unsubstituted heterocyclic group include a 2-pyridyl group, a 4-pyridyl group, a 2-thienyl group, a 2-furyl group, a 2-imidazolyl group, a pyrazinyl group, a 2-pyrimidinyl group, a 1-imidazolyl group, a 1-indolyl group, a phthalimido group, a 1,3,4-thiadiazol-2-yl group, a benzoxazol-2-yl group, a 2-quinolyl group, a 2,4-dioxo-1,3-imidazolidin-5-yl group, 2,4-dioxo-1,3-imidazolidin-3-yl group, a succinimido group, a phthalimido group, a 1,2,4-triazol-2-yl group, or a 1-pyrazolyl group, etc.

The aliphatic group, aromatic group and heterocyclic group may have one or more substituent as indicated above. Representative examples of the substituents include a halogen atom, the group $R_{47}O-$, the group

$R_{46}S-$, the group 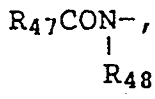, the group 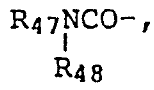, the group 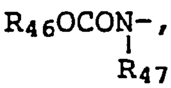, the group 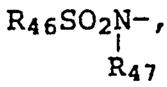, the group 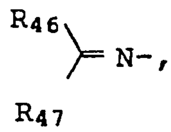, the group 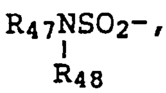, the group $R_{46}SO_2-$, the group $R_{47}OCO-$, the group 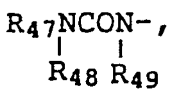, the group 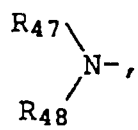, a group as defined for $R_{46}$, the group 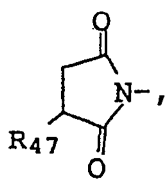, the group $R_{46}COO-$, the group $R_{41}OSO_2-$, a cyano group, a nitro group, etc. In the above described formulae, $R_{46}$ represents an aliphatic group, an aromatic group or a heterocyclic group ; and $R_{47}$, $R_{48}$ and $R_{49}$ each represents a hydrogen atom, an aliphatic group, an aromatic group or a heterocyclic group. The aliphatic group, aromatic group and heterocyclic group each has the same meaning as defined above.

Preferred embodiments for $R_{51}$ to $R_{62}$, p and h are described below.

$R_{51}$ is preferably an aliphatic group or an aromatic group.

$R_{52}$, $R_{53}$ and $R_{55}$ each is preferably an aromatic group.

$R_{54}$ is preferably the group $R_{41}CONH-$ or the group

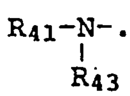.

$R_{56}$ and $R_{57}$ each is preferably an aliphatic group, the group $R_{41}O-$ or the group $R_{41}S-$.

$R_{58}$ is preferably an aliphatic group or an aromatic group.

$R_{59}$ in the general formula (Cp-6) is preferably a chlorine atom, an aliphatic group or the group $R_{41}CONH-$.

p in the general formula (Cp-6) is preferably 1 or 2.

$R_{60}$ is preferably an aromatic group.

$R_{59}$ in the general formula (Cp-7) is preferably the group $R_{41}CONH-$.

p in the general formula (Cp-7) is preferably 1.

$R_{61}$ is preferably an aliphatic group or an aromatic group.

h in the general formula (Cp-8) is preferably 0 or 1.

$R_{62}$ is preferably the group $R_{41}OCONH-$, the group $R_{41}CONH-$ or the group $R_{41}SO_2NH-$. The position of $R_{62}$ is preferably the 5-position of the naphthol ring.

Representative examples for $R_{51}$ to $R_{62}$ groups are set forth below.

Examples for $R_{51}$ include a tert-butyl group, a 4-methoxyphenyl group, a phenyl group, a 3-[2-(2,4-di-tert-amylphenoxy)butanamido]phenyl group, a 4-octadecyloxyphenyl group, a methyl group, etc.

Examples of $R_{52}$ and $R_{53}$ include a 2-chloro-5-dodecyloxycarbonylphenyl group, a 2-chloro-5-hexadecylsulfonamidophenyl group, a 2-chloro-5-tetradecanamidophenyl group, a 2-chloro-5-[4-(2,4-di-tert-amylphenoxy)butanamido]phenyl group, a 2-chloro-5-[2-(2,4-di-tert-amylphenoxy)butanamido]phenyl group,

2-methoxyphenyl group, a 2-methoxy-5-tetradecyloxycarbonylphenyl group, a 2-chloro-5-(1-ethoxycarbonylethoxycarbonyl)phenyl group, a 2-pyridyl group, a 2-chloro-5-octyloxycarbonylphenyl group, a 2,4-dichlorophenyl group, a 2-chloro-5-(1-dodecyloxycarbonylethoxycarbonyl)phenyl group, a 2-chlorophenyl group, a 2-ethoxyphenyl group, etc.

Examples for $R_{54}$ include a 3-[2-(2,4-di-tert-amylphenoxybutanamido]benzamido group, a 3-[4-(2,4-di-tert-amylphenoxy)butanamido]benzamido group, a 2-chloro-5-tetradecanamidoanilino group, a 5-(2,4-di-tert-amylphenoxyacetamido)benzamido group, a 2-chloro-5-dodecenylsuccinimidoanilino group, a 2-chloro-5-[2-(3-tert-butyl-4-hydroxyphenoxy)tetradecanamido]anilino group, a 2,2-dimethylpropanimido group, a 2-(3-pentadecylphenoxy)butanamido group, a pyrrolidino group, an N,N-dibutylamino group, etc.

Examples for $R_{55}$ include a 2,4,6-trichlorophenyl group, a 2-chlorophenyl group, a 2,5-dichlorophenyl group, a 2,3-dichlorophenyl group, a 2,6-dichloro-4-methoxyphenyl group, a 4-[2-(2,4-di-tert-amylphenoxy)butanamido]phenyl group, a 2,6-dichloro-4-methanesulfonylphenyl group, etc.

Examples for $R_{56}$ include a methyl group, an ethyl group, an isopropyl group, a methoxy group, an ethoxy group, a methylthio group, an ethylthio group, a 3-phenylureido group, a 3-butylureido group, a 3-(2,4-di-tert-amylphenoxy)propyl group, etc.

Examples for $R_{57}$ include a 3-(2,4-di-tert-amylphenoxy)propyl group, a 3-[4-{2-[4-(4-hydroxyphenylsulfonyl)phenoxy]tetradecanamido}phenyl]propyl group, a methoxy group, an ethoxy group, a methylthio group, an ethylthio group, a methyl group, a 1-methyl-2-{2-octyloxy-5-[2-octyloxy-5-(1,1,3,3-tetramethylbutyl)phenylsulfon amido]phenylsulfonamido}ethyl group, a 3-[4-(4-dodecyloxyphenylsulfonamido)phenyl]propyl group, a 1,1-dimethyl-2-[2-octyloxy-5-(1,1,3,3-tetramethylbutyl)phenylsulfonamido]ethyl group, a dodecylthio group, etc.

Examples for $R_{58}$ include a 2-chlorophenyl group, a pentafluorophenyl group, a heptafluoropropyl group, a 1-(2,4-di-tert-amylphenoxy)propyl group, a 3-(2,4-di-tert-amylphenoxy)propyl group, a 2,4-di-tert-amylmethyl group, a furyl group, etc.

Examples for $R_{59}$ include a chlorine atom, a methyl group, an ethyl group, a propyl group, a butyl group, an isopropyl group, a 2-(2,4-di-tert-amylphenoxy)butanamido group, a 2-(2,4-di-tert-amylphenoxy)hexanamido group, a 2-(2,4-di-tert-octylphenoxy)octanamido group, a 2-(2-chlorophenoxy)tetradecanamido group, a 2,2-dimethylpropanamido group, a 2-[4-(4-hydroxyphenylsulfonyl)phenoxy]tetradecanamido group, a 2-[2-(2,4-di-tert-amylphenoxyacetamido)phenoxy]butanamido group, etc.

Examples for $R_{60}$ include a 4-cyanophenyl group, a 2-cyanophenyl group, a 4-butylsulfonylphenyl group, a 4-propylsulfonylphenyl group, a 4-ethoxycarbonylphenyl group, a 4-N,N-diethylsulfamoylphenyl group, a 3,4-dichlorophenyl group, a 3-methoxycarbonylphenyl group, etc.

Examples for $R_{61}$ include a dodecyl group, a hexadecyl group, a cyclohexyl group, a butyl group, a 3-(2,4-di-tert-amylphenoxy)propyl group, a 4-(2,4-di-tert-amyl phenoxy)butyl group, a 3-dodecyloxypropyl group, a 2-tetradecyloxyphenyl group, a tert-butyl group, a 2-(2-hexyldecyloxy)phenyl group, a 2-methoxy-5-dodecyloxycarbonylphenyl group, a 2-butoxyphenyl group, a 1-naphthyl group, etc.

Examples for $R_{62}$ include an isobutyloxycarbonylamino group, an ethoxycarbonylamino group, a phenylsulfonylamino group, a methanesulfonamido group, a butanesulfonamido group, a 4-methylbenzenesulfonamido group, a benzamido group, a trifluoroacetamido group, a 3-phenylureido group, a butoxycarbonylamino group, an acetamido group, etc.

$LVG_1$, $LVG_2$, $LVG_3$, and $LVG_4$ are explained below in more detail.

In the above-described general formulae, $LVG_1$, $LVG_2$, $LVG_3$, and $LVG_4$ each represents a coupling releasing group or a hydrogen atom. Preferred examples of $LVG_1$ to $LVG_4$ are now given.

Examples of preferred $LVG_1$ groups include the group $R_{65}O$ –, an imido group which is connected to the coupling position through the nitrogen atom thereof (for example, a 2,4-dioxo-1,3-imidazolidin-3-yl group, a 2,4-dioxo-1,3-oxazolidin-3-yl group, a 3,5-dioxo-1,2,4-triazolidin-4-yl group, a succinimido group, a phthalimido group, or a 2,4-dioxo-1,3-imidazolidin-1-yl group, etc.), an unsaturated nitrogen-containing heterocyclic group which is connected to the coupling position through the nitrogen atom and contains preferably 1 to 4 nitrogen atoms and 1 to 4 the other hetero atoms (for example, a 1-imidazolyl group, a 1-pyrazolyl group, a 1,2,4-triazol-2(or-4)-yl group, a benzotriazol-1-yl group, or a 3-pyrazolin-5-one-2-yl group, etc.) or a group of $R_{66}S$ –.

Examples of preferred $LVG_2$ groups include the group $R_{66}S$ –, an unsaturated nitrogen-containing heterocyclic group which is connected to the coupling position through the nitrogen atom thereof (for example, a 1-pyrazolyl group, a 1-imidazolyl group, a 1,2,4-triazol-2(or-4)-yl group, a benzotriazol-1-yl group, a benzimidazolyl group, or a benzoimidazolyl group, etc.), the group $R_{65}O$ – or a hydrogen atom.

Examples of preferred $LVG_3$ groups include a halogen atom (e.g., a fluorine atom, a chlorine atom), the group $R_{66}S$ –, an unsaturated nitrogen-containing heterocyclic group which is connected to the coupling position through the nitrogen atom thereof (for example, a 1-pyrazolyl group, a 1-imidazolyl group, or a benzotriazol-1-yl group, etc.) or a hydrogen atom.

Examples of preferred $LVG_4$ groups include a halogen atom(e.g., a fluorine atom, a chlorine atom), the group $R_{66}O$ –, the group $R_{66}S$ – or a hydrogen atom.

In the above formulae, $R_{65}$ represents an aromatic group or a heterocyclic group, and $R_{66}$ represents an aliphatic group, an aromatic group or a heterocyclic group. The aromatic group, heterocyclic group and aliphatic group each has the same meaning an defined for $R_{41}$.

When $LVG_1$, $LVG_2$, or $LVG_3$ represents the above-described heterocyclic group, it may have one or more substituents at any position capable of being substituted. Representative examples of the substituents include those as illustrated for the heterocyclic group represented by $R_{41}$.

Representative examples of $LVG_1$, $LVG_2$, $LVG_3$ and $LVG_4$ are set forth below.

As $LVG_1$ a 1-benzyl-5-ethoxy-2,4-dioxo-1,3-imidazolidin-3-yl group, a 1-methyl-5-hexyloxy-2,4-dioxo-1,3-imidazolidin-3-yl group, a 1-phenyl-5-benzyl-2,4-dioxo-1,3,5-triazolidin-3-yl group, a 5,5-dimethyl-2,4-dioxo-1,3-oxazolidin-3-yl group, a 1-pyrazolyl group, a 4,5-bis(methoxycarbonyl)imidazol-1-yl group, a 2-phenylcarbamoyl-1,3-imidazolyl-1-yl group, a 4-phenylcarbamoyl-1,3-imidazolyl-1-yl group, a 6-methylxanthen-1-yl group, a 4-(4-hydroxyphenylsulfonyl)phenoxy group, a 4-isopropoxyphenoxy group, a 4-cyanophenoxy group, a 2-chloro-4-(2-chloro-4-hydroxyphenylsulfonyl)phenoxy group, a 5-phenoxycarbonyl-1-benzotriazolyl group, a 4-carboxyphenoxy group or a 4-(4-benzyloxyphenylsulfonyl)phenoxy group, etc., are exemplified.

As $LVG_2$ : a hydrogen atom, a 1-pyrazolyl group, a 3-chloro-5-methyl-1,2,4-triazol-2-yl group, a 5-phenoxycarbonyl-1-benzotriazolyl group, a 2-butoxy-5-(1,1,3,3-tetramethylbutyl)phenylthio group, a 4-chloro-1-pyrazolyl group, a 4-[3-(2-decyl-4-methylphenoxyacetoxy)propyl]pyrazol-1-yl group, a dodecyloxycarbonylmethylthio group, a 1-phenyltetrazolyl-5-thio group or a 4-dodecylsulfamoylphenoxy group, etc., are exemplified.

As $LVG_3$ : a chlorine atom, a hydrogen atom, a 4-methylphenoxy group, a 4-cyanophenoxy group, a 2-butoxy-5-(1,1,3,3-tetramethylbutyl)phenylthio group, a 1-pyrazolyl group or a 2-(2-phenoxyethoxy)-5-(1,1,3,3-tetramethylbutyl)phenylthio group, etc., are exemplified.

As $LVG_4$ : a chlorine atom, a hydrogen atom, a 4-methoxyphenoxy group, a 4-(1,1,3,3-tetramethylbutyl)phenoxy group, a 2-carboxyethylthio group, a 2-(2-carboxyethylthio)ethoxy group, a 1-phenyltetrazolyl-5-thio group, 1-ethyltetrazolyl-5-thio group, a 3-carboxypropoxy group, a 5-phenoxycarbonylbenzotirazol-1-methoxy group, a 2, 3-dihydroxy-4-(1-phenyltetrazolyl-5-thio)-5-propylcarbamoylphenoxy group, a 2-(1-carboxytridecylthio)ethoxy group, a 2-(2-methoxyethylcarbamoyl)ethoxy group or the disodium salt of a 2-[4-(8-acetamido-1-hydroxy-3,6-disulfonaphthyl-2-azo)phenoxy]ethoxy group, etc., are exemplified.

Specific examples of couplers which can be used together with the compounds according to the present invention are set forth below, but the present invention should not be construed as being limited thereto.

C－(1)

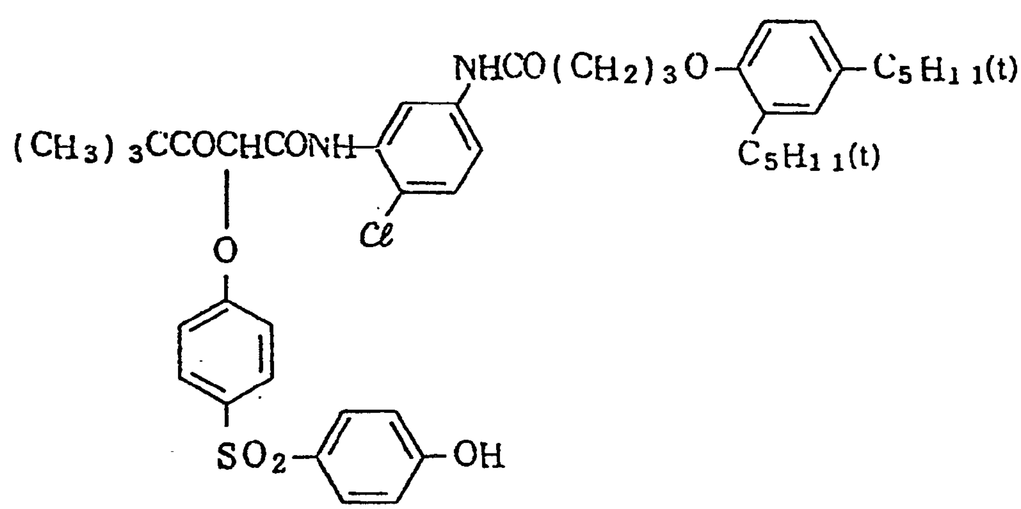

C — (2)

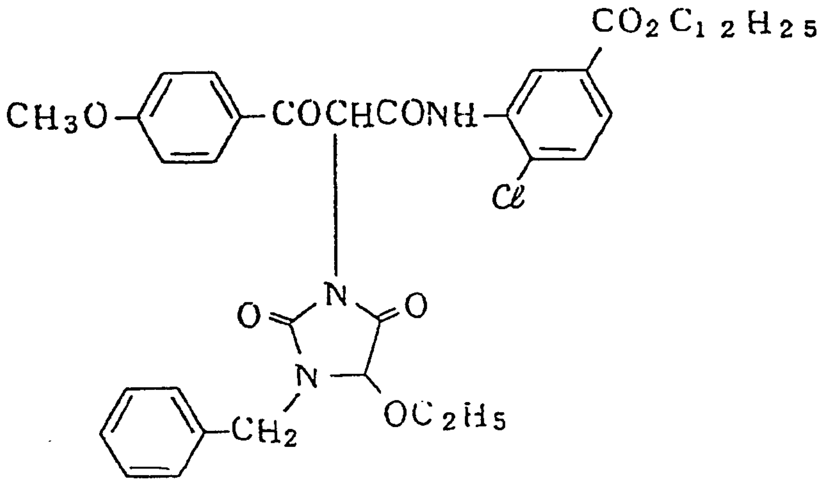

C — (3)

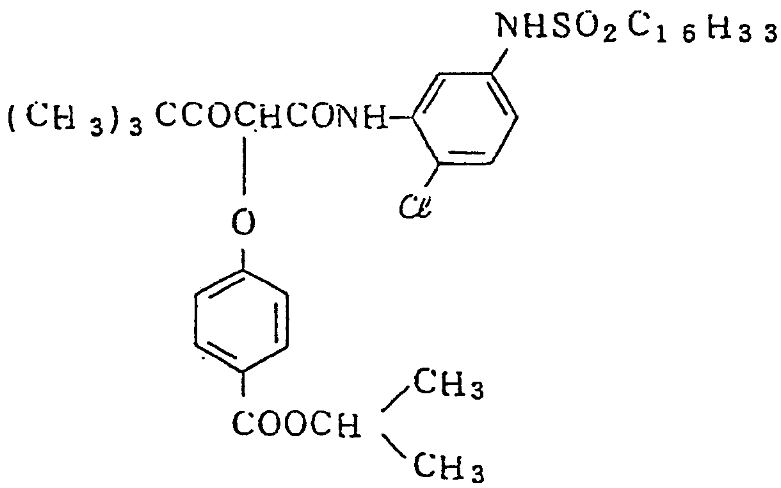

C — (4)

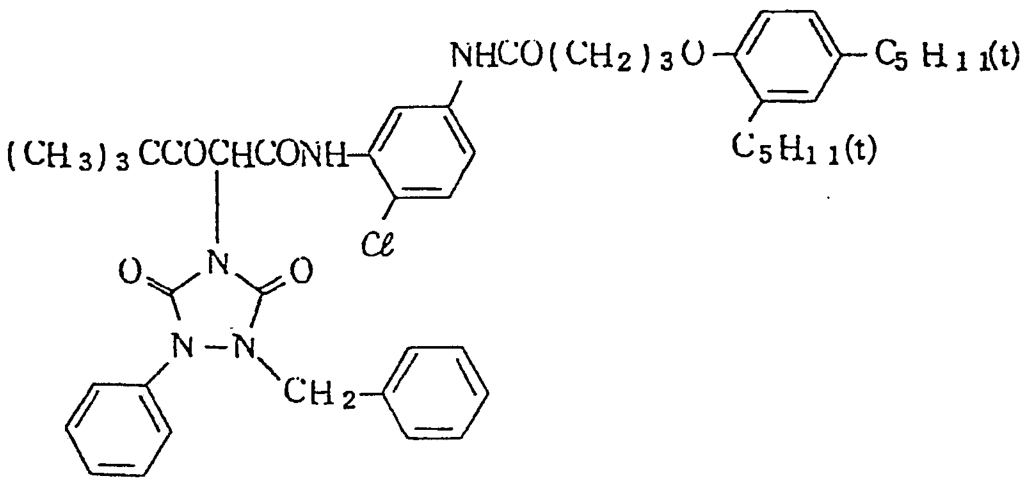

C — (5)

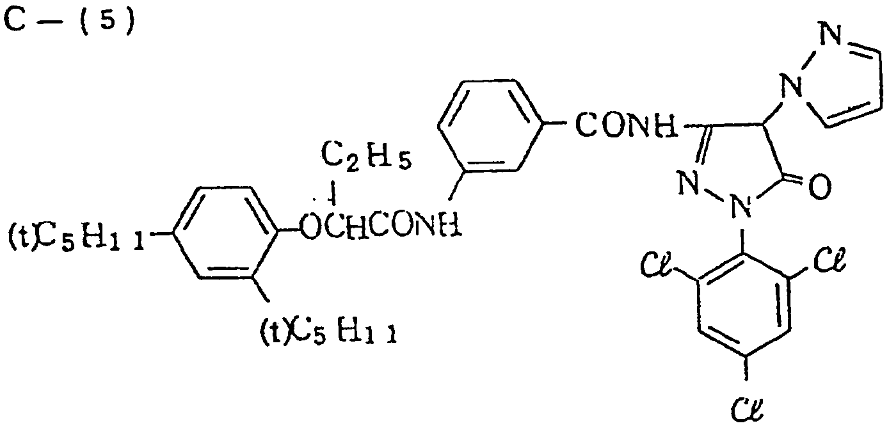

C — (6)

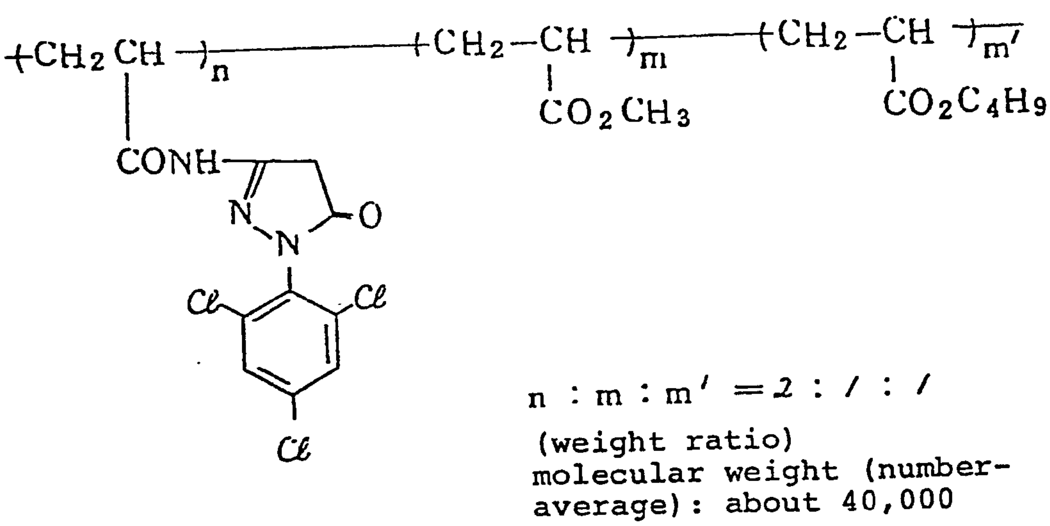

n : m : m' = 2 : 1 : 1

(weight ratio)
molecular weight (number-average): about 40,000

C – (7)

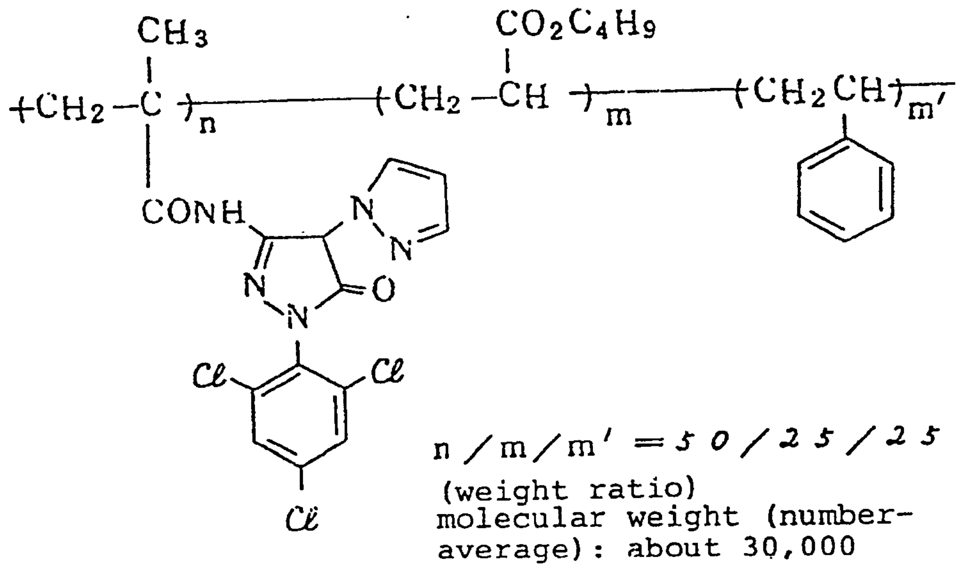

$n/m/m' = 50/25/25$
(weight ratio)
molecular weight (number-average): about 30,000

C – (8)

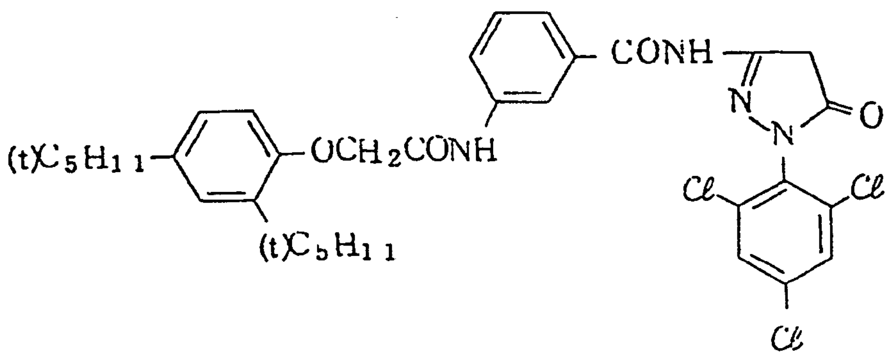

C – (9)

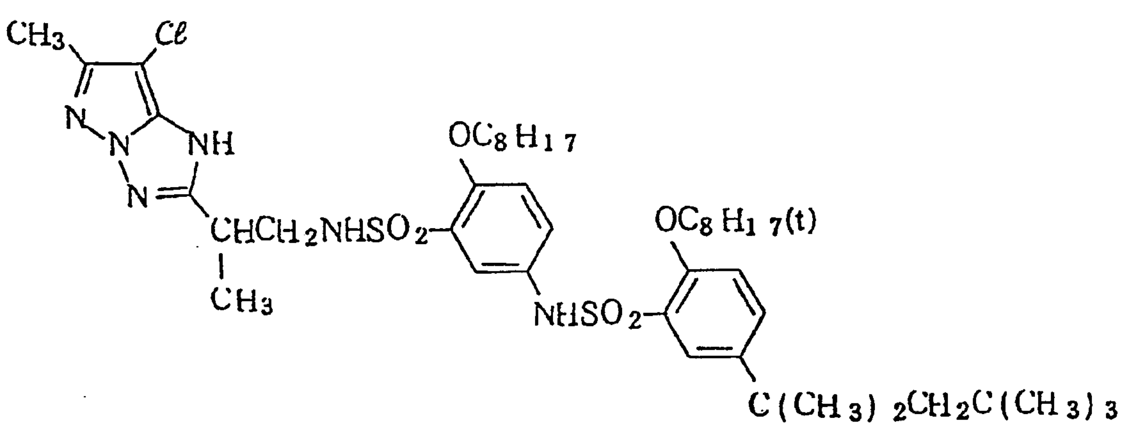

C — (10)

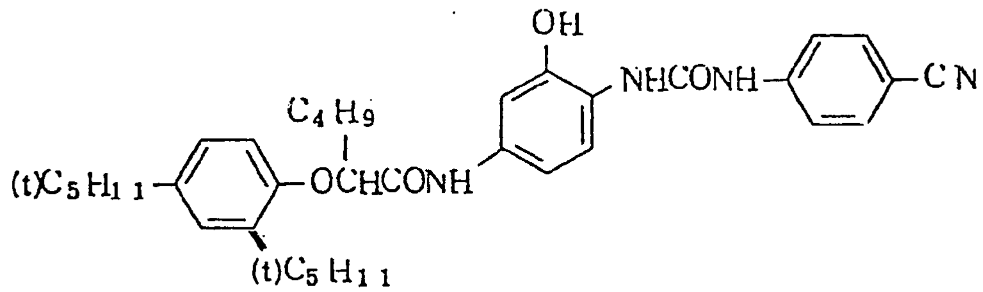

C — (11)

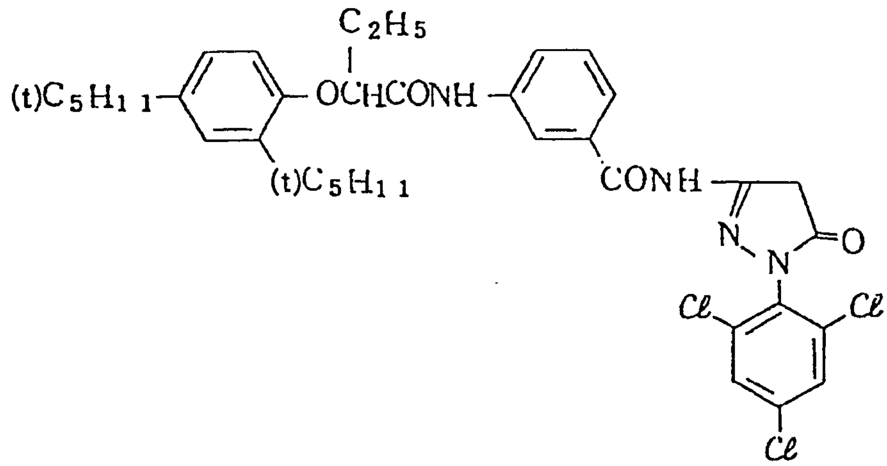

C — (12)

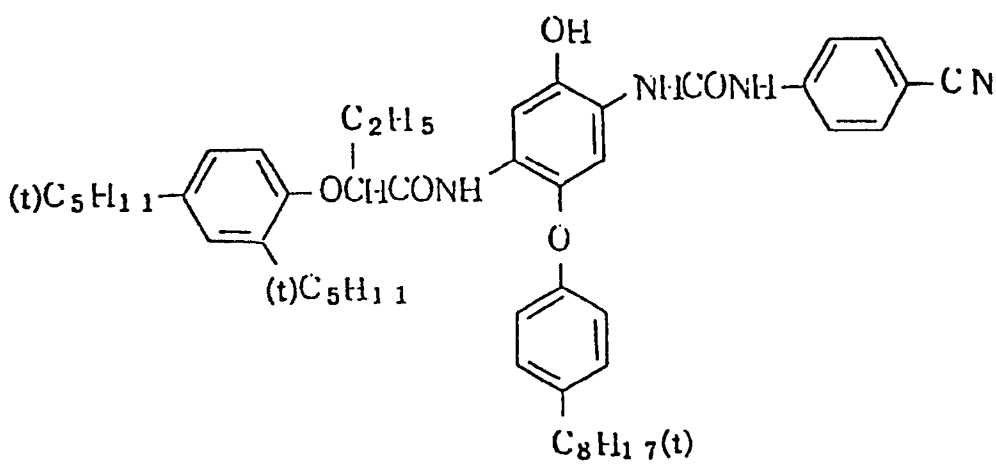

C — (13)

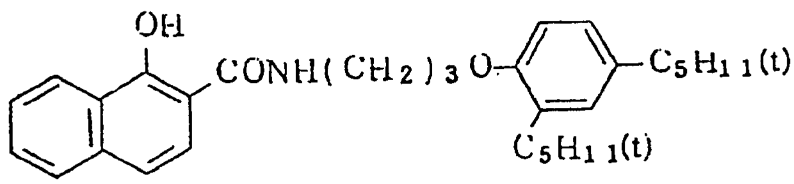

C — (14)

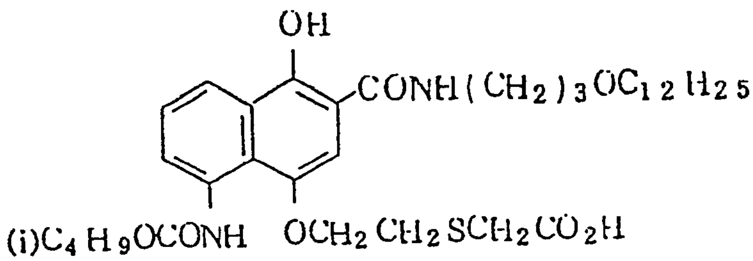

C — (15)

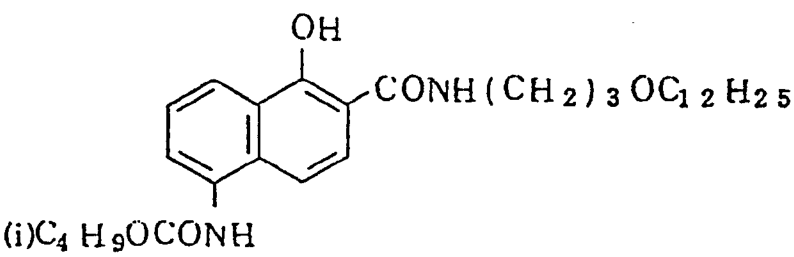

C — (16)

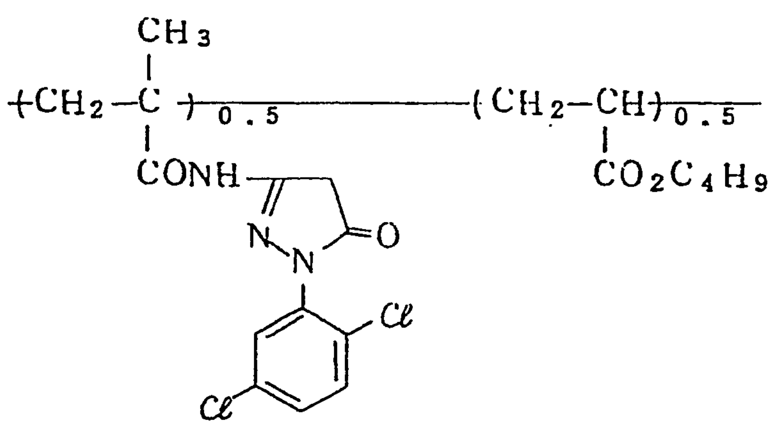

C — (17)

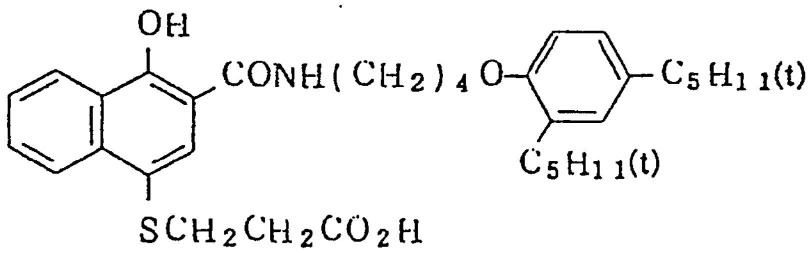

C — (18)

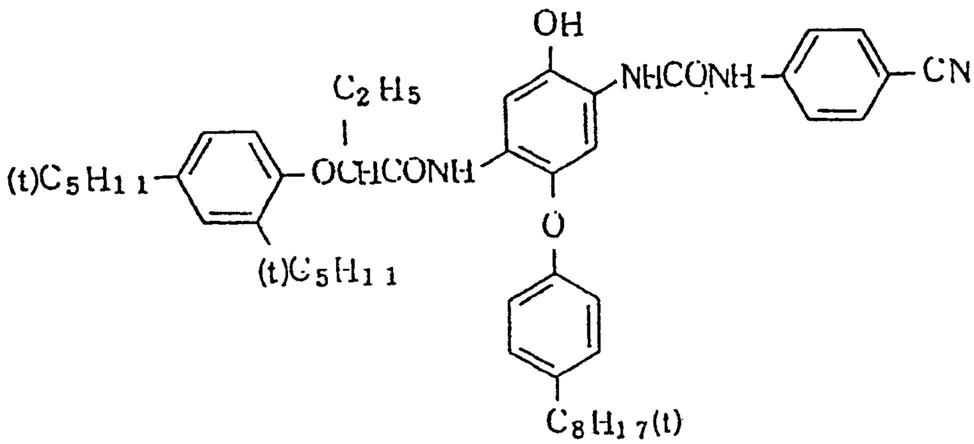

C — (19)

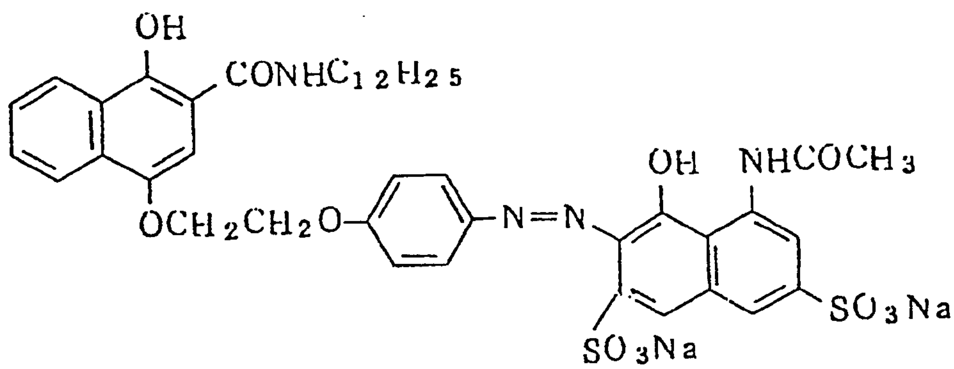

C– (20)

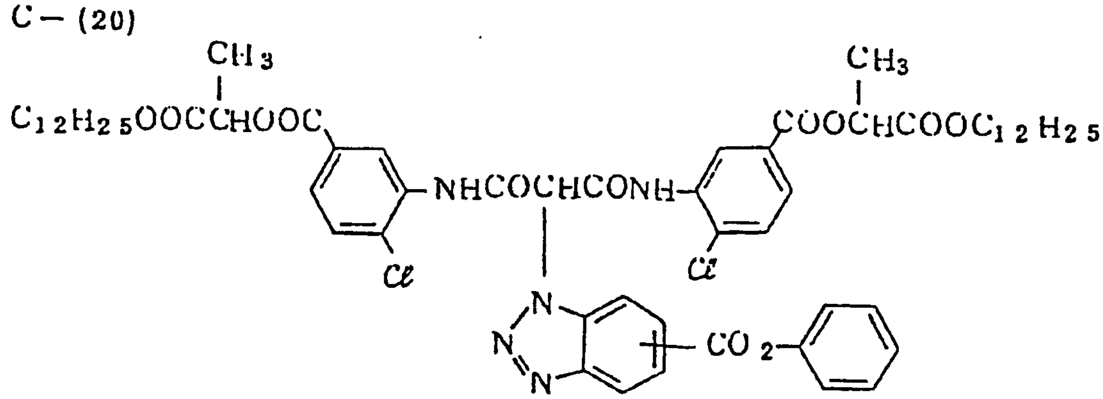

(a mixture of isomers containing 5-positioned and 6-positioned -$CO_2$- 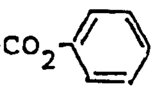 groups)

C– (21)

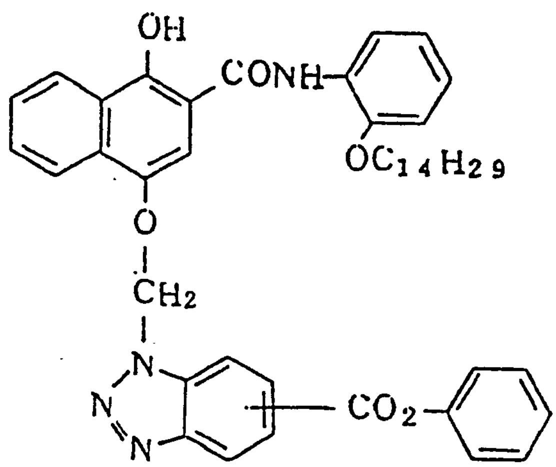

(a mixture of isomers containing 5-positioned and 6-positioned -$CO_2$- 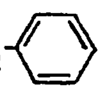 group)

C-(22)

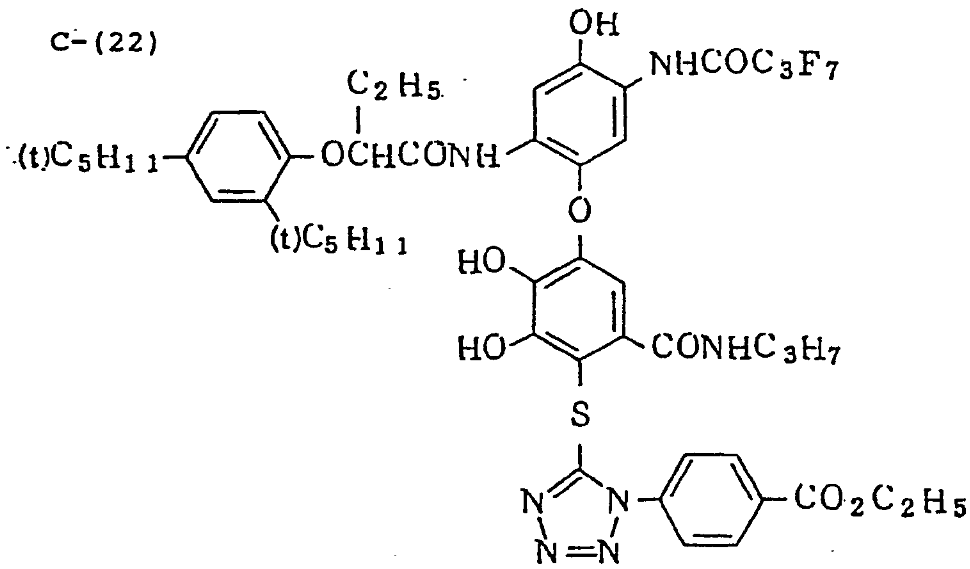

C-(23)

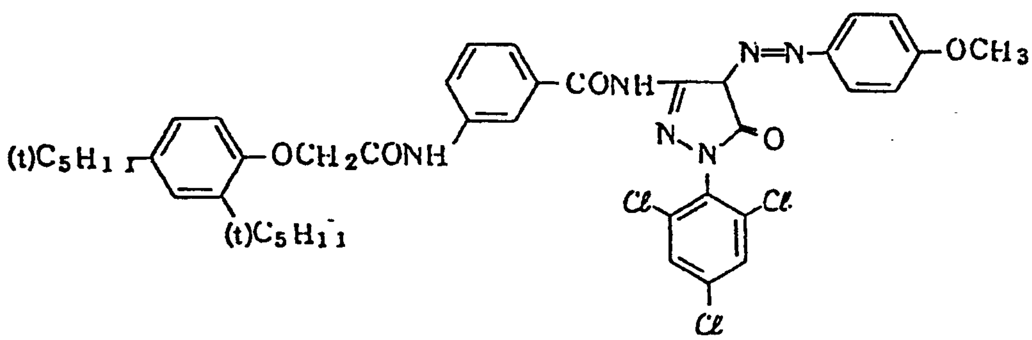

C-(24)

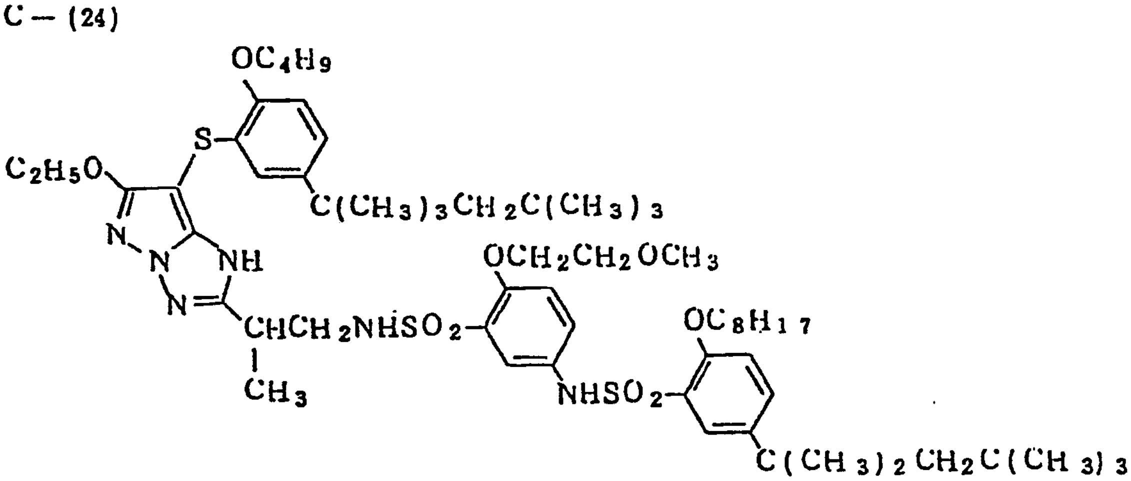

C — (25)

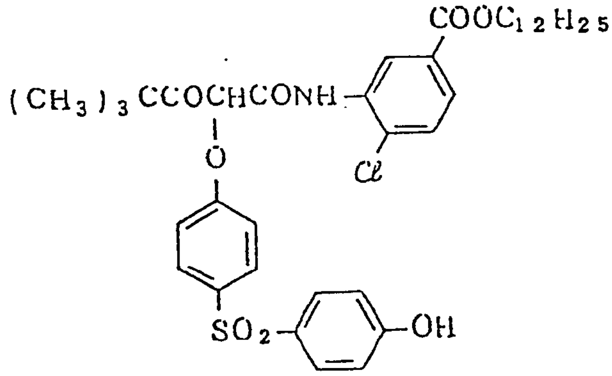

C — (26)

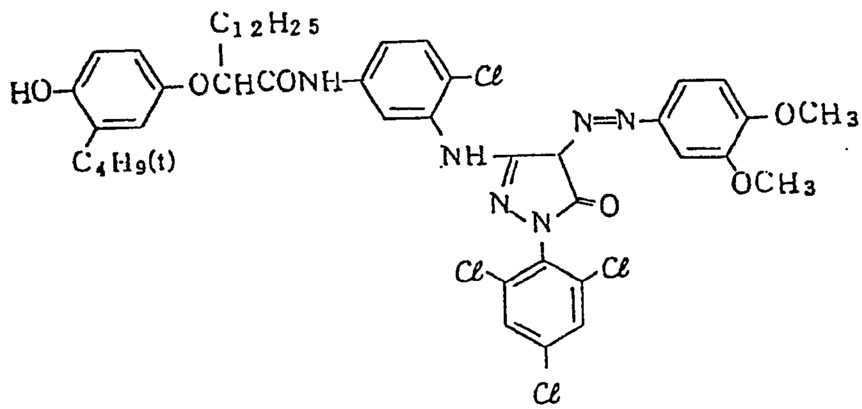

C — (27)

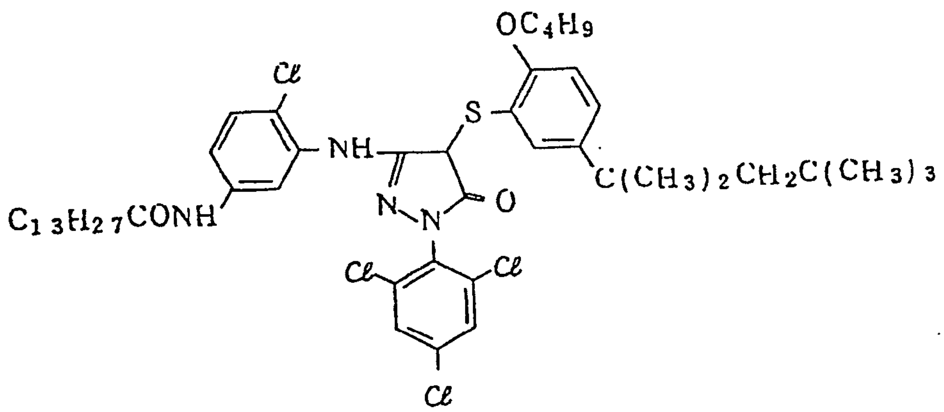

C — (28)

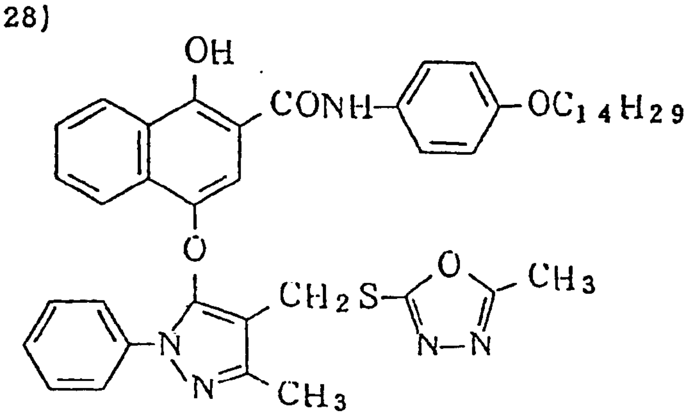

C — (29)

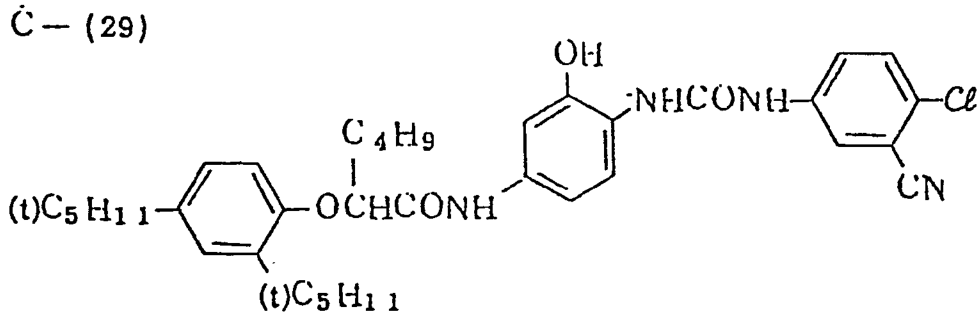

C — (30)

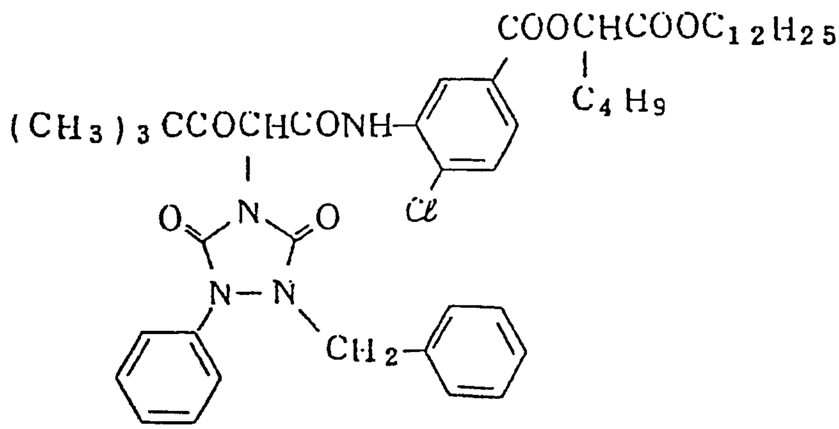

C — (31)

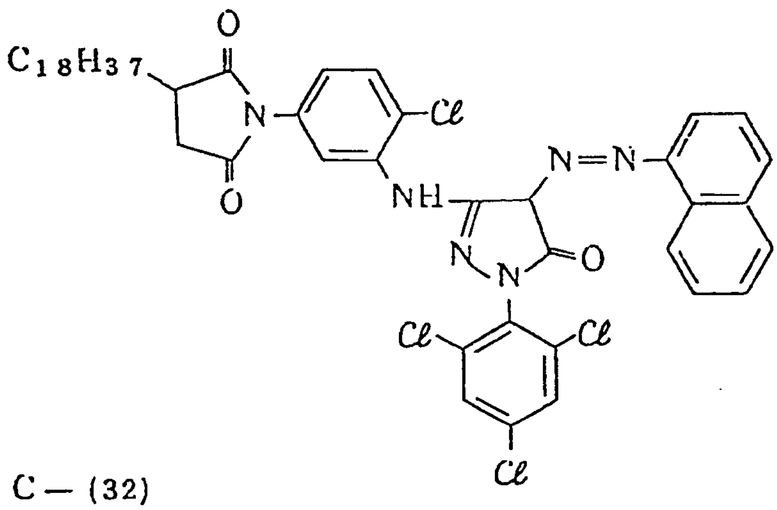

C — (32)

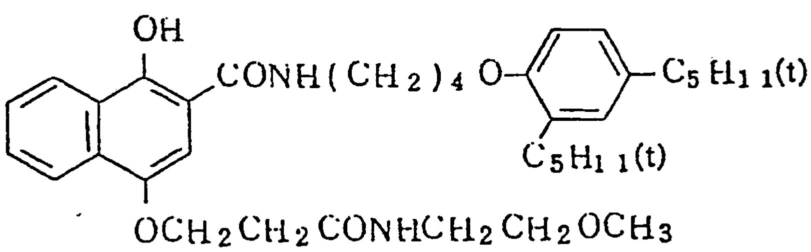

C — (33)

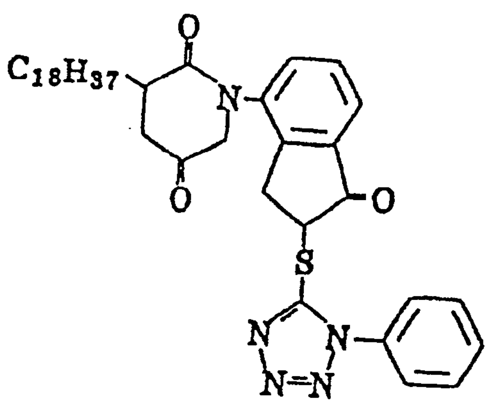

C — (34)

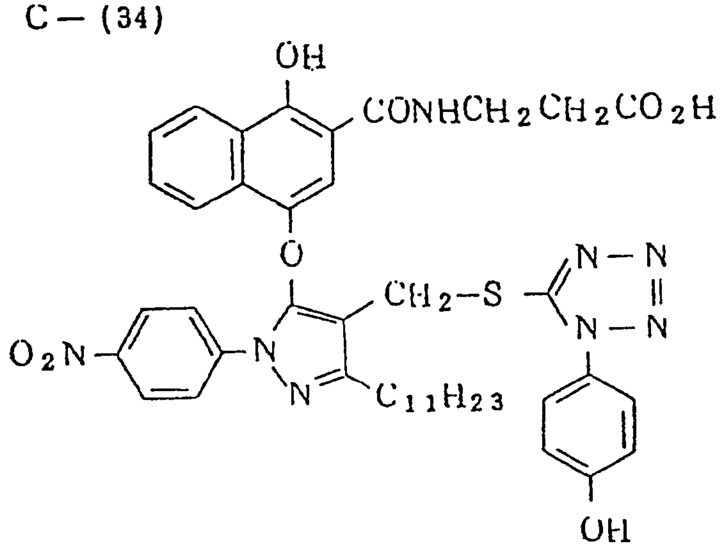

C — (35)

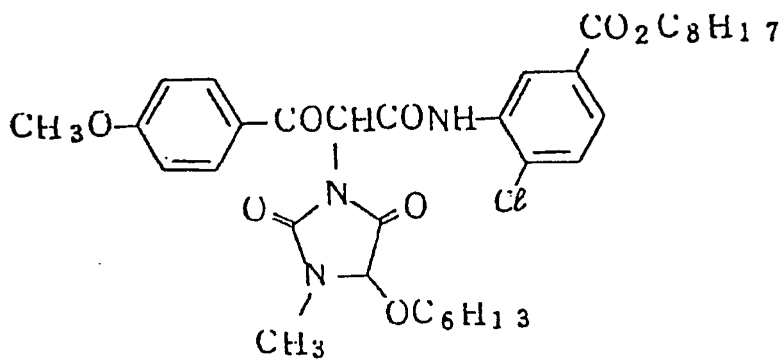

C — (36)

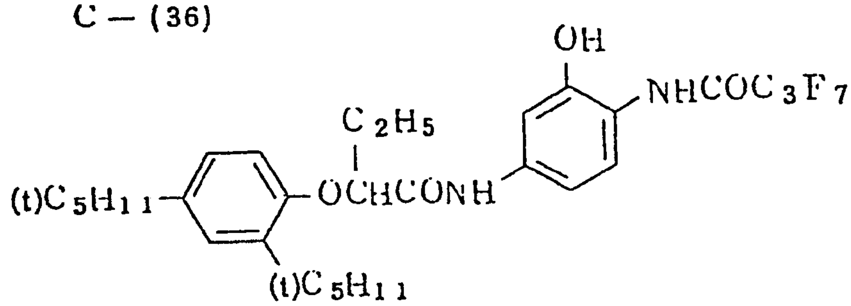

C — (37)

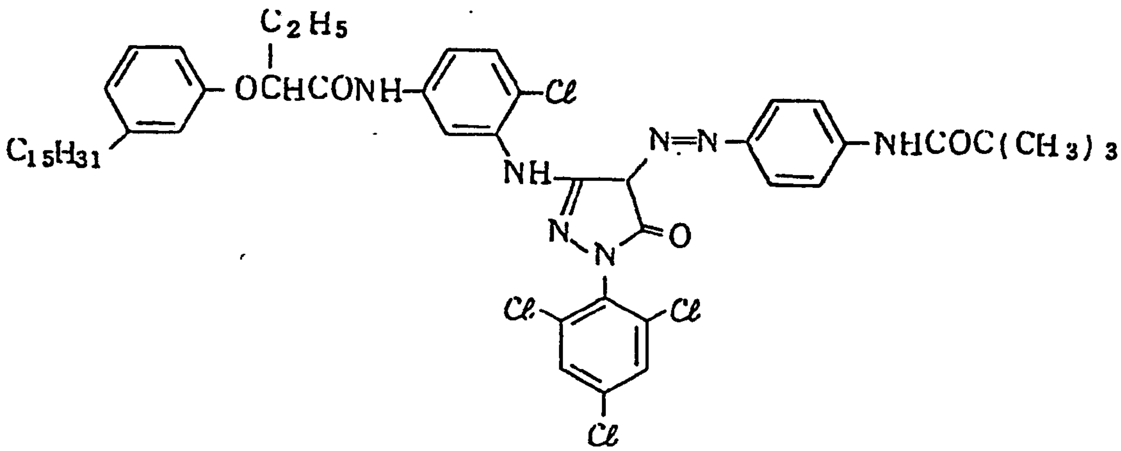

C — (38)

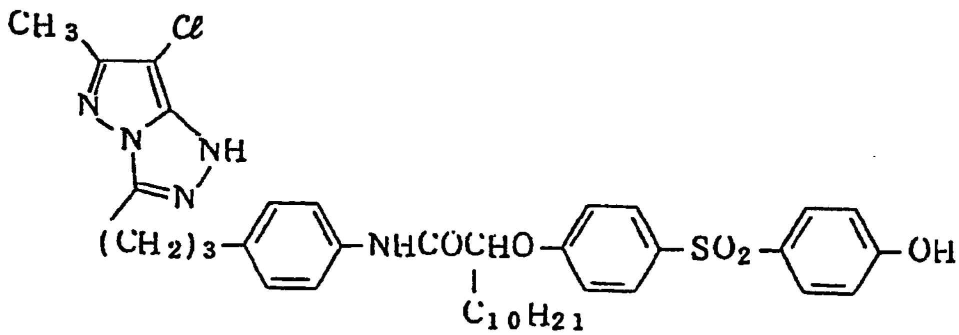

C — (39)

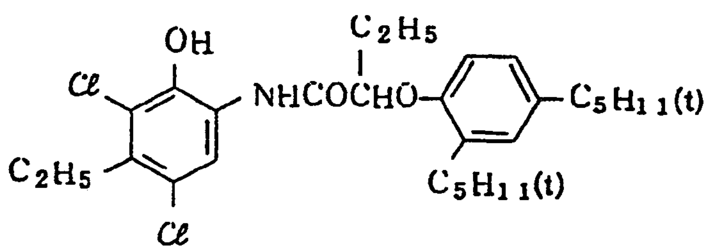

C — (40)

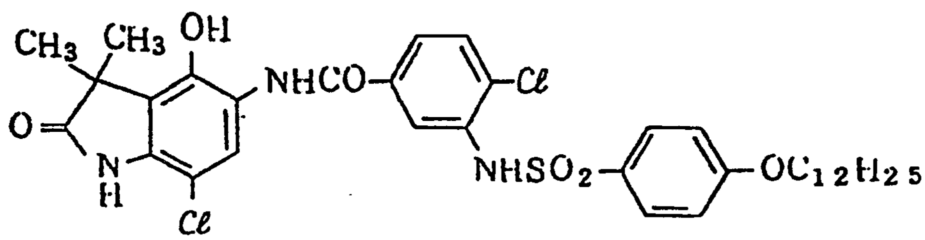

C－(41)

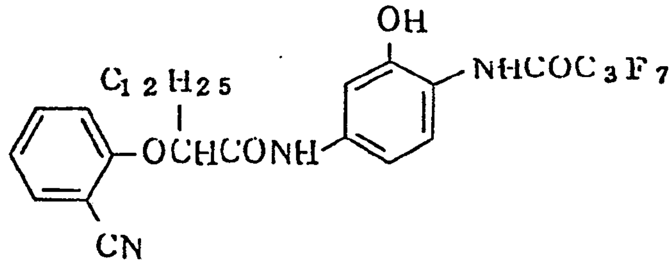

C－(42)

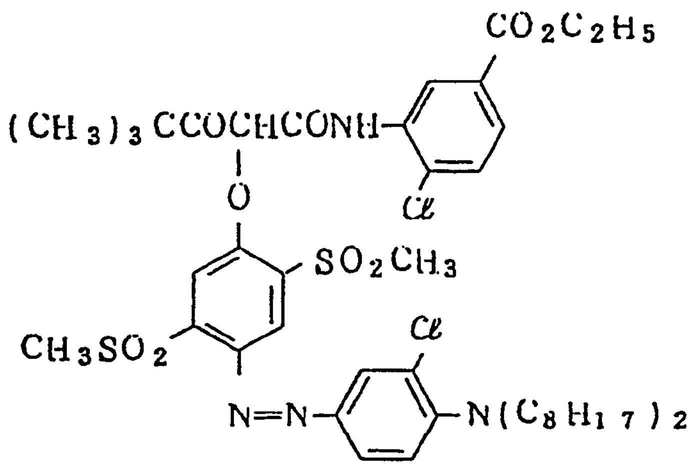

C－(43)

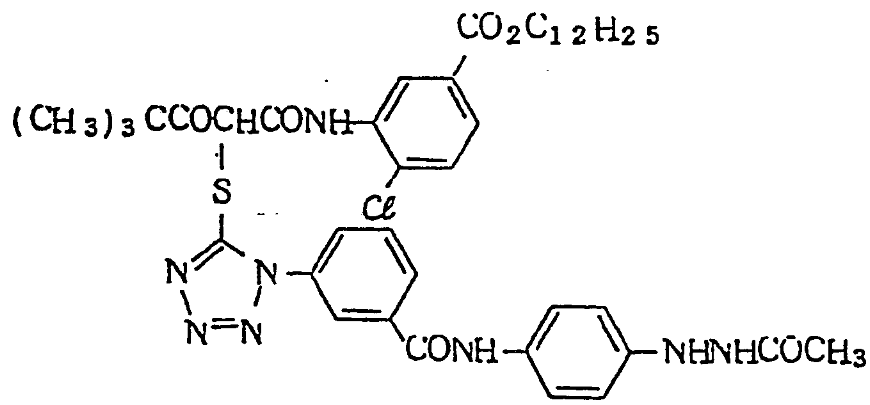

C- (44)

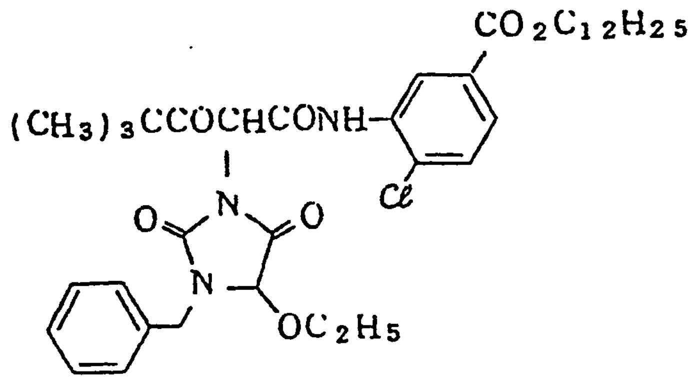

In the photographic emulsion layers of the photographic light-sensitive material used in the present invention, a preferably employed silver halide is silver iodobromide, silver iodochloride or silver iodochlorobromide each containing about 30 mol% or less of silver iodide. Silver iodobromide containing from about 2 mol% to about 25 mol% of silver iodide is particularly preferred.

Silver halide grains in the silver halide emulsion may have a regular crystal structure, for example, a cubic, octahedral or tetradecahedral structure, etc., an irregular crystal structure, for example, a spherical or tabular structure, etc., a crystal defect, for example, a twin plane, etc., or caqn be a composite structure thereof.

The particle size of the silver halide may be varied and includes from fine grains having about 0.2 micron or smaller than 0.2 micron diameter to large grains having up to about a 10 micron diameter as projected area. Further, a polydispersed emulsion and a monodispersed emulsion may be used.

The silver halide photographicemulsion used in the present invention can be prepared using known methods, for example, those as described in Research Disclosure, No. 17643 (December, 1978), pages 22 to 23, "I. Emulsion Preparation and Types" and ibid., No. 18716 (November, 1979), page 648, P. Glafkides, Chimie et Physique Photographique, Paul Montel (1967), G.F. Duffin, Photographic Emulsion Chemistry, The Focal Press (1966), and V.L. Zelikman et al., Making and Coating Photographic Emulsion, The Focal Press (1964), etc.

Monodispersed emulsions as described in U.S. Patents 3,574,628 and 3,655,394, British Patent 1,413,748, etc., are preferably used in the present invention.

Further, tabular silver halide grains having an aspect ratio of about 5 or more than 5 can be employed in the present invention. The tabular grains may be easily prepared by the method as described in Gutoff, Photographic Science and Engineering, Vol. 14, pages 248 to 257 (1970), U.S. Patents 4,434,226, 4,414,310, 4,433,048 and 4,439,520, British Patent 2,112,157, etc.

The crystal structure of the silver halide grains may be uniform or may be composed of different halide compositions between the inner portion and the outer portion, or may have a stratified structure.

Alternatively, silver halides emulsions in which silver halide grains having different compositions may be connected to each other by an epitaxial junction or by any suitable compound other than silver halide such as silver thiocyanate, lead oxide, etc.

Moreover, a mixture of grains having different crystal structures may be used.

The silver halide emulsions used in the present invention are usually formed with physical ripening, chemical ripening and spectral sensitization. Various kinds of additives which can be employed in these steps are described in Research Disclosure, No. 17643 (December, 1978) and ibid., No. 18716 (November, 1979) and relevant items are summarized in the table now shown.

Further, known photographic additives which can be used in the present invention are also described in the above literature and relevant items are summarized in the table below.

| | Kind of Additives | RD 17643 | RD 18716 |
|---|---|---|---|
| 1. | Chemical Sensitizers | Page 23 | Page 648, right column |
| 2. | Sensitivity Increasing Agents | | - ditto - |
| 3. | Spectral Sensitizers and Super Sensitizers | Pages 23 to 24 | Page 648, right column to page 649, right column |
| 4. | Whitening Agents | Page 24 | |
| 5. | Antifoggants and Stabilizers | Pages 24 to 25 | Page 649, right column |
| 6. | Light-Absobers, Filter Dyes and Ultraviolet Ray Absorbers | Pages 25 to 26 | Page 649, right column to page 650, left column |
| 7. | Antistaining Agents | Page 25, right column | Page 650, left column to right column |
| 8. | Dye Image Stabilizers | Page 25 | |
| 9. | Hardeners | Page 26 | Page 651, left column |
| 10. | Binders | Page 26 | - ditto - |
| 11. | Plasticizers and Lubricants | Page 27 | Page 650, right column |
| 12. | Coating Aids and Surfactants | Pages 26 to 27 | - ditto - |
| 13. | Antistatic Agents | Page 27 | - ditto - |

The compounds represented by general formula (I) and the couplers which can be used in the photographic light-sensitive material of the present invention can be introduced into the photographic light-sensitive material according to various known dispersing methods.

Suitable examples of organic solvent having a high boiling point which can be employed in an oil droplet-in-water type dispersing method are described in U.S. Patent 2,322,027, etc.

Processes and effects of latex dispersing methods and specific examples of latexes for loading are described, for example, in U.S. Patent 4,199,363, West German Patent Application (OLS) Nos. 2,541,274 and 2,541,230, etc.

Suitable supports which can be used in the present invention are described, for example, in Research Disclosure, No. 17643, page 28 and Research Disclosure, No. 18716, page 647, right column to page 648, left column, as mentioned above.

The color photographic light-sensitive material according to the present invention can be subjected to development processing in a conventional manner as described in Research Disclosure, No. 17643, pages 28 to 29 and Research Disclosure, No. 18716, page 651, left column to right column, as mentioned above.

After development, bleach-fixing or fixing, the silver halide color photographic material according to the present invention is usually subjected to a water washing process and/or a stabilizing process.

The amount of water to be used in the water washing step can be set in a broad range, in accordance with the characteristic of the photographic light-sensitive material which is processed (for example, depending upon the raw material components, such as coupler, etc.) or the use of the material, as well as the temperature of the washing water, the number of the washing tanks (the number of the washing stages), the replenishment system (countercurrent or parallelcurrent) and other various kinds of conditions. Among the above conditions, the relation between the number of the washing tanks and the amount of the washing water in a multi-stage countercurrent system can be obtained by the method described in Journal of the Society of Motion Picture and Television Engineers, Vol. 64, pages 248 to 253 (May, 1955).

According to the multi-stage countercurrent system described in the above publication, the amount of water to be used in the water washing step can be greatly reduced, however, bacteria would propagate in the tank because of the need to prolong the staying time of washing water in the tank so that some floating substances formed would adhere to the photographic material which is processed. This is a troublesome problem. As the means for preventing the propagation of bacteria, the method described in Japanese Patent Application (OPI) No. 288838/87, where calcium and magnesium are reduced, can extremely effectively be utilized in the practice of the present invention. Also, the isothiazolone compounds and the thiabendazoles described in Japanese Patent Application (OPI) No. 8542/82, the chlorine series bactericides such as the chlorinated sodium isocyanurates, benzotriazoles and other various bactericides or fungicides described in H. Horiguchi, Chemistry of Bactericides and Fungicides, Bactericidal and Fungicidal Technique to Microorganism, edited by Sanitary Technical Association (Japan) and Encyclopedia of Bactericides and Fungicides, edited by Japan Bactericidal and Fungicidal Association can also be used.

The pH of the washing water in the processing of the photographic material in accordance with the present invention is from 4 to 9, preferably 5 to 8. The temperature and time for the washing can be set variously in accordance with the characteristics of the photographic material which is processed, the use thereof, etc., and in general, it is from 15 to 45°C and 20 seconds to 10 minutes, and preferably from 25 to 40°C and 30 seconds to 5 minutes.

Further, the photographic material of the present invention can directly be processed with a stabilizer solution without taking the water washing step. When such the stabilization step is directly carried out, any and every known method, for example, the methods described in Japanese Patent Application (OPI) Nos. 8543/82, 14834/83, 184343/84, 220345/85, 238832/85, 239784/85, 239749/85, 4054/86 and 118749/86, etc. can be utilized. A stabilizer solution containing 1-hydroxyehtylidene-1,1-diphosphonic acid, 5-chloro-2-methyl-4-isothiazoline-3-one, bismuth compounds or ammonium compounds can preferably be used in the above described stabilization step.

After the water washing step described above, further stabilization step may be carried out. In this case, a stabilizer solution containing formalin and a surfactant, which is usually used as a final processing solution for a color photographic material for photography.

When the photographic light-sensitive material of the present invention is a black-and-white silver halide photographic material, it can be subjected to development processing according to methods as described in Research Disclosure, Vol. 176, No. 17643, pages 28 to 29 and Research Disclosure, Vol. 187, No. 18716, page 651, left column to right column.

Specific examples of color photographic light-sensitive materials to which the photographic light-sensitive material of the present invention can be applied include color negative films, color reversal films, color paper, color reversal paper, color negative films for cinematography, color positive films for cinematography, etc. The present invention is preferably utilized in color negative films and color reversal films.

Specific examples of black-and-white photographic light-sensitive materials to which the photographic light-sensitive material of the present invention can be applied include black-and-white negative light-sensitive materials, light-sensitive materials for X-ray use, light-sensitive materials for lithography, black-and-white printing paper, etc. The present invention is preferably utilized in black-and-white negative light-sensitive materials.

The present invention is described in detail with reference to the following examples, but the present invention is not to be construed as being limited thereto.

## EXAMPLE 1

### Sample 101 :

On a cellulose triacetate film support provided with a conventional subbing layer were coated layers having the compositions set forth below to prepare a multilayer color photographic light-sensitive material, which was designated as Sample 101.

With respect to the compositions of the layers, unless otherwise stated, coated amounts are shown in units

of g/m$^2$ (dry thickness), coated amounts of silver halide and colloidal silver are shown as the silver coated amount in units of g/m$^2$ and sensitizing dyes are shown as a molar amount per mol of silver halide present in the layer.

<u>First Layer</u>: Antihalation Layer

| | |
|---|---|
| Black colloidal silver | 0.18 (as silver) |
| Gelatin | 0.40 |

<u>Second Layer</u>: Intermediate Layer

| | |
|---|---|
| 2,5-Di-tert-pentadecylhydroquinone | 0.18 |
| EX-1 | 0.07 |
| EX-3 | 0.02 |
| U-1 | 0.08 |
| U-2 | 0.08 |
| HBS-1 | 0.10 |
| HBS-2 | 0.02 |
| Gelatin | 1.04 |

<u>Third Layer</u>: First Red-Sensitive Emulsion Layer

| | |
|---|---|
| Silver iodobromide emulsion (silver iodide: 6 mol%, average particle size: 0.6 μm) | 0.55 (as silver) |
| Sensitizing dye I | $6.9 \times 10^{-5}$ |
| Sensitizing dye II | $1.8 \times 10^{-5}$ |
| Sensitizing dye III | $3.1 \times 10^{-4}$ |

| | |
|---|---|
| Sensitizing dye IV | $4.0 \times 10^{-5}$ |
| EX-2 | 0.350 |
| HBS-1 | 0.005 |
| EX-10 | 0.020 |
| Gelatin | 1.20 |

Fourth Layer: Second Red-Sensitive Emulsion Layer

| | |
|---|---|
| Silver iodobromide emulsion (silver iodide: 8 mol%, average particle size: 0.8 μm) | 1.0 (as silver) |
| Sensitizing dye I | $5.1 \times 10^{-5}$ |
| Sensitizing dye II | $1.4 \times 10^{-5}$ |
| Sensitizing dye III | $2.3 \times 10^{-4}$ |
| Sensitizing dye IV | $3.0 \times 10^{-5}$ |
| EX-2 | 0.300 |
| EX-3 | 0.050 |
| EX-10 | 0.015 |
| HBS-2 | 0.050 |
| Gelatin | 1.30 |

Fifth Layer: Third Red-Sensitive Emulsion Layer

| | |
|---|---|
| Silver iodobromide emulsion (silver iodide: 1 mol%, average particle size: 1.1 μm) | 1.60 (as silver) |
| Sensitizing dye IX | $5.4 \times 10^{-5}$ |
| Sensitizing dye II | $1.4 \times 10^{-5}$ |
| Sensitizing dye III | $2.4 \times 10^{-4}$ |
| Sensitizing dye IV | $3.1 \times 10^{-5}$ |
| EX-5 | 0.150 |
| EX-3 | 0.055 |

| | |
|---|---|
| EX-4 | 0.060 |
| HBS-1 | 0.32 |
| Gelatin | 1.63 |

Sixth Layer: Intermediate Layer

| | |
|---|---|
| EX-11 | 0.055 |
| Gelatin | 1.06 |
| HBS-2 | 0.025 |

Seventh Layer: First Green-Sensitive Emulsion Layer

| | |
|---|---|
| Silver iodobromide emulsion (silver iodide: 6 mol%, average particle size: 0.6 µm) | 0.40 (as silver) |
| Sensitizing dye V | $3.0 \times 10^{-5}$ |
| Sensitizing dye VI | $1.0 \times 10^{-4}$ |
| Sensitizing dye VII | $3.8 \times 10^{-4}$ |
| EX-6 | 0.260 |
| EX-1 | 0.021 |
| EX-7 | 0.030 |
| EX-8 | 0.025 |
| HBS-1 | 0.100 |
| HBS-4 | 0.05 |
| Gelatin | 0.75 |

Eighth Layer: Second Green-Sensitive Emulsion Layer

| | |
|---|---|
| Silver iodobromide emulsion (silver iodide: 9 mol%, average particle size: 0.7 µm) | 0.80 (as silver) |
| Sensitizing dye V | $2.1 \times 10^{-5}$ |
| Sensitizing dye VI | $7.0 \times 10^{-5}$ |
| Sensitizing dye VII | $2.6 \times 10^{-4}$ |

| | |
|---|---|
| EX-6 | 0.150 |
| EX-8 | 0.010 |
| EX-1 | 0.008 |
| EX-7 | 0.012 |
| HBS-1 | 0.60 |
| HBS-4 | 0.05 |
| Gelatin | 1.10 |

Ninth Layer: Third Green-Sensitive Emulsion Layer

| | |
|---|---|
| Silver iodobromide emulsion (silver iodide: 12 mol%, average particle size: 1.0 μm) | 1.2 (as silver) |
| Sensitizing dye V | $3.5 \times 10^{-5}$ |
| Sensitizing dye VI | $8.0 \times 10^{-5}$ |
| Sensitizing dye VII | $3.0 \times 10^{-4}$ |
| EX-6 | 0.065 |
| EX-1 | 0.025 |
| HBS-4 | 0.03 |
| HBS-2 | 0.55 |
| Gelatin | 1.74 |

Tenth Layer: Yellow Filter Layer

| | |
|---|---|
| Yellow colloidal silver | 0.05 (as silver) |
| EX-11 | 0.085 |
| HBS-1 | 0.030 |
| Gelatin | 0.95 |

Eleventh Layer: First Blue-Sensitive Emulsion Layer

| | |
|---|---|
| Silver iodobromide emulsion (silver iodide: 6 mol%, average particle size: 0.6 μm) | 0.24 (as silver) |

| | |
|---|---|
| Sensitizing dye VIII | $3.5 \times 10^{-4}$ |
| EX-9 | 0.85 |
| EX-8 | 0.12 |
| HBS-1 | 0.28 |
| Gelatin | 1.28 |

Twelfth Layer: Second Blue-Sensitive Emulsion Layer

| | |
|---|---|
| Silver iodobromide emulsion (silver iodide: 10 mol%, average particle size: 0.8 µm) | 0.45 (as silver) |
| Sensitizing dye VIII | $2.1 \times 10^{-4}$ |
| EX-9 | 0.20 |
| EX-8 | 0.015 |
| HBS-1 | 0.03 |
| Gelatin | 0.46 |

Thirteenth Layer: Third Blue-Sensitive Emulsion Layer

| | |
|---|---|
| Silver iodobromide emulsion (silver iodide: 1 mol%, average particle size: 1.3 µm) | 0.77 (as silver) |
| Sensitizing dye VIII | $2.2 \times 10^{-4}$ |
| EX-9 | 0.20 |
| HBS-1 | 0.07 |
| Gelatin | 0.69 |

Fourteenth Layer: First Protective Layer

| | |
|---|---|
| Silver iodobromide emulsion (silver iodide: 1 mol%, average particle size: 0.07 µm) | 0.5 (as silver) |
| U-1 | 0.11 |
| U-2 | 0.17 |
| HBS-1 | 0.90 |

| | |
|---|---|
| Gelatin | 1.00 |

Fifteenth Layer: Second Protective Layer

| | |
|---|---|
| Polymethyl acrylate particle (diameter: about 1.5 μm) | 0.54 |
| S-1 | 0.05 |
| S-2 | 0.20 |
| Gelatin | 0.72 |

Gelatin Hardener H-1 and a surface active agent were added to each of the layers in addition to the above described components.

Samples 102 and 108 :

Samples 102 and 108 were prepared in the same manner as described for Sample 101, except that EX-10 added to the third layer and the fourth layer was substituted with the compound as shown in Table 1 below in an amount so as to obtain substantially the same sensitivity and gradation.

These samples were uniformly exposed through a green filter BPN-53 manufactured by Fuji Photo Film Co., Ltd., at a color temperature of 4,800°K with an amount of 0.1 lux·sec. and then imagewise exposed through a red filter SC-62 manufactured by the Fuji Photo Film Co., Ltd., and thereafter subjected to color development processing according to the steps shown below. With the samples thus processed, magenta density at an exposure amount required to obtain a cyan density of 1.5 and a magenta density at the exposure amount required for obtaining a cyan fog density were measured, and the value obtained by subtracting the latter from the former was determined. The value was designated the degree of color contamination and is shown in Table 1 below.

Further, these samples were exposed to white light through a pattern for MTF measurement and subjected to color development processing with these samples MTF values of cyan image and magenta image were measured. The results thus-obtained are also shown in Table 1 below.

The color development processing was carried out according to the processing steps set forth below at a processing temperature of 38°C.

| Processing Step | Time |
|---|---|
| Color Development | 3 min. 15 sec. |
| Bleaching | 6 min. 30 sec. |
| Washing with Water | 2 min. 10 sec. |
| Fixing | 4 min. 20 sec. |
| Washing with Water | 3 min. 15 sec. |
| Stabilizing | 1 min. 05 sec. |

The composition of a processing solution used in each step is given below.

Color Developing Solution:

| | |
|---|---|
| Diethylenetriaminepentaacetic acid | 1.0 g |
| 1-Hydroxyethylidene-1,1-diphosphonic acid | 2.0 g |
| Sodium sulfite | 4.0 g |
| Potassium carbonate | 30.0 g |
| Potassium bromide | 1.4 g |
| Potassium iodide | 1.3 mg |
| Hydroxylamine sulfate | 2.4 g |
| 4-(N-Ethyl-N-β-hydroxyethylamino)-2-methylaniline sulfate | 4.5 g |
| Water to make | 1.0 liter |
| pH | 10.0 |

Bleaching Solution:

| | |
|---|---|
| Iron (III) ammonium ethylenediaminetetraacetate | 100.0 g |
| Disodium ethylenediaminetetraacetate | 10.0 g |
| Ammonium bromide | 150 g |
| Ammonium nitrate | 10.0 g |
| Water to make | 1.0 liter |
| pH | 6.0 |

Fixing Solution:

| | |
|---|---|
| Disodium ethylenediaminetetra-acetate | 1.0 g |
| Sodium sulfite | 4.0 g |
| Ammonium thiosulfate (70% aq. soln.) | 175.0 mℓ |
| Sodium bisulfite | 4.6 g |
| Water to make | 1.0 liter |
| pH | 6.6 |

Stabilizing Solution:

| | |
|---|---|
| Formalin (40 wt%) | 2.0 ml |
| Polyoxyethylene-p-monononyl-phenylether (average degree of polymerization: 10) | 0.3 g |
| Water to make | 1.0 liter |

TABLE 1

| Sample | Compound in Third Layer and Fourth Layer | Amount Added in Molar Ratio* | Degree of Color Contamination | MTF Value Cyan Image 25 cycles | MTF Value Magenta Image 25 cycles |
|---|---|---|---|---|---|
| 101 (Comparison) | EX-10 | 1 | +0.05 | 0.38 | 0.59 |
| 102 ( " ) | EX-8 | 1.5 | -0.01 | 0.39 | 0.61 |
| 103 ( " ) | EX-12 | 0.7 | +0.02 | 0.38 | 0.59 |
| 104 ( " ) | EX-13 | 1.5 | +0.04 | 0.38 | 0.59 |
| 105 ( " ) | EX-14 | 1.9 | -0.03 | 0.41 | 0.62 |
| 106 (Present Invention) | (1)** | 2.5 | -0.10 | 0.44 | 0.65 |
| 107 ( " ) | (3)** | 2.0 | -0.09 | 0.43 | 0.64 |
| 108 ( " ) | (4)** | 1.8 | -0.09 | 0.43 | 0.64 |

*: based on the amount of EX-10
**: Those compounds have been earlier identified in the group of Compounds (1)-(21)

From the results shown in Table 1 it can be seen that the samples in which the compounds according to the present invention were used were excellent in color reproducibility as indicated by the degree of color contamination and sharpness as indicated by the MTF value.

The chemical structure or chemical names of the compounds employed in Example 1 are shown below.

U-1

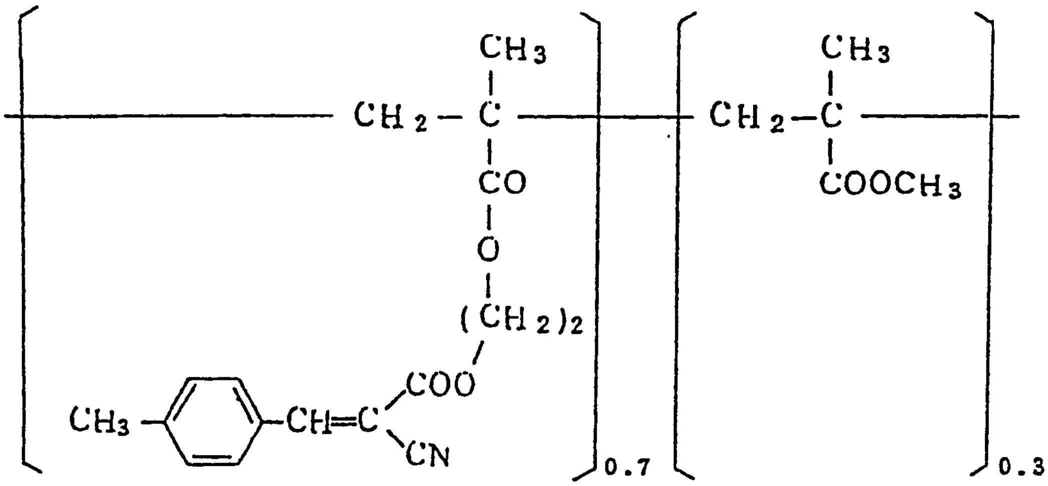

U-2

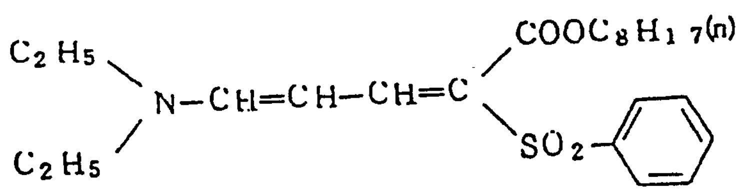

EX-1 (same as C-(23) which has been earlier identified)

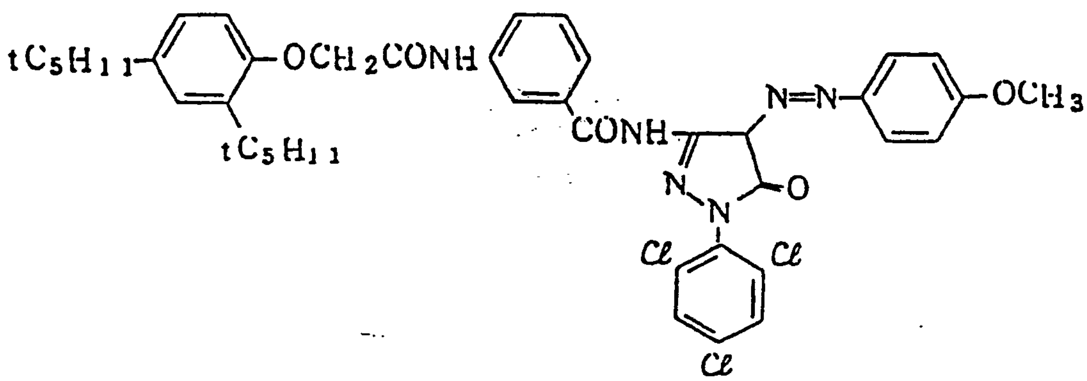

EX-2 (same as C-(15) which has been earlier identified)

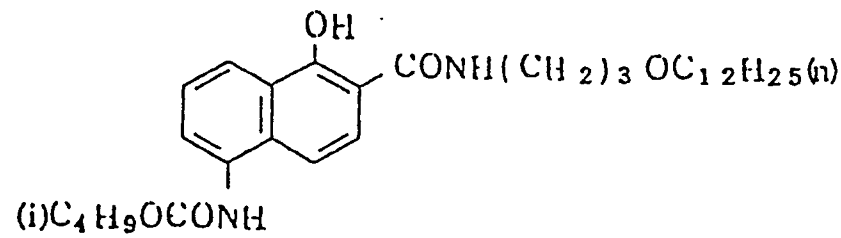

EX-3 (same as C-(19) which has been earlier identified)

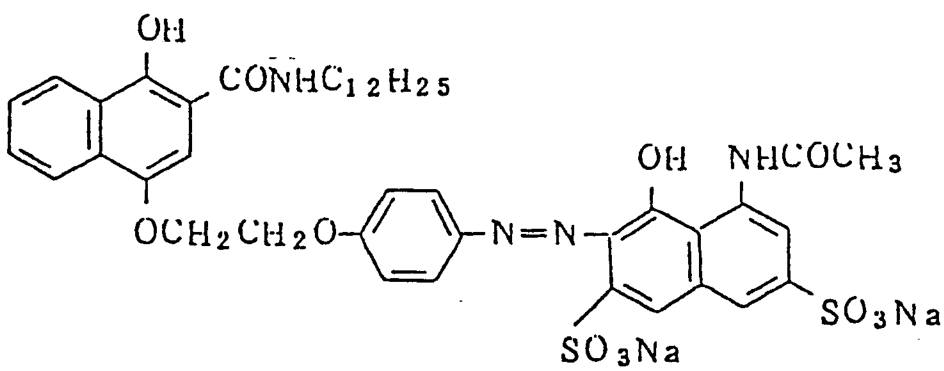

EX-4 (same as C-(14) which has been earlier identified)

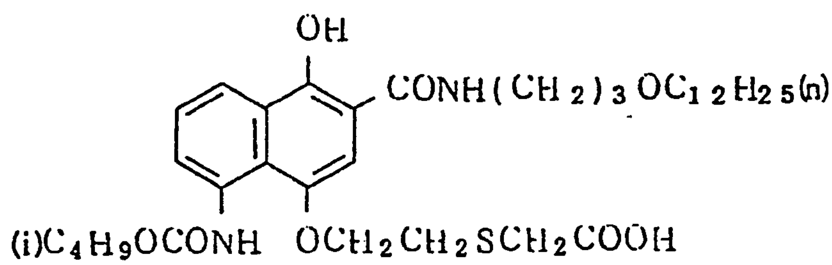

EX-5 (same as C-(12) which has been earlier identified)

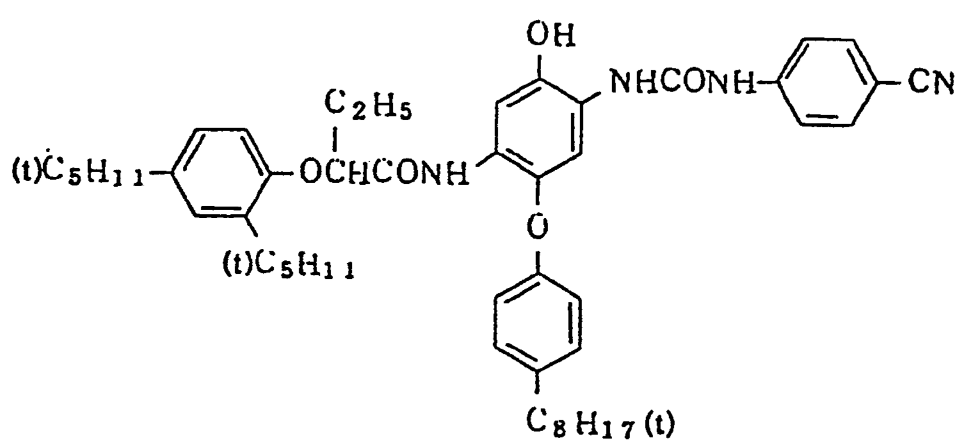

EX-6 (same as C-(7) which has been earlier identified)

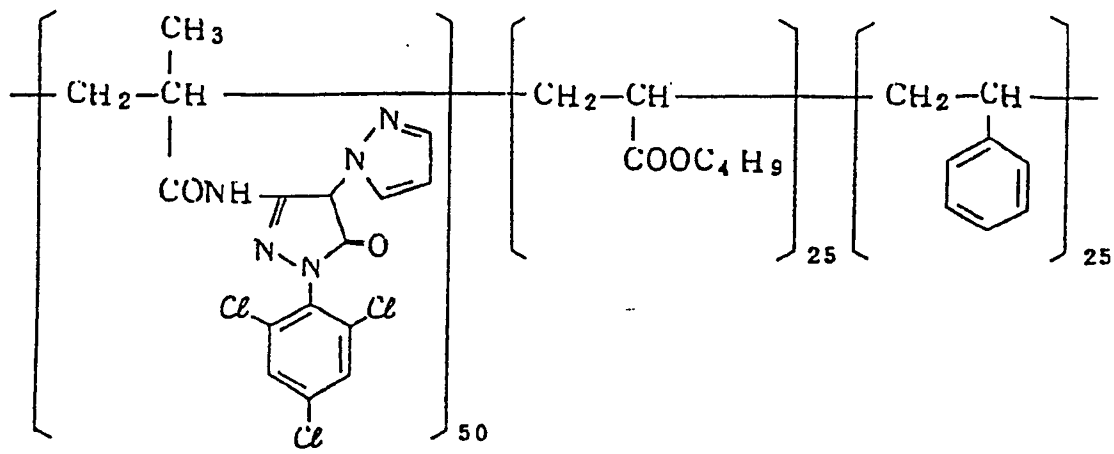

[average molecular weight: 30,000]

EX-7 (same as C-(37) which has been earlier identified)

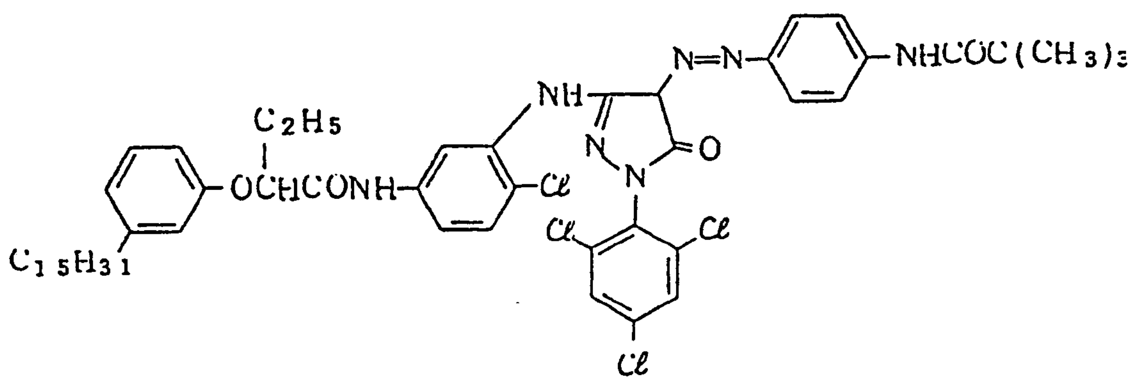

EX-8 (coupler as described in U.S. Patent 4,477,563)

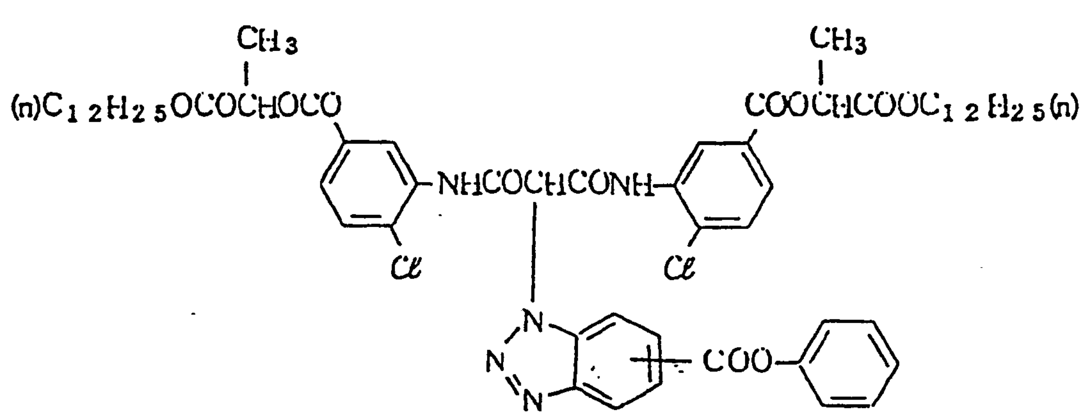

EX-9 (same as C-(2) which has been earlier identified)

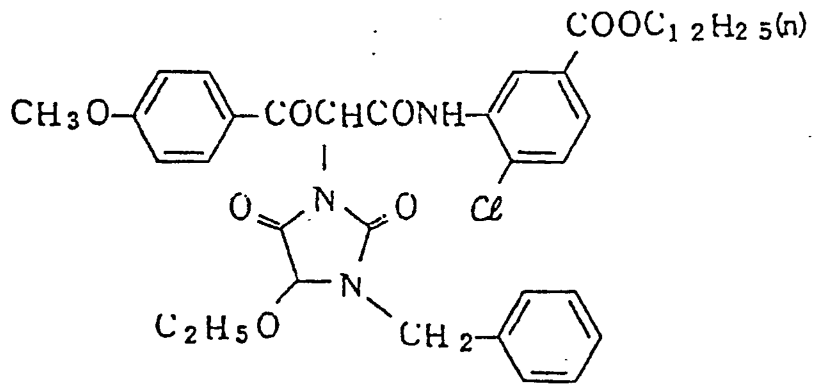

EX-10 (coupler as described in U.S. Patent 3,227,554)

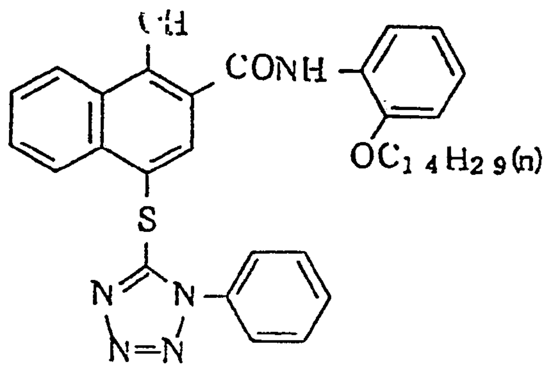

EX-11

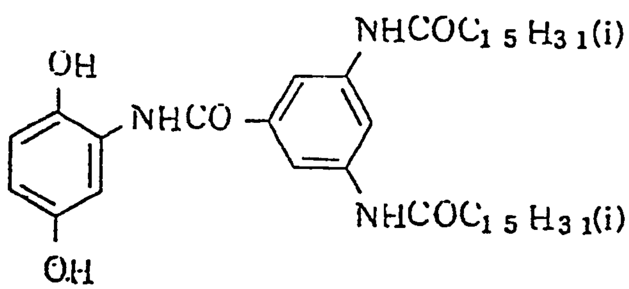

EX-12 (coupler as described in U.S. Patent 4,248,962)

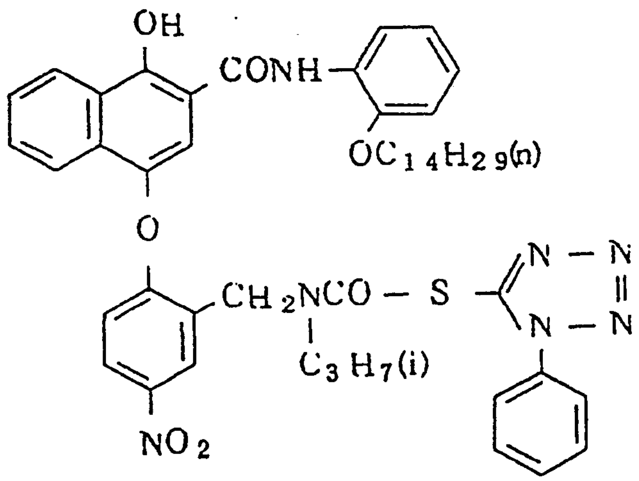

EX-13 (coupler as described in British Patent 1,400,149)

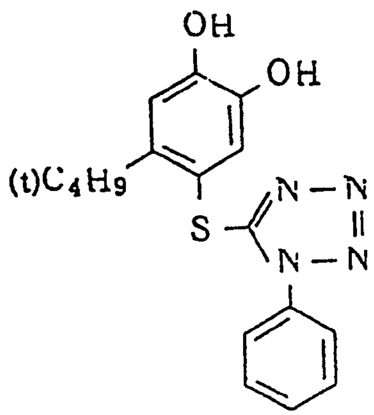

EX-14 (coupler as described in Japanese Patent Application (OPI) No. 185950/85)

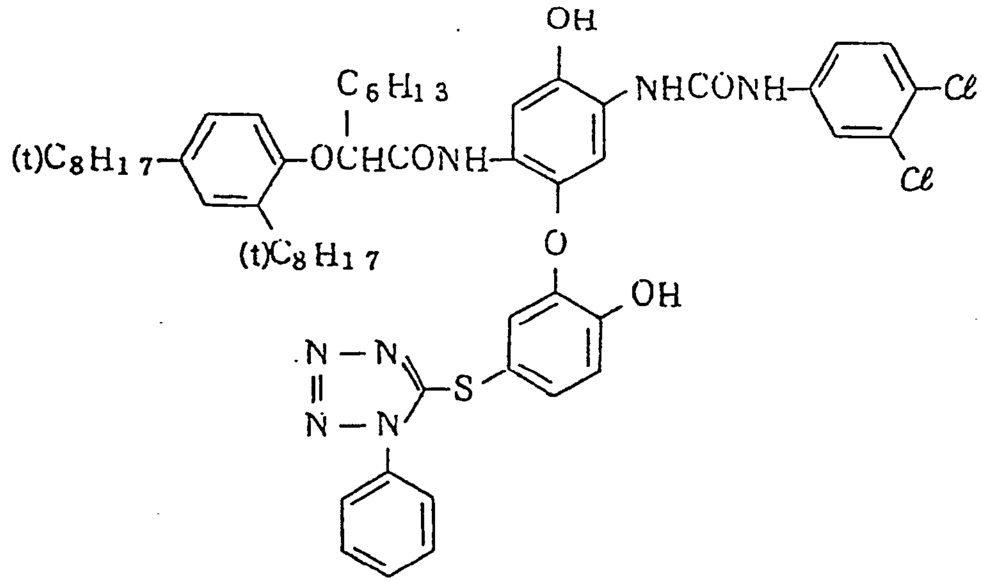

S-1

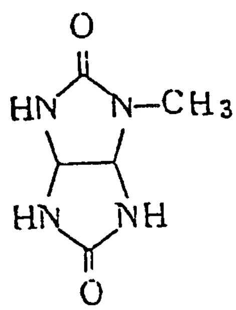

S-2

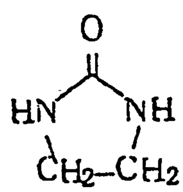

| | |
|---|---|
| HBS-1 | Tricresyl phosphate |
| HBS-2 | Dibutyl phthalate |
| HBS-3 | Bis(2-ethylhexylphosphate) |
| HBS-4 | |

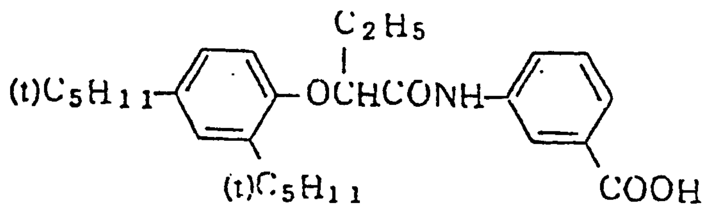

H-1

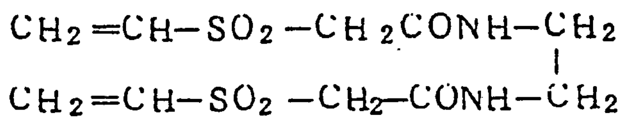

Sensitizing Dye I

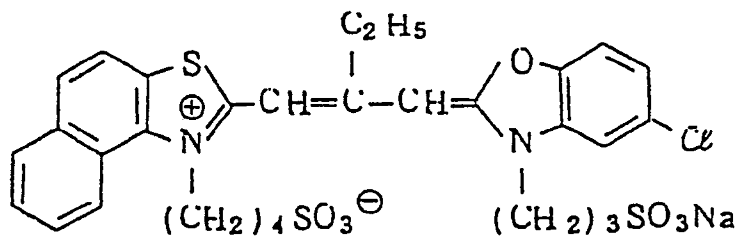

Sensitizing Dye II

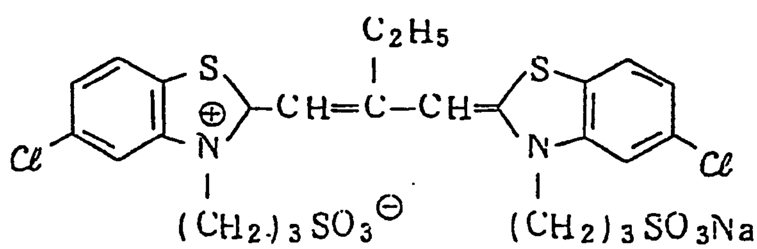

Sensitizing Dye III

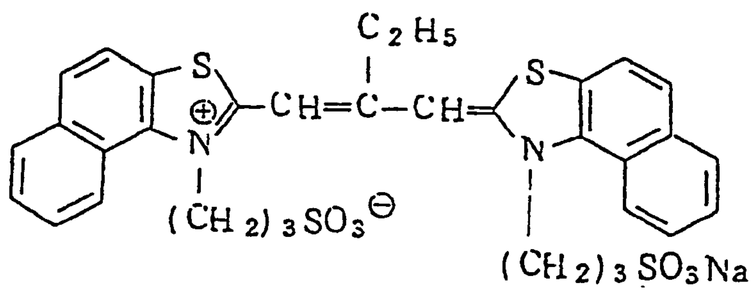

Sensitizing Dye IV

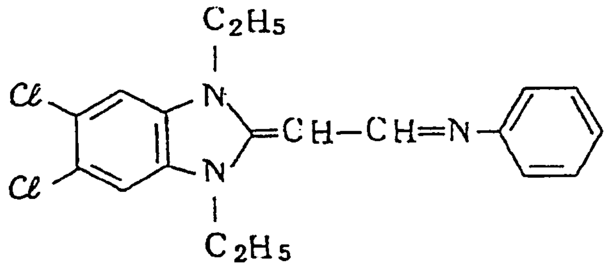

Sensitizing Dye V

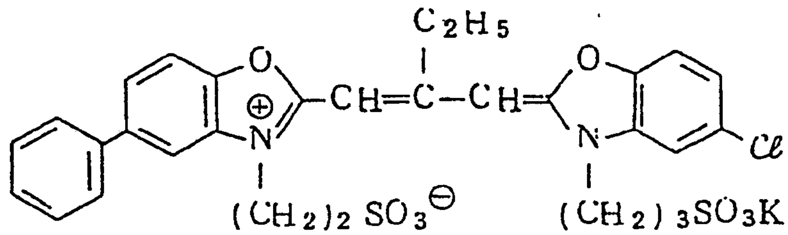

Sensitizing Dye VI

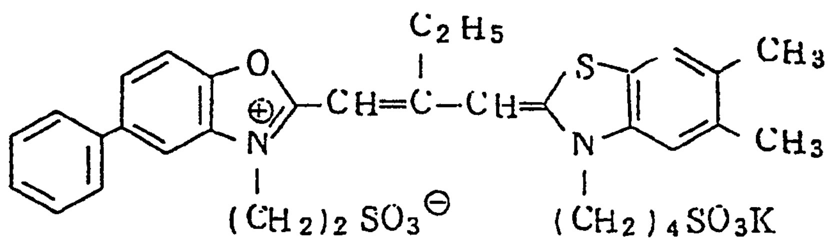

Sensitizing Dye VII

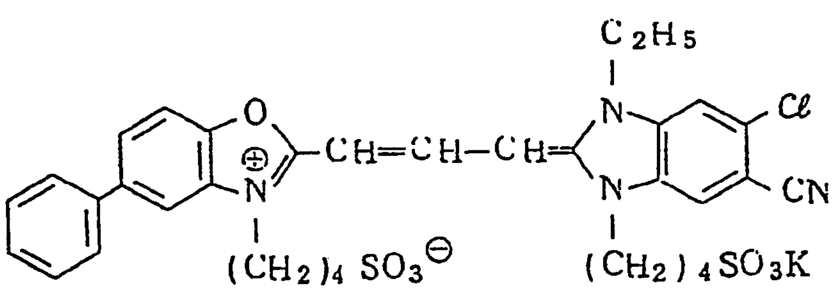

Sensitizing Dye VIII

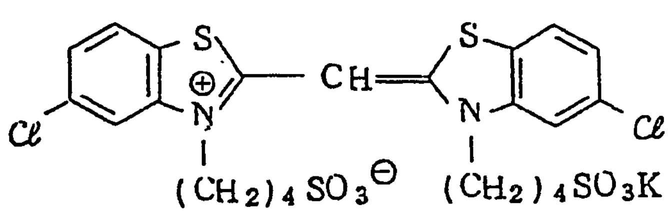

Sensitizing Dye IX

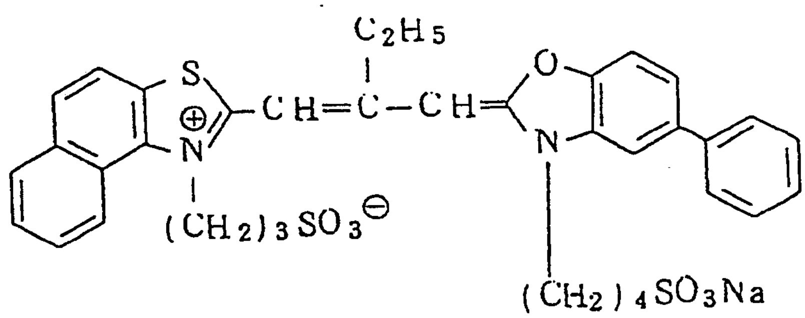

EXAMPLE 2

On a cellulose triacetate film support having a subbing layer, each layer having the composition shown below was coated to prepare a multilayer color photographic light-sensitive material, which was designated Sample 201. Coating basis was as explained for Example 1 unless otherwise indicated.

First Layer: Antihalation Layer

A gelatin layer (dry layer thickness of 2 μm) containing;

| | |
|---|---|
| Black colloidal silver | 0.25 $g/m^2$ |
| Ultraviolet ray absorbing agent U-1 | 0.04 $g/m^2$ |
| Ultraviolet ray absorbing agent U-2 | 0.1 $g/m^2$ |
| Ultraviolet ray absorbing agent U-3 | 0.1 $g/m^2$ |
| High boiling point organic solvent Oil-2 | 0.01 $ml/m^2$ |

Second Layer: Intermediate Layer

A gelatin layer (dry layer thickness of 1 μm) containing;

| | |
|---|---|
| Compound Cpd C | 0.05 $g/m^2$ |
| Compound (13) | 0.05 $g/m^2$ |
| High boiling point organic solvent Oil-1 | 0.05 $ml/m^2$ |

Third Layer: First Red-Sensitive Emulsion Layer

A gelatin layer (dry layer thickness of 1 μm) containing;

| | |
|---|---|
| Silver iodobromide emulsion (iodide content: 4 mol%, average particle size: 0.3 μm) spectrally sensitized with sensitizing dye S-1 and sensitizing dye S-2 | 0.5 g/m² (as silver) |
| Coupler F-1 | 0.25 g/m² |
| Compound I-2 | 2 × $10^{-3}$ g/m² |
| High boiling point organic solvent Oil-1 | 0.025 ml/m² |

Fourth Layer: Second Red-Sensitive Emulsion Layer

A gelatin layer (dry layer thickness of 2.5 μm) containing;

| | |
|---|---|
| Silver iodobromide emulsion (iodide content: 3 mol%, average particle size: 0.6 μm) spectrally sensitized with sensitizing dye S-1 and sensitizing dye S-2 | 0.8 g/m² (as silver) |
| Coupler F-1 | 0.70 g/m² |
| Compound I-2 | 1 × $10^{-3}$ g/m² |
| High boiling point organic solvent Oil-1 | 0.33 ml/m² |
| Dye D-1 | 0.02 g/m² |

Fifth Layer: Intermediate Layer

A gelatin layer (dry layer thickness of 1 μm) containing;

| | |
|---|---|
| Compound Cpd C | 0.1 g/m² |
| High boiling point organic solvent Oil-1 | 0.1 ml/m² |
| Dye D-2 | 0.02 g/m² |

Sixth Layer: First Green-Sensitive Emulsion Layer

A gelatin layer (dry layer thickness of 1 μm) containing;

| | |
|---|---|
| Silver iodobromide emulsion (iodide content: 4 mol%, average particle size: 0.3 μm) spectrally sensitized with sensitizing dye S-3 and sensitizing dye S-4 | 0.7 $g/m^2$ (as silver) |
| Coupler F-2 | 0.20 $g/m^2$ |
| Coupler F-4 | 0.10 $g/m^2$ |
| High boiling point organic solvent Oil-1 | 0.26 $ml/m^2$ |

Seventh Layer: Second Green-Sensitive Emulsion Layer

A gelatin layer (dry layer thickness of 2.5 μm) containing;

| | |
|---|---|
| Silver iodobromide emulsion (iodide content: 2.5 mol%, average particle size: 0.6 μm) spectrally sensitized with sensitizing dye S-3 and sensitizing dye S-4 | 0.7 $g/m^2$ (as silver) |
| Coupler F-3 | 0.10 $g/m^2$ |
| Coupler F-4 | 0.10 $g/m^2$ |
| High boiling point organic solvent Oil-2 | 0.05 $ml/m^2$ |
| Dye D-3 | 0.05 $g/m^2$ |

Eighth Layer: Intermediate Layer

A gelatin layer (dry layer thickness of 1 μm) containing;

| | |
|---|---|
| Compound Cpd C | 0.05 $g/m^2$ |
| High boiling point organic solvent Oil-2 | 0.1 $ml/m^2$ |

| | |
|---|---|
| Dye D-4 | 0.01 g/m$^2$ |

<u>Ninth Layer</u>: Yellow Filter Layer

A gelatin layer (dry layer thickness of 1 μm) containing;

| | |
|---|---|
| Yellow colloidal silver | 0.1 g/m$^2$ |
| Compound Cpd C | 0.02 g/m$^2$ |
| High boiling point organic solvent Oil-1 | 0.04 ml/m$^2$ |

<u>Tenth Layer</u>: First Blue-Sensitive Emulsion Layer

A gelatin layer (dry layer thickness of 1.5 μm) containing;

| | |
|---|---|
| Silver iodobromide emulsion (iodide content: 2 mol%, average particle size: 0.3 μm) spectrally sensitized with sensitizing dye S-5 | 0.6 g/m$^2$ (as silver) |
| Coupler F-5 | 0.1 g/m$^2$ |
| Coupler F-6 | 0.4 g/m$^2$ |
| High boiling point organic solvent Oil-1 | 0.1 ml/m$^2$ |

<u>Eleventh Layer</u>: Second Blue-Sensitive Emulsion Layer

A gelatin layer (dry layer thickness of 3 μm) containing;

| | |
|---|---|
| Silver iodobromide emulsion (iodide content: 2 mol%, average particle size: 0.6 μm) spectrally sensitized with sensitizing dye S-6 | 1.1 g/m$^2$ (as silver) |
| Coupler F-5 | 0.4 g/m$^2$ |
| Coupler F-7 | 0.8 g/m$^2$ |

| | |
|---|---|
| High boiling point organic solvent Oil-1 | 0.23 ml/m$^2$ |
| Dye D-5 | 0.02 g/m$^2$ |

Twelfth Layer: First Protective Layer

A gelatin layer (dry layer thickness of 2 μm) containing;

| | |
|---|---|
| Ultraviolet ray absorbing agent U-1 | 0.02 g/m$^2$ |
| Ultraviolet ray absorbing agent U-2 | 0.32 g/m$^2$ |
| Ultraviolet ray absorbing Agent U-3 | 0.03 g/m$^2$ |
| High boiling point organic solvent Oil-2 | 0.28 ml/m$^2$ |

Thirteenth Layer: Second Protective Layer

A gelatin layer (dry layer thickness of 2.5 μm) containing;

| | |
|---|---|
| Surface-fogged, fine grain silver iodobromide emulsion (iodide content: 1 mol%, average particle size: 0.06 μm) | 0.1 g/m$^2$ (as silver) |
| Polymethyl methacrylate Particles (average particle size: 1.5 μm) | 0.2 g/m$^2$ |

Gelatin hardener H-1 (same as described in Example 1) and a surface active agent were incorporated into each of the layers in addition to the above described components.

The compounds employed for the preparation of the sample are illustrated hereinafter.

Sample 201 thus-prepared was exposed and subjected to reversal processing according to the steps shown below.

The color image obtained was excellent in sharpness and graininess.

| Processing Steps | Time | Temperature |
|---|---|---|
| First Development | 6 minutes | 38°C |
| Washing with Water | 2 minutes | " |
| Reversal | 2 minutes | " |
| Color Development | 6 minutes | " |
| Controlling | 2 minutes | " |
| Bleaching | 6 minutes | " |
| Fixing | 4 minutes | " |
| Washing with Water | 4 minutes | " |
| Stabilizing | 1 minute | Normal temperature |
| Drying | | |

The compositions of the processing solutions used for the above-described steps were as follows :

First Developing Solution:

| | |
|---|---|
| Water | 700 ml |
| Pentasodium nitrilo-N,N,N-trimethylene-phosphonate | 2 g |
| Sodium sulfite | 20 g |
| Hydroquinone monosulfonate | 30 g |
| Sodium carbonate (monohydrate) | 30 g |
| 1-Phenyl-4-methyl-4-hydroxymethyl-3-pyrazolidone | 2 g |
| Potassium bromide | 2.5 g |
| Potassium thiocyanate | 1.2 g |
| Potassium iodide (0.1% solution) | 2 ml |
| Water to make | 1000 ml |

Reversal Solution

| | |
|---|---|
| Water | 700 ml |
| Pentasodium nitrilo-N,N,N-trimethylene-phosphonate | 3 g |
| Stannous chloride (dihydrate) | 1 g |
| p-Aminophenol | 0.1 g |
| Sodium hydroxide | 8 g |
| Glacial acetic acid | 15 ml |
| Water to make | 1000 ml |

Color Developing Solution

| | |
|---|---|
| Water | 700 ml |
| Pentasodium nitrilo-N,N,N-trimethylene-phosphonate | 3 g |
| Sodium sulfite | 7 g |
| Sodium tertiary phosphate (12 hydrate) | 36 g |
| Potassium bromide | 1 g |
| Potassium iodide (0.1 wt% solution) | 90 ml |
| Sodium hydroxide | 3 g |
| Citrazinic acid | 1.5 g |
| N-Ethyl-N-(β-methanesulfonamidoethyl)-3-methyl-4-aminoaniline sulfate | 11 g |
| 3,6-Dithiaoctane-1,8-diol | 1 g |
| Water to make | 1000 ml |
| | (pH 12.0) |

Controlling Solution

| | |
|---|---|
| Water | 700 ml |
| Sodium sulfite | 12 g |

| | |
|---|---|
| Sodium ethylenediaminetetraacetate (dihydrate) | 8 g |
| Thioglycerol | 0.4 ml |
| Glacial acetic acid | 3 ml |
| Water to make | 1000 ml |

Bleaching Solution

| | |
|---|---|
| Water | 800 ml |
| Sodium ethylenediaminetetraacetate (dihydrate) | 2 g |
| Ammonium ethylenediaminetetraacetate iron (III) (dihydrate) | 120 g |
| Potassium bromide | 100 g |
| Water to make | 1000 ml |

Fixing Solution

| | |
|---|---|
| Water | 800 ml |
| Ammonium thiosulfate | 80.0 g |
| Sodium sulfite | 5.0 g |
| Sodium bisulfite | 5.0 g |
| Water to make | 1000 ml |

Stabilizing Solution

| | |
|---|---|
| Water | 800 ml |
| Formalin (37 wt%) | 5.0 ml |
| Fuji Driwel (surface active agents, manufactured by Fuji Photo Film Co., Ltd.) | 5.0 ml |
| Water to make | 1000 ml |

The compound employed for preparing Sample 201 in Example 2 were shown below.

F-1 (same as C-(41))*

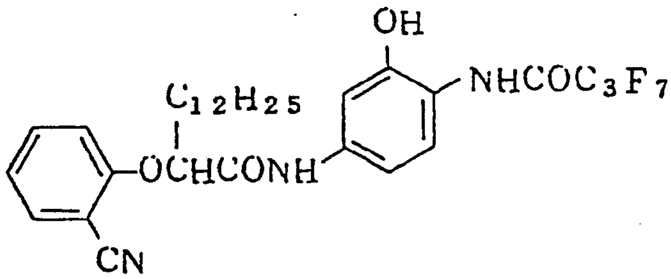

* : The C-series compounds have been earlier identified.

F-2 (same as C-(11))

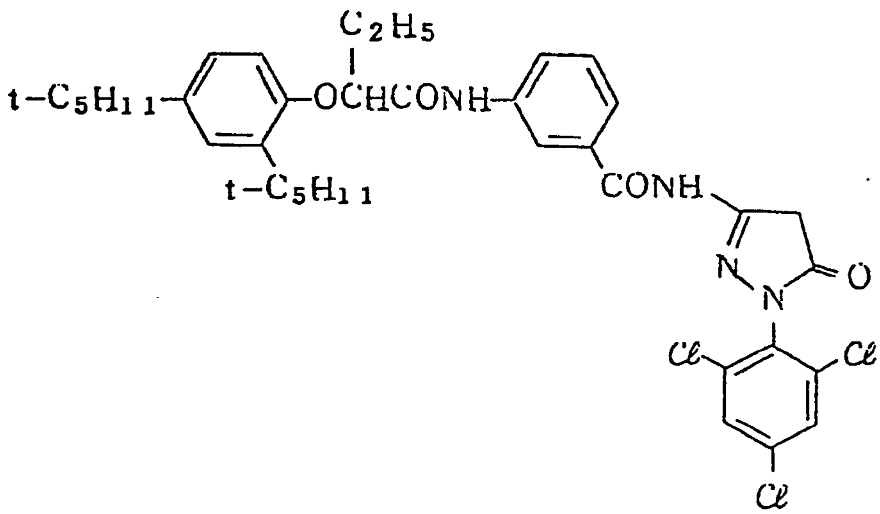

F-3 (same as C-(16))

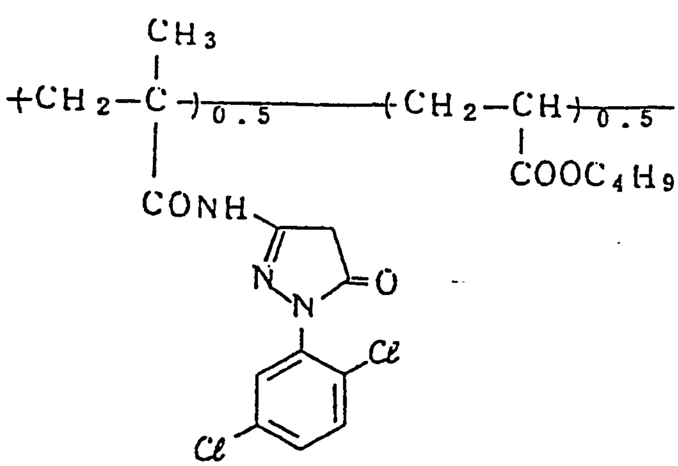

F-4 (same as C-(9))

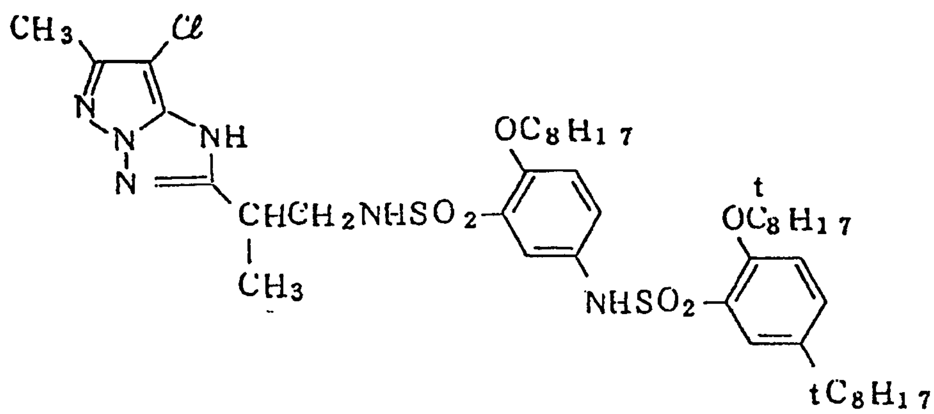

F-5 (same as C-(44))

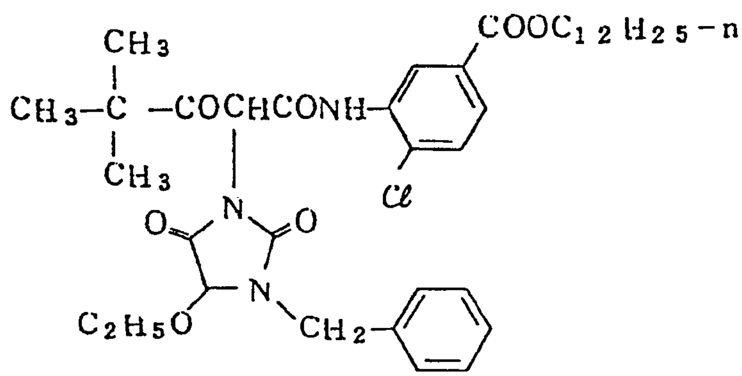

F-6 (same as C-(3))

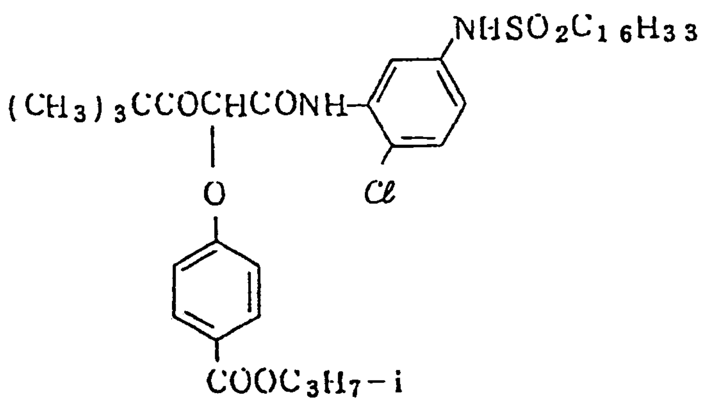

F-7 (same as C-(25))

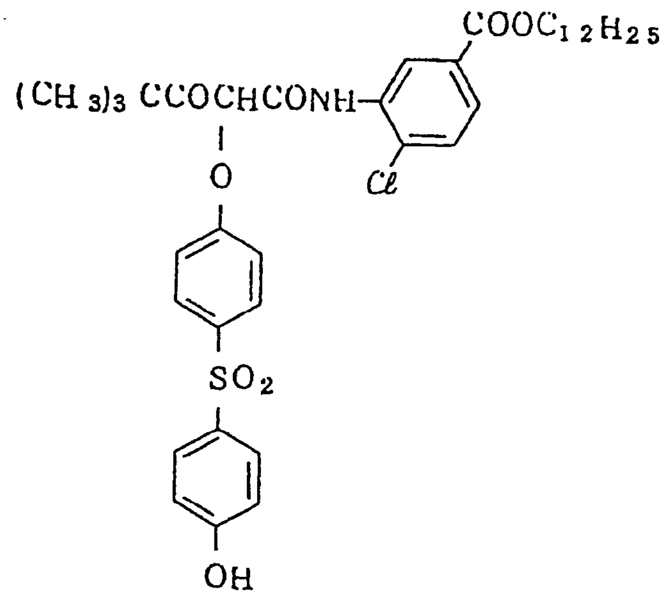

Compound I-2

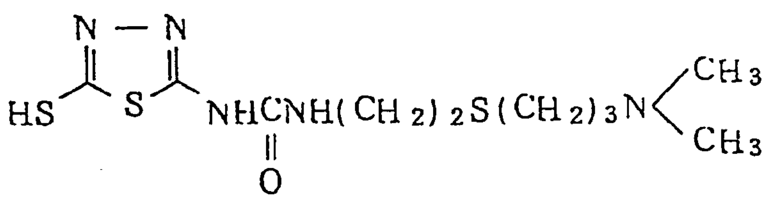

U-1

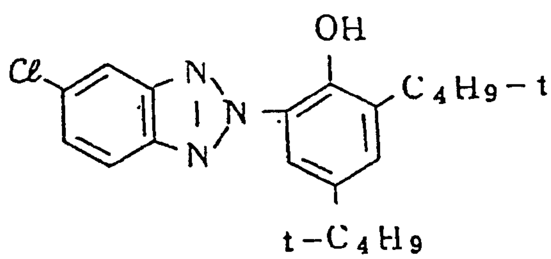

U-2

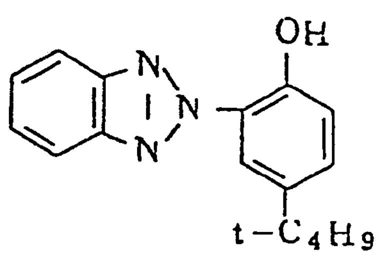

U-3

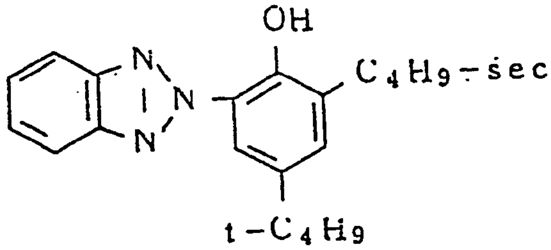

Cpd C

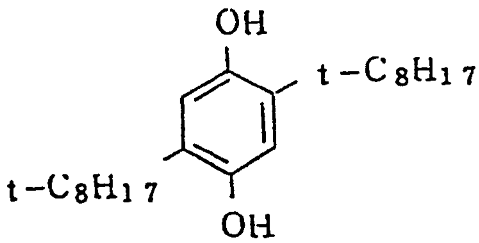

S-1

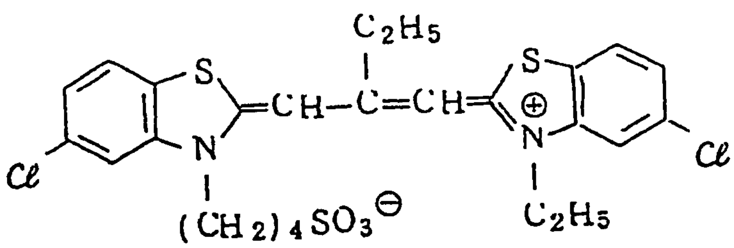

S-2

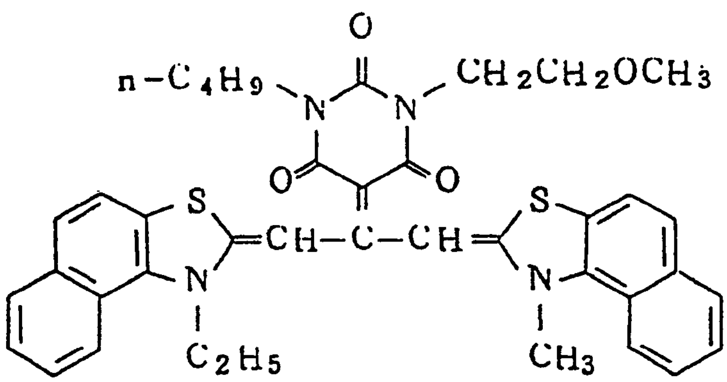

S-3

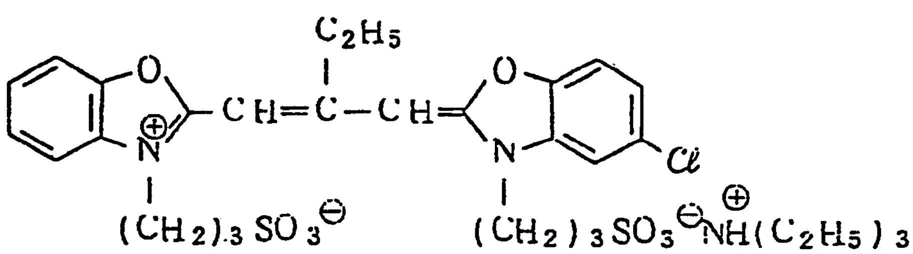

S-4

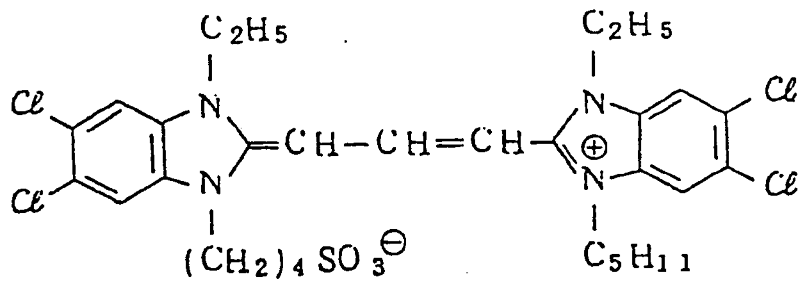

S-5

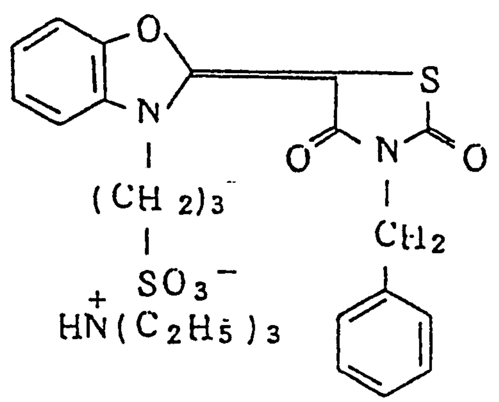

S-6

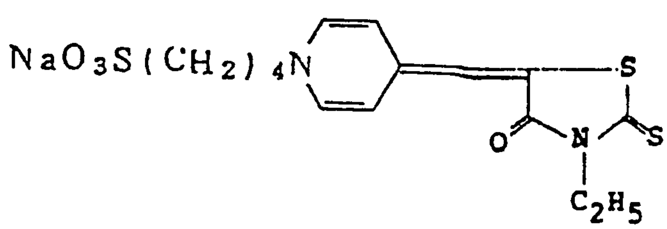

D-1

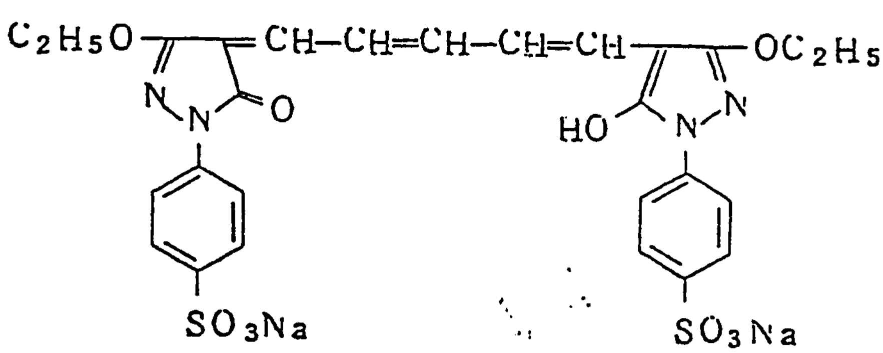

D-2

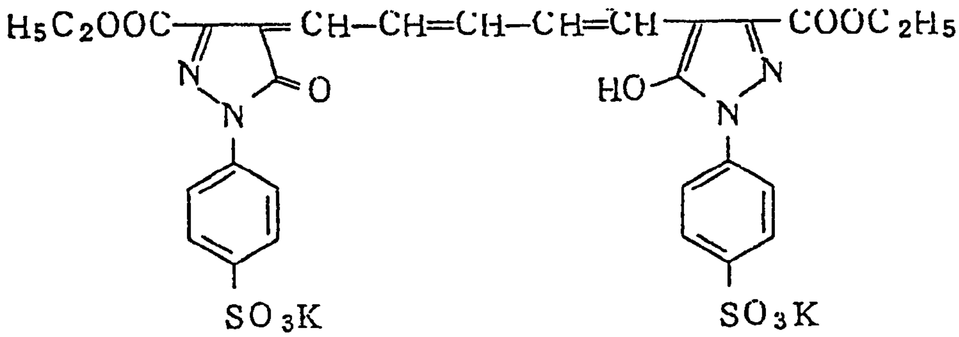

D-3

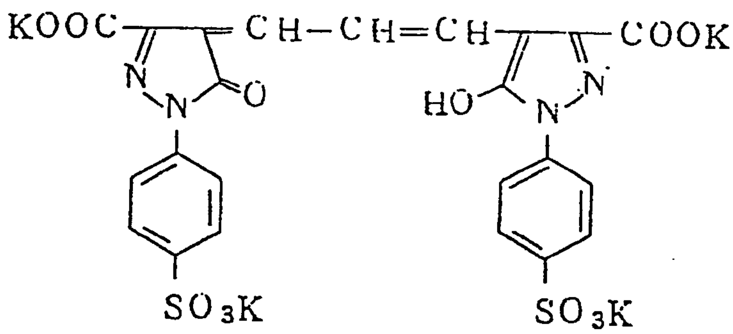

D-4

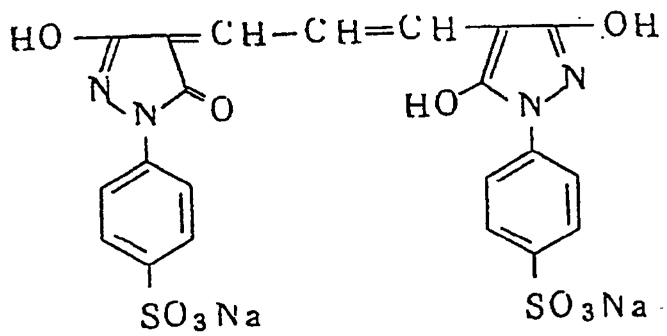

D-5

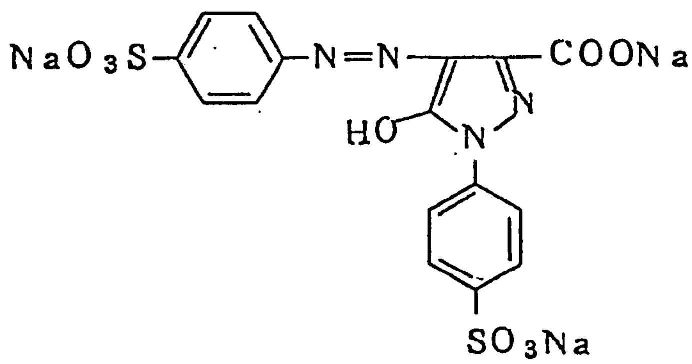

Oil-1 Tricresyl phosphate

Oil-2 Dibutyl phthalate

**Claims**

1. A silver halide photographic material comprising a support having thereon at least one silver halide emulsion layer, characterized in that the silver halide photographic material contains at least one compound represented by the following general formula (I) :

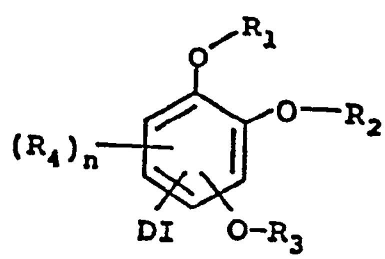

(I)

wherein $R_1$ and $R_2$ each represents a hydrogen atom or a group capable of being cleaved with an alkali ; $R_3$ represents an aromatic group or a heterocyclic group, the group represented by $R_3$ is unable to be released upon the reaction with an oxidation product of a developing agent ; $R_4$ represents a group capable of substituting on the benzene ring ; n represents an integer from 0 to 2 ; and DI when released represents a development inhibitor or a precursor thereof, when n represents 2, two $R_4$'s may be the same or different, and when any two substituents of $R_1$, $R_2$ and $R_4$ are present on the adjacent positions, they may be divalent groups and connected with each other to form a cyclic structure.

2. A silver halide photographic material as claimed in Claim 1, wherein $R_1$ and $R_2$ each represents a hydrogen atom.

3. A silver halide photographic material as claimed in Claim 1, wherein the group capable of being cleaved with an alkali is the group $R_5$-CO – or the group $R_5$O-CO – wherein $R_5$ represents an aliphatic group, an aromatic group or a heterocyclic group.

4. A silver halide photographic material as claimed in Claim 1, wherein the compounds represented by general formula (I) are compounds represented by the following general formula (Ia) or (Ib) :

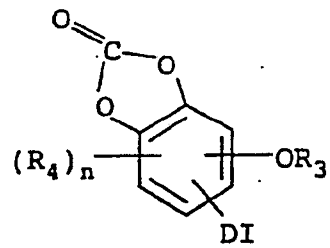

(Ia)

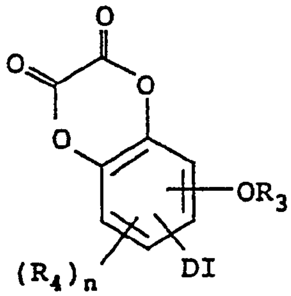

(Ib)

wherein $R_3$, $R_4$, n and DI each has the same meaning as defined for general formula (I).

5. A silver halide photographic material as claimed in Claim 1, wherein the group represented by $R_4$ is selected from the group $R_6O\text{-}CO-$, the group

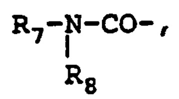

the group $R_6-$, the group $R_7\text{-}CO-$, the group $R_6\text{-}S-$, a halogen atom, the group $R_6\text{-}SO_2-$, the group

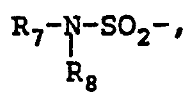

and a nitro group, wherein $R_6$ represents an aliphatic group, an aromatic group or a heterocyclic group ; and $R_7$ and $R_8$ each represents a hydrogen atom, an aliphatic group, an aromatic group or a heterocyclic group.

6. A silver halide photographic material as claimed in Claim 1, wherein the aromatic group represented by $R_3$ is a substituted or unsubstituted aromatic group having from 6 to 20 carbon atoms.

7. A silver halide photographic material as claimed in Claim 1, wherein the heterocyclic group represented by $R_3$ is a substituted or unsubstituted hetero cyclic group containing at least one hetero atom selected from a nitrogen atom, an oxygen atom and a sulfur atom.

8. A silver halide photographic material as claimed in Claim 3, wherein the aliphatic group represented by $R_5$ is a saturated or unsaturated, straight chain, branched chain or cyclic, substituted or unsubstituted aliphatic hydrocarbon group having 1 to 40 carbon atoms.

9. A silver halide photographic material as claimed in Claim 3, wherein the aromatic group represented by $R_5$ is a substituted or unsubstituted aromatic group having from 6 to 20 carbon atoms.

10. A silver halide photographic material as claimed in Claim 3, wherein the heterocyclic group represented by $R_5$ is a substituted or unsubstituted hetero cyclic group containing at least one hetero atom selected from a nitrogen atom, an oxygen atom and a sulfur atom.

11. A silver halide photographic material as claimed in Claim 5, wherein the aliphatic group represented by $R_6$, $R_7$ or $R_8$ is a saturated or unsaturated, straight chain, branched chain or cyclic, substituted or unsubstituted aliphatic hydrocarbon group having 1 to 40 carbon atoms.

12. A silver halide photographic material as claimed in Claim 5, wherein the aromatic group represented by $R_6$, $R_7$ or $R_8$ is a substituted or unsubstituted aromatic group having from 6 to 20 carbon atoms.

13. A silver halide photographic material as claimed in Claim 5, wherein the heterocyclic group represented

by $R_6$, $R_7$ or $R_8$ is a substituted or unsubstituted hetero cyclic group containing at least one hetero atom selected from a nitrogen atom, an oxygen atom and a sulfur atom.

14. A silver halide photographic material as claimed in Claim 1, wherein the development inhibitor represented by DI is selected from a tetrazolylthio group, a thiazolylthio group, a benzothiadiazolylthio group, a benzoxazolylthio group, a benzotriazolyl group, indazolyl group, a benzimidazolylthio group, a triazolylthio group, a thiadiazolylthio group, a triazolyl group substituted with a thioether group and an oxadiazolyl group.

15. A silver halide photographic material as claimed in Claim 1, wherein the precursor of the development inhibitor represented by DI is a group represented by the following formula :

$$* - TIME - DI'$$

wherein the symbol * denotes the position at which the group is connected to the residue of the compound represented by the general formula (I) ; TIME represents a group capable of releasing DI' after being released as TIME-DI' ; and DI' represents a development inhibitor which is the same as the development inhibitor as defined for DI.

16. A silver halide photographic material as claimed in Claim 1, wherein the compound represented by the general formula (I) is represented by the following general formula (II) :

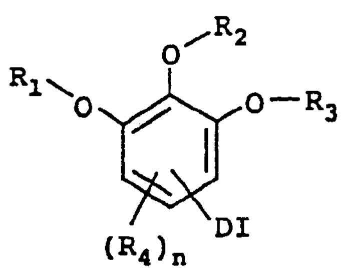

(II)

wherein $R_1$, $R_2$, $R_3$, $R_4$, n and DI each has the same meaning as defined in the general formula (I).

17. A silver halide photographic material as claimed in Claim 1, wherein the silver halide photographic material further contains a coupler.

18. A silver halide photographic material as claimed in Claim 17, wherein the coupler is selected from a yellow coupler, a magenta coupler, a cyan coupler and a non-color forming coupler.

19. A silver halide photographic material as claimed in Claim 17, wherein the coupler is selected from an acylacetamide coupler, a malondiamide coupler, a 5-pyrazolone coupler, a pyrazoloimidazole coupler, a pyraazolotriazole coupler, a phenol coupler and a naphthol coupler.

20. A silver halide photographic material as claimed in Claim 17, wherein the coupler is represented by the following general formula (Cp-1), (Cp-2), (Cp-3), (Cp-4), (Cp-5), (Cp-6), (Cp-7) or (Cp-8) :

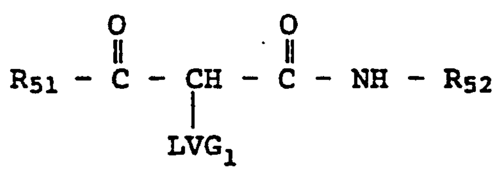

(Cp-1)

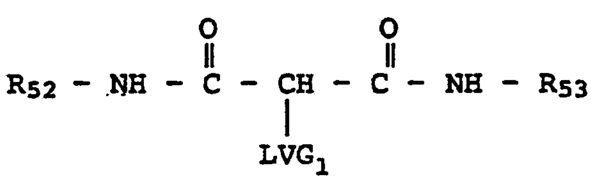

(Cp-2)

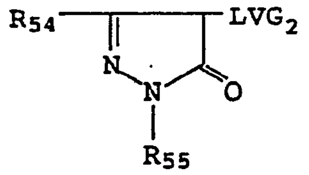
(Cp-3)

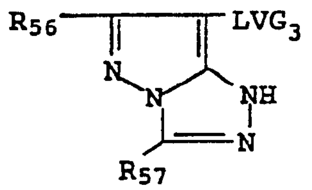
(Cp-4)

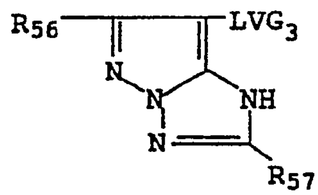
(Cp-5)

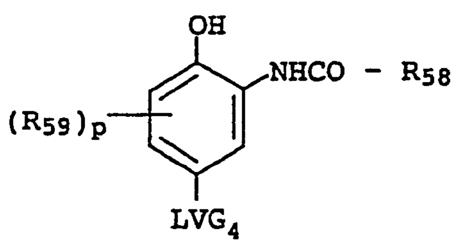
(Cp-6)

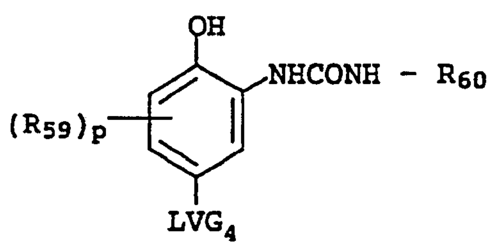
(Cp-7)

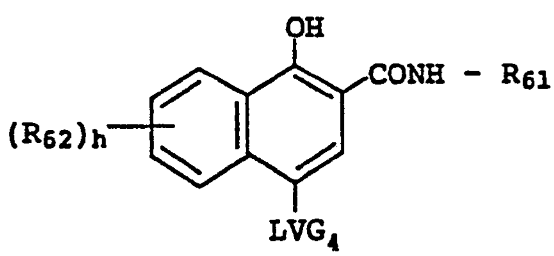
(Cp-8)

wherein $LVG_1$, $LVG_2$, $LVG_3$ and $LVG_4$ each represents a coupling releasing group or a hydrogen atom ; $R_{41}$ represents an aliphatic group, an aromatic group or a heterocyclic group ; $R_{42}$ represents an aromatic group or a heterocyclic group ; and $R_{43}$, $R_{44}$ and $R_{43}$ each represents a hydrogen atom, an aliphatic group, an aromatic

group or a heterocyclic group ;

$R_{51}$ represents a group as defined for $R_{41}$;

$R_{52}$ and $R_{53}$ each represents a group as defined for $R_{42}$;

$R_{54}$ represents a group as defined for $R_{41}$, the group $R_{41}CON(R_{43})-$, the group $R_{41}N(R_{43})-$, the group $R_{41}SO_2N(R_{43})-$, the group $R_{41}S-$, the group $R_{43}O-$, the group $R_{45}N(R_{43})CON(R_{44})-$, the group $R_{41}OOC-$, the group $R_{44}N(R_{43})CO-$ or the group $N{\equiv}C-$;

$R_{55}$ represents a group as defined for $R_{41}$;

$R_{56}$ and $R_{57}$ each represents a group as defined for $R_{43}$, the group $R_{41}S-$, the group $R_{43}O-$, the group $R_{41}CON(R_{43})-$, the group $R_{41}N(R_{43})-$, the group $R_{41}OCON(R_{43})-$, the group $R_{41}O-$, the group $R_{43}N(R_{44})CON(R_{45})-$ or the group $R_{41}SO_2N(R_{43})-$;

$R_{58}$ represents a group as defined for $R_{41}$.

$R_{59}$ represents a group as defined for $R_{41}$, the group $R_{41}CON(R_{43})-$, the group $R_{41}OCON(R_{43})-$, the group $R_{43}SO_2N(R_{43})-$, the group $R_{43}N(R_{44})CON(R_{45})-$, the group $R_{43}N(R_{44})SO_2N(R_{45})-$, the group $R_{41}O-$, the group $R_{41}S-$, a halogen atom or the group $R_{41}N(R_{43})-$.

p represents an integer from 0 to 3, when p represents 2 or more, two or more $R_{59}$'s may be the same or different, or each of two $R_{59}$'s may be a divalent group and connected with each other to form a cyclic structure;

$R_{60}$ represents a group as defined for $R_{41}$;

$R_{61}$ represents a group as defined for $R_{41}$;

$R_{62}$ represents a group as defined for $R_{41}$, the group $R_{41}CONH-$, the group $R_{41}OCONH-$, the group $R_{41}SO_2NH-$, the group $R_{43}\underset{R_{44}}{N}CO\underset{R_{45}}{N}-$, the group $R_{43}\underset{R_{44}}{N}SO_2\underset{R_{45}}{N}-$, the group $R_{43}O-$, the group $R_{41}S-$, a halogen atom or the group $R_{41}\underset{R_{43}}{N}-$ ; and

h represents an integer from 0 to 4, when h represents 2 or more, two or more $R_{62}$'s may be the same or different.

21. A silver halide photographic material as claimed in Claim 20, wherein the aliphatic group, aromatic group, or heterocyclic group has at least one substituent selected from a halogen atom, the group $R_{47}O-$, the group $R_{46}S-$, the group $R_{47}CO\underset{R_{48}}{N}-$, the group $R_{47}\underset{R_{48}}{N}CO-$, the group $R_{46}OCO\underset{R_{47}}{N}-$, the group $R_{46}SO_2\underset{R_{47}}{N}-$, the group 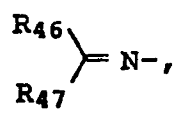 the group $R_{47}\underset{R_{48}}{N}SO_2-$, the group $R_{46}SO_2-$, the group $R_{47}OCO-$, the group $R_{47}\underset{R_{48}}{N}CO\underset{R_{49}}{N}-$, the group 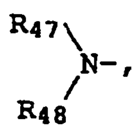 a group as defined for $R_{46}$, the group 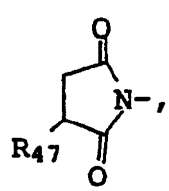 the group $R_{46}COO-$, the group $R_{47}OSO_2-$, a cyano group, or a nitro group, wherein $R_{46}$ represents an aliphatic group, an aromatic group or a heterocyclic group ; and $R_{47}$, $R_{48}$ and $R_{49}$ each represents a hydrogen atom, an aliphatic group, an aromatic group or a heterocyclic group.

22. A silver halide photographic material as claimed in Claim 20, wherein $R_{51}$ represents an aliphatic group or an aromatic group ; $R_{52}$, $R_{53}$ and $R_{55}$ each represents an aromatic group ; $R_{54}$ represents the group $R_{41}CONH-$ or the group

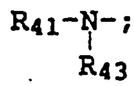

$R_{56}$ and $R_{57}$ each represents an aliphatic group, the group $R_{41}O$ – or the group $R_{41}S$ ;
$R_{58}$ represents an aliphatic group or an aromatic group ;
$R_{59}$ in the general formula (Cp-6) represents a chlorine atom, an aliphatic group or the group $R_{41}CONH$ –;
p in the general formula (Cp-6) represents 1 or 2 ;
$R_{60}$ represents an aromatic group ;
$R_{59}$ in the general formula (Cp-7) represents the group $R_{41}CONH$ – ;
p in the general formula (Cp-7) represents 1 ;
$R_{61}$ represents an aliphatic group or an aromatic group ;
h in the general formula (Cp-8) represents 0 or 1 ; and
$R_{62}$ represents the group $R_{41}OCONH$ –, the group $R_{41}CONH$ – or the group $R_{41}SO_2NH$ –.

23. A silver halide photographic material as claimed in Claim 20, wherein $LVG_1$ represents the group $R_{65}O$ –, an imido group which is connected to the coupling position through the nitrogen atom thereof, an unsaturated nitrogen-containing heterocyclic group which is connected to the coupling position through the nitrogen atom thereof or the group $R_{66}S$ –, wherein $R_{65}$ represents an aromatic group or a heterocyclic group and $R_{66}$ represents an aliphatic group, an aromatic group or a heterocyclic group.

24. A silver halide photographic material as claimed in Claim 20, wherein $LVG_2$ represents the group $R_{66}S$ –, an unsaturated nitrogen-containing heterocyclic group which is connected to the coupling position through the nitrogen atom thereof, the group $R_{65}O$ – or a hydrogen atom, wherein $R_{65}$ represents an aromatic group or a heterocyclic group and $R_{66}$ represents an aliphatic group, an aromatic group or a heterocyclic group.

25. A silver halide photographic material as claimed in Claim 20, wherein $LVG_3$ represents a halogen atom, the group $R_{66}S$ – or an unsaturated nitrogen-containing heterocyclic group which is connected to the coupling position through the nitrogen atom thereof.

## Ansprüche

1. Photographisches Silberhalogenidmaterial, umfassend einen Träger, auf dem mindestens eine Silberhalogenidemulsionsschicht vorgesehen ist, dadurch gekennzeichnet, daß das photographische Silberhalogenidmaterial mindestens eine Verbindung enthält, die durch die folgende allgemeine Formel (I) angegeben wird:

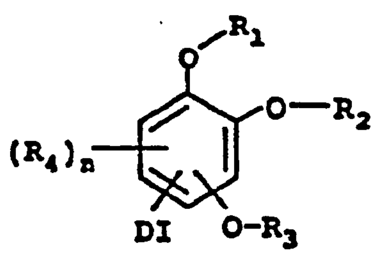

(I)

worin bedeuten :
$R_1$ und $R_2$ jeweils ein Wasserstoffatom oder eine Gruppe, die mit einem Alkali abgespalten werden kann;
$R_3$ eine aromatische Gruppe oder heterocyclische Gruppe, wobei die durch $R_3$ angegebene Gruppe nicht fähig ist, als Folge der Reaktion mit einem Oxidationsprodukt eines Entwicklungsmittels freigesetzt zu werden;
$R_4$ eine Gruppe, die in der Lage ist, am Benzolring zu substituieren ;
n eine ganze Zahl von 0 bis 2 ; und
DI wem freigesetzt, einen Entwicklungsinhibitor oder einen Vorläufer hiervon,
wobei, wem n 2 bedeutet, zwei $R_4$ gleich oder verschieden sein können und, wenn zwei der Substituenten $R_1$, $R_2$ und $R_4$ in benachbarten Positionen vorliegen, diese zweiwertige Gruppen sein können und miteinander zur Bildung einer zyklischen Struktur verbunden sein können.

2. Photographisches Silberhalogenidmaterial nach Anspruch 1, wobei $R_1$ und $R_2$ jeweils ein Wasserstoffatom bedeuten.

3. Photographisches Silberhalogenidmaterial nach Anspruch 1, wobei die Gruppe, die mit einem Alkali abgespalten werden kann, die Gruppe $R_5$-CO – oder die Gruppe $R_5$O-CO – ist, worin $R_5$ eine aliphatische

Gruppe, aromatische Gruppe oder heterocyclische Gruppe bedeutet.

4. Photographisches Silberhalogenidmaterial nach Anspruch 1, wobei die durch die allgemeine Formel (I) angegebenen Verbindungen Verbindungen sind, die durch die folgenden allgemeinen Formeln (Ia) oder (Ib) angegeben werden :

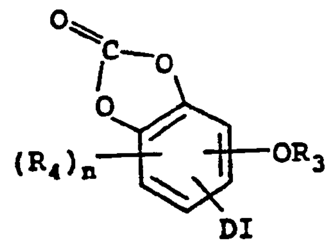

(Ia)

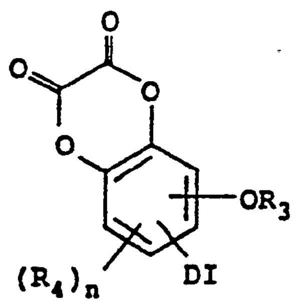

(Ib)

worin $R_3$, $R_4$, n und DI jeweils die gleichen Bedeutungen haben, wie bei der allgemeinen Formel (I) definiert.

5. Photographisches Silberhalogenidmaterial nach Anspruch 1, wobei die durch $R_4$ angegebene Gruppe gewählt wird aus der Gruppe

$R_6O\text{-}CO\text{-}$, der Gruppe $R_7\text{-}\underset{R_8}{N}\text{-}CO\text{-}$, der Gruppe $R_6\text{-}$, der Gruppe $R_7\text{-}CO\text{-}$,

der Gruppe $R_6\text{-}S\text{-}$, einem Halogenatom, der Gruppe $R_6\text{-}SO_2\text{-}$,

der Gruppe $R_7\text{-}\underset{R_8}{N}\text{-}SO_2\text{-}$,

und einer Nitrogruppe, worin $R_6$ eine aliphatische Gruppe, aromatische Gruppe oder heterocyclische Gruppe darstellt ; und $R_7$ und $R_8$ jeweils ein Wasserstoffatom, eine aliphatische Gruppe, aromatische Gruppe oder heterocyclische Gruppe bedeuten.

6. Photographisches Silberhalogenidmaterial nach Anspruch 1, wobei die durch $R_3$ angegebene aromatische Gruppe eine substituierte oder unsubstituierte aromatische Gruppe mit 6 bis 20 Kohlenstoffatomen ist.

7. Photographisches Silberhalogenidmaterial nach Anspruch 1, wobei die durch $R_3$ angegebene heterocyclische Gruppe eine substituierte oder unsubstituierte heterocyclische Gruppe ist, die mindestens ein Heteroatom enthält, gewählt aus einem Stickstoffatom, Sauerstoffatom und Schwefelatom.

8. Photographisches Silberhalogenidmaterial nach Anspruch 3, wobei die durch $R_5$ angegebene aliphatische Gruppe eine gesättigte oder ungesättigte, geradkettige, verzweigtkettige oder cyclische, substituierte oder unsubstituierte aliphatische Kohlenwasserstoffgruppe mit 1 bis 40 Kohlenstoffatomen ist.

9. Photographisches Silberhalogenidmaterial nach Anspruch 3, wobei die durch $R_5$ angegebene aromatische Gruppe eine substituierte oder unsubstituierte aromatische Gruppe mit 6 bis 20 Kohlenstoffatomen ist.

10. Photographisches Silberhalogenidmaterial nach Anspruch 3, wobei die durch $R_5$ angegebene heterocyclische Gruppe eine substituierte oder unsubstituierte heterocyclische Gruppe ist, die mindestens ein Heteroatom enthält, gewählt aus einem Stickstoffatom, Sauerstoffatom und Schwefelatom.

11. Photographisches Silberhalogenidmaterial nach Anspruch 5, wobei die durch $R_6$, $R_7$ oder $R_8$ angegebene aliphatische Gruppe eine gesättigte oder ungesättigte, geradkettige, verzweigtkettige oder cyclische, substituierte oder unsubstituierte aliphatische Kohlenwasserstoffgruppe mit 1 bis 40 Kohlenstoffatomen ist.

12. Photographisches Silberhalogenidmaterial nach Anspruch 5, wobei die $R_6$, $R_7$ oder $R_8$ angegebene aromatische Gruppe eine substituierte oder unsubstituierte aromatische Gruppe mit 6 bis 20 Kohlenstoffatomen ist.

13. Photographisches Silberhalogenidmaterial nach Anspruch 5, wobei die durch $R_6$, $R_7$ oder $R_8$ angegebene heterocyclische Gruppe eine substituierte oder unsubstituierte heterocyclische Gruppe ist, die mindestens ein Heteroatom enhält, gewählt aus einem Stickstoffatom, Sauerstoffatom und Schwefelatom.

14. Photographisches Silberhalogenidmaterial nach Anspuch 1, wobei der durch DI angegebene Entwicklungsinhibitor gewählt wird aus einer Tetrazolylthiogruppe, Thiazolylthiogruppe, Benzothiadiazolylthiogruppe, Benzoxazolylthiogruppe, Benzotriazolylgruppe, Indazolylgruppe, Benzimidazolylthiogruppe, Triazolylthiogruppe, Thiadiazolylthiogruppe, mit einer Thioethergruppe substituierten Triazolylgruppe und einer Oxadiazolylgruppe.

15. Photographisches Silberhalogenidmaterial nach Anspruch 1, wobei der durch DI angegebene Vorläufer des Entwicklungsinhibitors eine durch die folgende Formel angegebene Gruppe ist :

$$* - TIME - DI'$$

worin das Symbol * die Position angibt, an der die Gruppe an den Rest der durch die allgemeine Formel (I) angegebenen Verbindung gebunden ist ; TIME eine Gruppe darstellt, die in der Lage ist, DI' freizusetzen, nachdem sie als TIME-DI' freigesetzt worden ist ; und DI' einen Entwicklungsinhibitor darstellt, welcher der gleiche Entwicklungsinhibitor ist, wie für DI definiert.

16. Photographisches Silberhalogenidmaterial nach Anspruch 1, wobei die durch die allgemeine Formel (I) angegebene Verbindung durch die folgende allgemeine Formel (II) dargestellt wird.

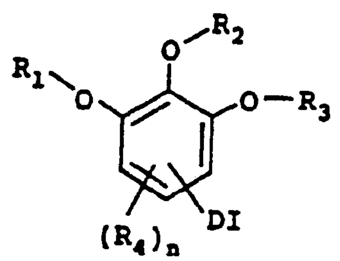

(II)

worin $R_1$, $R_2$, $R_3$, $R_4$, n und DI jeweils die gleiche Bedeutung hat, wie in der allgemeinen Formel (I) definiert.

17. Photographisches Silberhalogenidmaterial nach Anspruch 1, wobei das photographische Silberhalogenidmaterial weiterhin einen Kuppler enhält.

18. Photographisches Silberhalogenidmaterial nach Anspruch 17, wobei der Kuppler gewählt wird aus einem Gelbkuppler, Magenta-Kuppler, Cyan-Kuppler und einem nichtfarbebildenenden Kuppler.

19. Photographisches Silberhalogenidmaterial nach Anspruch 17, wobei der Kuppler gewählt wird aus einem Acylacetamidkuppler, Malondiamidkuppler, 5-Pyrazolonkuppler, Pyrazoloimidazolkuppler, Pyrazolotriazolkuppler, Phenolkuppler und Naphtholkuppler.

20. Photographisches Silberhalogenidmaterial nach Anspruch 17, wobei der Kuppler durch die folgende allgemeine Formel (Cp-1), (Cp-2), (Cp-3), (Cp-4), (Cp-5), (Cp-6), (Cp-7) oder (Cp-8) dargestellt wird :

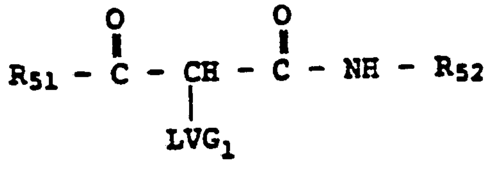

(Cp-1)

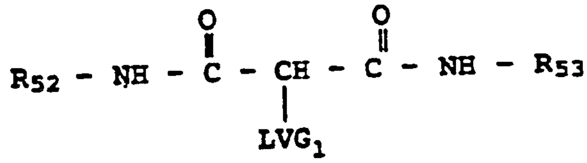

(Cp-2)

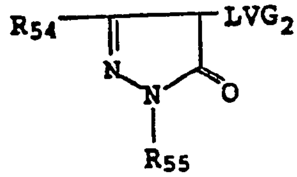

(Cp-3)

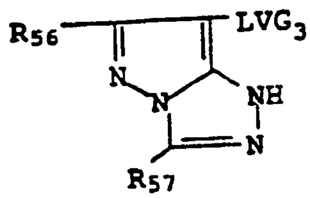

(Cp-4)

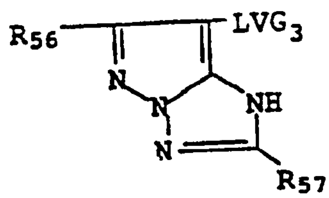

(Cp-5)

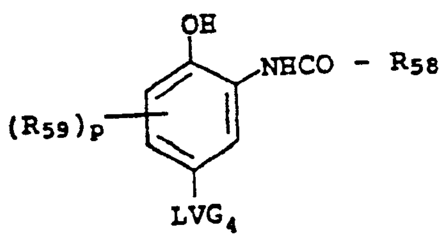

(Cp-6)

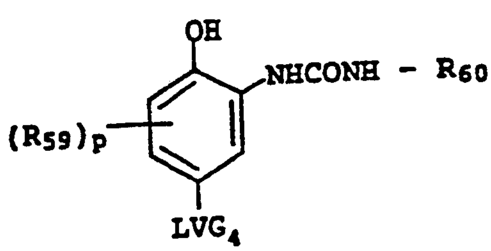

(Cp-7)

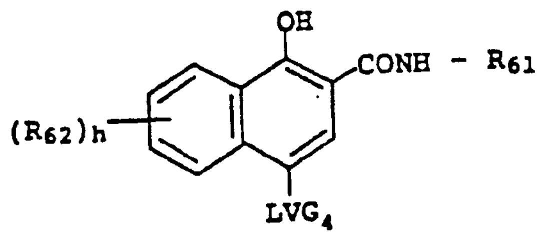

(Cp-8)

worin bedeuten :

$LVG_1$, $LVG_2$, $LVG_3$ und $LVG_4$ jeweils eine kupplungsfreisetzende Gruppe oder ein Wasserstoffatom ; $R_{41}$ eine aliphatische Gruppe, aromatische Gruppe oder heterocyclische Gruppe ;

$R_{42}$ eine aromatische Gruppe oder heterocyclische Gruppe ; und

$R_{43}$, $R_{44}$ und $R_{45}$ jeweils ein Wasserstoffatom, eine aliphatische Gruppe, aromatische Gruppe oder heterocyclische Gruppe ;

$R_{51}$ eine Gruppe, wie für $R_{41}$ definiert;

$R_{52}$ und $R_{53}$ jeweils eine Gruppe, wie für $R_{42}$ definiert;

$R_{54}$ eine Gruppe, wie für $R_{41}$ definiert, die Gruppe $R_{41}CO\underset{R_{43}}{N}-$ , die Gruppe $R_{41}\underset{R_{43}}{N}-$ , die Gruppe $R_{41}SO_2\underset{R_{43}}{N}-$ ,

die Gruppe $R_{41}S-$, die Gruppe $R_{43}O-$, die Gruppe $R_{45}\underset{R_{43}}{N}CO\underset{R_{44}}{N}-$ ,

die Gruppe $R_{41}OOC-$, die Gruppe $R_{44}\underset{R_{43}}{N}CO-$ oder

die Gruppe $N{\equiv}C-$;

$R_{55}$ eine Gruppe wie für $R_{41}$ definiert;

$R_{56}$ und $R_{57}$ jeweils eine Gruppe wie für $R_{43}$ definiert, die Gruppe $R_{41}S-$, die Gruppe $R_{43}O-$, die Gruppe $R_{41}CO\underset{R_{43}}{N}-$ , die Gruppe $R_{41}\underset{R_{43}}{N}-$ , die Gruppe $R_{41}OCO\underset{R_{43}}{N}-$ ,

die Gruppe $R_{41}O-$, die Gruppe $R_{43}\underset{R_{44}}{N}CO\underset{R_{45}}{N}-$ oder die Gruppe

$$R_{41}SO_2\underset{R_{43}}{N}- \;;$$

$R_{58}$ eine Gruppe, wie für $R_{41}$ definiert;

$R_{59}$ eine Gruppe, wie für $R_{41}$ definiert, die Gruppe $R_{41}CO\underset{\substack{|\\R_{43}}}{N}-$ , die Gruppe $R_{41}OCO\underset{\substack{|\\R_{43}}}{N}-$, die Gruppe $R_{43}SO_2\underset{\substack{|\\R_{43}}}{N}-$ ,

die Gruppe $R_{43}\underset{\substack{|\\R_{44}}}{N}CO\underset{\substack{|\\R_{45}}}{N}-$ , die Gruppe $R_{43}\underset{\substack{|\\R_{44}}}{N}SO_2\underset{\substack{|\\R_{45}}}{N}-$ ,

die Gruppe $R_{41}O-$, die Gruppe $R_{41}S-$, ein Halogenatom oder

die Gruppe $R_{41}\underset{\substack{|\\R_{43}}}{N}-$ ;

p eine ganze Zahl von 0 bis 3, wobei wenn p 2 oder mehr bedeutet, zwei oder mehrere von $R_{59}$ gleich oder voneinander verschieden sein können oder jede der zwei von $R_{59}$ kann eine zweiwertige Gruppe sein, die miteinander verbunden sind zur Bildung einer cyclischen Struktur ;

$R_{60}$ eine Gruppe, wie für $R_{41}$ definiert;

$R_{61}$ eine Gruppe, wie für $R_{41}$ definiert;

$R_{62}$ eine Gruppe, wie für $R_{41}$ definiert, die Gruppe $R_{41}CONH-$, die Gruppe $R_{41}OCONH-$, die Gruppe $R_{41}SO_2NH-$,

die Gruppe $R_{43}\underset{\substack{|\\R_{44}}}{N}CO\underset{\substack{|\\R_{45}}}{N}-$ , die Gruppe $R_{43}\underset{\substack{|\\R_{44}}}{N}SO_2-\underset{\substack{|\\R_{45}}}{N}-$ ,

die Gruppe $R_{43}O-$,

die Gruppe $R_{41}S-$, ein Halogenatom oder die Gruppe $R_{41}\underset{\substack{|\\R_{43}}}{N}-$ ; und

h eine ganze Zahl von 0 bis 4, wobei wenn h 2 oder mehr bedeutet, zwei oder mehrere $R_{62}$ gleich oder verschieden sein können.

21. Photographisches Silberhalogenidmaterial nach Anspruch 20, wobei die aliphatische Gruppe, aromatische Gruppe oder heterocyclische Gruppe mindestens einen Substituenten aufweist, gewählt aus einem Halogenatom, der Gruppe $R_{47}O-$, der Gruppe

$R_{46}S$, der Gruppe $R_{47}CO\underset{R_{48}}{\underset{|}{N}}-$ , der Gruppe $R_{47}\underset{R_{48}}{\underset{|}{N}}CO-$,

der Gruppe $R_{46}OCO\underset{R_{47}}{\underset{|}{N}}-$ , der Gruppe $R_{46}SO_2\underset{R_{47}}{\underset{|}{N}}-$ ,

der Gruppe $\begin{matrix}R_{46}\\ R_{47}\end{matrix}\!\!>\!=N-$, der Gruppe $R_{47}\underset{R_{48}}{\underset{|}{N}}SO_2-$ ,

der Gruppe $R_{46}SO_2$, der Gruppe $R_{47}OCO-$,
der Gruppe $R_{47}\underset{R_{48}}{\underset{|}{N}}CO\underset{R_{49}}{\underset{|}{N}}-$ ,

der Gruppe $\begin{matrix}R_{47}\\ R_{48}\end{matrix}\!\!>\!N-$, einer Gruppe, wie für $R_{46}$ definiert,

der Gruppe 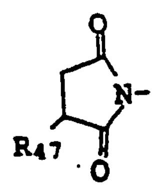, der Gruppe $R_{46}COO-$,

der Gruppe $R_{47}OSO_2-$, einer Cyanogruppe, oder einer Nitrogruppe, worin $R_{46}$ eine aliphatische Gruppe, aromatische Gruppe oder heterocyclische Gruppe bedeutet; und $R_{47}$, $R_{48}$ und $R_{49}$ jeweils ein Wasserstoffatom, eine aliphatische Gruppe, aromatische Gruppe oder heterocyclische Gruppe bedeutet.

22. Photographisches Silberhalogenidmaterial nach Anspruch 20, worin $R_{51}$ eine aliphatische Gruppe oder aromatische Gruppe bedeutet; $R_{52}$, $R_{53}$ und $R_{55}$ jeweils eine aromatische Gruppe bedeutet; $R_{54}$ die Gruppe $R_{41}CONH-$ oder die Gruppe

$$R_{41}-\underset{R_{43}}{\underset{|}{N}}-$$

bedeutet;

$R_{56}$ und $R_{57}$ jeweils eine aliphatische Gruppe, die Gruppe $R_{41}O-$ oder die Gruppe $R_{41}S-$ bedeutet;

$R_{58}$ eine aliphatische oder aromatische Gruppe bedeutet;

$R_{59}$ in der allgemeinen Formel (Cp-6) ein Chloratom, eine aliphatische Gruppe oder die Gruppe $R_{41}CONH-$ bedeutet;

p in der allgemeinen Formel (Cp-6) 1 oder 2 bedeutet;

$R_{60}$ eine aromatische Gruppe bedeutet:

$R_{59}$ in der allgemeinen Formel (Cp-7) die Gruppe $R_{41}CONH-$ bedeutet;

p in der allgemeinen Formel (Cp-7) 1 bedeutet;

$R_{61}$ eine aliphatische Gruppe oder aromatische Gruppe bedeutet;

h in der allgemeinen Formel (Cp-8) 0 oder 1 bedeutet ; und

$R_{62}$ die Gruppe $R_{41}OCONH$ –, die Gruppe $R_{41}CONH$ – oder die Gruppe $R_{41}SO_2NH$ – bedeutet.

23. Photographisches Silberhalogenidmaterial nach Anspruch 20, worin $LVG_1$ die Gruppe $R_{65}O$ –, eine Imidogruppe, die an die Kupplungsposition über deren Stickstoffatom gebunden ist, eine ungesättige Stickstoff enthaltende heterocyclische Gruppe, die an die Kupplungsposition über deren Stickstoffatom gebunden ist oder die Gruppe $R_{66}S$ – bedeutet, worin $R_{65}$ eine aromatische oder heterocyclische Gruppe und $R_{66}$ eine aliphatische Gruppe, aromatische Gruppe oder eine heterocyclische Gruppe bedeuten.

24. Photographisches Silberhalogenidmaterial nach Anspruch 20, wobei $LVG_2$ die Gruppe $R_{66}S$ –, eine ungesättigte Stickstoff enthaltende heterocyclische Gruppe, die an die Kupplungsposition über deren Stickstoffatom gebunden ist, die Gruppe $R_{65}O$ – oder ein Wasserstoffatom bedeutet, worin $R_{65}$ eine aromatische Gruppe oder heterocyclische Gruppe und $R_{66}$ eine aliphatische Gruppe, aromatische Gruppe oder heterocyclische Gruppe bedeuten.

25. Photographisches Silberhalogenidmaterial nach Anspruch 20, wobei $LVG_3$ ein Halogenatom, die Gruppe $R_{66}S$ – oder eine ungesättigte Stickstoff enthaltende heterocyclische Gruppe, die an die Kupplungsposition über deren Stickstoffatom gebunden ist, bedeutet.

**Revendications**

1. Un matériau photographique à l'halogénure d'argent comprenant un support qui porte au moins une couche d'émulsion d'halogénure d'argent, caractérisé en ce que le matériau photographique a l'halogénure d'argent contient au moins un composé représenté par la formule générale (I) suivante :

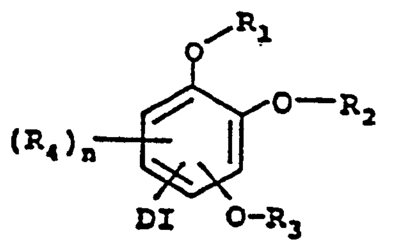

(I)

dans laquelle $R_1$ et $R_2$ représentent chacun un atome d'hydrogène ou un groupe éliminable par un alcali ; $R_3$ représente un groupe aromatique ou un groupe hétérocyclique, le groupe représenté par $R_3$ n'étant pas éliminable par la réaction avec un produit d'oxydation d'un agent développateur ; $R_4$ représente un groupe capable de substituer le noyau benzénique ; n représente un entier de 0 a 2 ; et DI, lorsqu'il est libéré, représente un inhibiteur de développement ou son précurseur, lorsque n est égal à 2, deux groupes $R_4$ peuvent être identiques ou différents, et lorsque deux des substituants $R_1$, $R_2$ et $R_4$ sont présents sur des positions voisines, ils peuvent être des groupes divalents et reliés entre eux pour former une structure cyclique.

2. Un matériau photographique à l'halogénure d'argent selon la revendication 1, dans lequel $R_1$ et $R_2$ représentent chacun un atome d'hydrogène.

3. Un matériau photographique a l'halogénure d'argent selon la revendication 1, dans lequel le groupe éliminable par un alcali est le groupe $R_5$-CO – ou le groupe $R_5$O-CO –, dans lesquels $R_5$ représente un groupe aliphatique, un groupe aromatique ou un groupe hétérocyclique.

4. Un matériau photographique à l'halogénure d'argent selon la revendication 1, dans lequel les composés représentés par la formule générale (I) sont des composés représentés par la formule générale (Ia) ou (Ib) suivante :

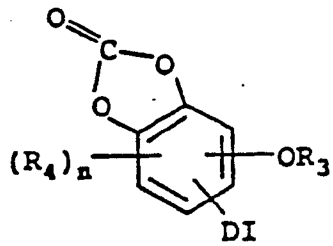

(Ia)

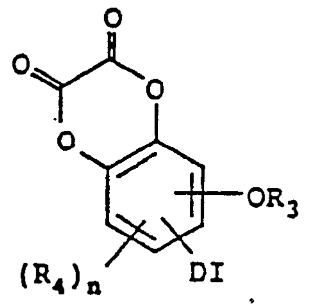

(Ib)

dans lesquelles $R_3$, $R_4$, n et DI ont chacun la même signification que pour la formule générale (I).

5. Un matériau photographique a l'halogénure d'argent selon la revendication 1, dans lequel le groupe représenté par $R_4$

est choisi parmi le groupe $R_6-O-CO-$, le groupe $R_7-N(R_8)-CO-$, le groupe $R_6-$, le groupe $R_7-CO-$, le groupe $R_6-S-$, un atome d'halogène, le groupe $R_6-SO_2-$, le groupe $R_7-N(R_8)-SO_2-$ et un groupe nitro, dans

lesquels $R_6$ représente un groupe[8] aliphatique, un groupe aromatique ou un groupe hétérocyclique ; et $R_7$ et $R_8$ représentent chacun un atome d'hydrogène, un groupe aliphatique, un groupe aromatique ou un groupe hétérocyclique.

6. Un matériau photographique a l'halogénure d'argent selon la revendication 1, dans lequel le groupe aromatique représenté par $R_3$ est un groupe aromatique substitué ou non en $C_6$-$C_{20}$.

7. Un matériau photographique a l'halogénure d'argent selon la revendication 1, dans lequel le groupe hétérocyclique représenté par $R_3$ est un groupe hétérocyclique substitué ou non contenant au moins un hétéroatome choisi parmi un atome d'azote, un atome d'oxygène et un atome de soufre.

8. Un matériau photographique a l'halogénure d'argent selon la revendication 3, dans lequel le groupe aliphatique représenté par $R_5$ est un groupe hydrocarboné aliphatique saturé ou insaturé, a chaîne droite ou ramifiée ou cyclique, substitué ou non en $C_1$-$C_{40}$.

9. Un matériau photographique a l'halogénure d'argent selon la revendication 3, dans lequel le groupe aromatique représenté par $R_5$ est un groupe aromatique substitué ou non en $C_6$-$C_{20}$.

10. Un matériau photographique à l'halogénure d'argent selon la revendication 3, dans le groupe hétérocyclique représenté par $R_5$ es un groupe hétérocyclique substitué ou non ayant au moins un hétéroatome choisi parmi un atome d'azote, un atome d'oxygène et un atome de soufre.

11. Un matériau photographique à l'halogénure d'argent selon la revendication 5, dans lequel le groupe aliphatique représenté par $R_6$, $R_7$ ou $R_8$ est un groupe hydrocarboné aliphatique saturé ou insaturé, à chaîne droite, ramifiée ou cyclique, substitué ou non, en $C_1$-$C_{40}$.

12. Un matériau photographique à l'halogénure d'argent selon la revendication 5, dans lequel le groupe aromatique représenté par $R_6$, $R_7$ ou $R_8$ est un groupe aromatique substitué ou non en $C_6$-$C_{20}$.

13. Un matériau photographique a l'halogénure d'argent selon la revendication 5, dans lequel le groupe hétérocyclique représenté par $R_6$, $R_7$ ou $R_8$ est un groupe hétérocyclique substitué ou non contenant au moins un hétéroatome choisi parmi un atome d'azote, un atome d'oxygène et un atome de soufre.

14. Un matériau photographique a l'halogénure d'argent selon la revendication 1, dans lequel l'inhibiteur de développement représenté par DI est choisi parmi un groupe tétrazolylthio, un groupe thiazolylthio, un

groupe benzothiadiazolylthio, un groupe benzoxazolylthio, un groupe benzotriazolyle, un groupe indazolyle, un groupe benzimidazolylthio, un groupe triazolylthio, un groupe thiadiazolylthio, un groupe triazolyle substitué par un groupe thioéther et un groupe oxadiazolyle.

15. Un matériau photographique a l'halogénure d'argent selon la revendication 1, dans lequel le précurseur de l'inhibiteur de développement représenté par DI est un groupe représenté par la formule suivante :

$$* - TIME - DI'$$

dans laquelle le symbole * désigne la position dans laquelle le groupe est relié au résidu du composé représenté par la formule générale (I) ; TIME représente un groupe capable d'éliminer DI' après avoir été éliminé sous la forme TIME-DI' ; et DI' représente un inhibiteur de développement qui est le même que l'inhibiteur de développement défini pour DI.

16. Un matériau photographique a l'halogénure d'argent selon la revendication 1, dans lequel le composé représenté par la formule générale (I) est représenté par la formule générale (II) suivante :

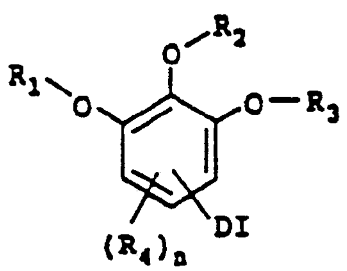

(II)

dans laquelle $R_1$, $R_2$, $R_3$, $R_4$, n et DI ont chacun la même signification que dans la formule générale (I).

17. Un matériau photographique a l'halogénure d'argent selon la revendication 1, dans lequel le matériau photographique a l'halogénure d'argent contient en outre un coupleur.

18. Un matériau photographique à l'halogénure d'argent selon la revendication 17, dans lequel le coupleur est choisi parmi un coupleur pour jaune, un coupleur pour magenta, un coupleur pour cyan et un coupleur non chromogène.

19. Un matériau photographique a l'halogénure d'argent selon la revendication 17, dans lequel le coupleur est choisi parmi un coupleur du type acylacétamide, un coupleur du type malonodiamide, un coupleur du type 5-pyrazolone, un coupleur du type pyrazoloimidazole, un coupleur du type pyrazolotriazole, un coupleur du type phénol et un coupleur du type naphtol.

20. Un matériau photographique à l'halogénure d'argent selon la revendication 17, dans lequel le coupleur est représenté par l'une des formules générales (Cp-1), (Cp-2), (Cp-3), (Cp-4), (Cp-5), (Cp-6), Cp-7) et (Cp-8) suivantes :

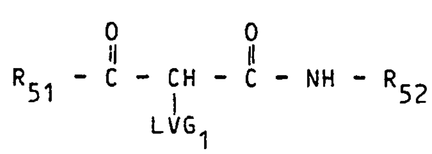

(Cp-1)

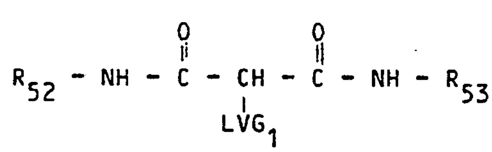

(Cp-2)

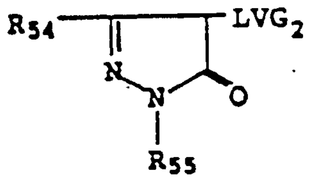

(Cp-3)

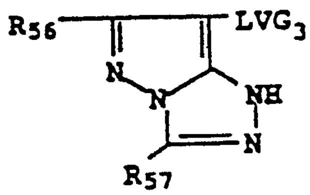

(Cp-4)

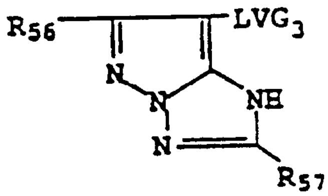

(Cp-5)

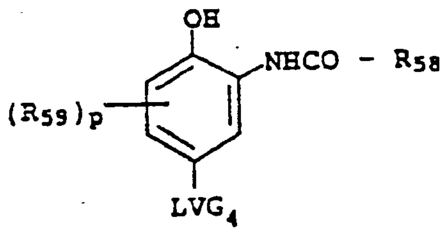

(Cp-6)

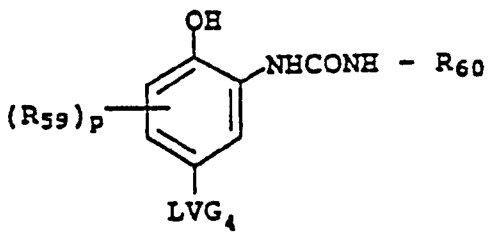

(Cp-7)

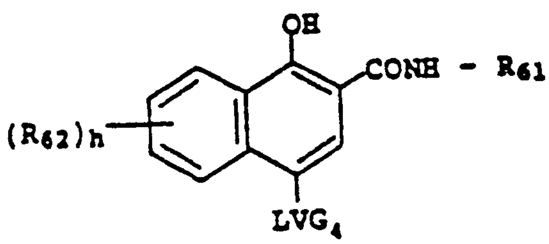

(Cp-8)

dans lesquelles $LVG_1$, $LVG_2$, $LVG_3$ et $LVG_4$ représentent chacun un groupe éliminable dans la copulation ou un atome d'hydrogène ; $R_{41}$ représente un groupe aliphatique, un groupe aromatique ou un groupe hétérocyclique ; $R_{42}$ représente un groupe aromatique ou un groupe hétérocyclique ; et $R_{43}$, $R_{44}$ et $R_{45}$ représentent chacun un atome d'hydrogène, un groupe aliphatique, un groupe aromatique ou un groupe hétérocyclique ;

$R_{51}$ représente un groupe tel que défini pour $R_{41}$ ;

$R_{52}$ et $R_{53}$ représentent chacun un groupe tel que défini pour $R_{42}$ ;

$R_{54}$ représente un groupe tel que défini pour $R_{41}$, le groupe $R_{41}CO\underset{R_{43}}{\underset{|}{N}}-$, le groupe $R_{41}\underset{R_{43}}{\underset{|}{N}}-$, le groupe $R_{41}SO_2\underset{R_{43}}{\underset{|}{N}}-$, le groupe $R_{41}S-$, le groupe $R_{43}O-$, le groupe $R_{45}\underset{R_{43}}{\underset{|}{N}}CO\underset{R_{44}}{\underset{|}{N}}-$, le groupe $R_{41}OOC-$, le groupe $R_{44}\underset{R_{43}}{\underset{|}{N}}CO-$ ou le groupe $N \equiv C-$;

$R_{55}$ représente un groupe tel que défini pour $R_{41}$ ;

$R_{56}$ et $R_{57}$ représentent chacun un groupe tel que défini pour $R_{43}$, le groupe $R_{41}S-$, le groupe $R_{43}O-$, le groupe $R_{41}CO\underset{R_{43}}{N}-$, le groupe $R_{41}\underset{R_{43}}{N}-$, le groupe $R_{41}OCO\underset{R_{43}}{N}-$, le groupe $R_{41}O-$, le groupe $R_{43}\underset{R_{44}}{N}CO\underset{R_{45}}{N}-$ ou le groupe $R_{41}SO_2\underset{R_{43}}{N}-$ ;

$R_{58}$ représente un groupe tel que défini pour $R_{41}$ ;

$R_{59}$ représente un groupe tel que défini pour $R_{41}$, le groupe $R_{41}CO\underset{R_{43}}{N}-$, le groupe $R_{41}OCO\underset{R_{43}}{N}-$, le groupe $R_{43}SO_2\underset{R_{43}}{N}-$, le groupe $R_{43}\underset{R_{44}}{N}CO\underset{R_{45}}{N}-$, le groupe $R_{43}\underset{R_{44}}{N}SO_2\underset{R_{45}}{N}-$, le groupe $R_{41}O-$, le groupe $R_{41}S-$, un atome d'halogène ou le groupe $R_{41}\underset{R_{43}}{N}-$ ;

p représente un entier de 0 à 3, lorsque p est égal à 2 ou plus, deux restes $R_{59}$ ou plus peuvent être identiques ou différents, ou bien deux restes $R_{59}$ peuvent être chacun un groupe divalent et être reliés entre eux pour former une structure cyclique ;

$R_{60}$ représente un groupe tel que défini pour $R_{41}$ ;

$R_{61}$ représente un groupe tel que défini pour $R_{41}$ ;

$R_{62}$ représente un groupe tel que défini pour $R_{41}$, le groupe $R_{41}CONH-$, le groupe $R_{41}OCONH-$, le groupe $R_{41}SO_2NH-$, le groupe $R_{43}\underset{R_{44}}{N}CO\underset{R_{45}}{N}-$, le groupe $R_{43}\underset{R_{44}}{N}SO_2\underset{R_{45}}{N}-$, le groupe $R_{43}O-$, le groupe $R_{41}S-$, un atome d'halogène ou le groupe $R_{41}\underset{R_{43}}{N}-$ ; et

h représente un entier de 0 à 4, lorsque h est égal à 2 ou plus, deux restes $R_{62}$ ou plus peuvent être identiques ou différents.

21. Un matériau photographique à l'halogénure d'argent selon la revendication 20, dans lequel le groupe aliphatique, le groupe aromatique ou le groupe hétérocyclique a au moins un substituant choisi parmi un atome d'halogène, le groupe $R_{47}O-$, le groupe

$R_{46}S-$, le groupe $R_{47}CO\underset{R_{48}}{N}-$, le groupe $R_{47}\underset{R_{48}}{N}CO-$, le groupe $R_{46}OCO\underset{R_{47}}{N}-$, le

groupe $R_{46}SO_2N(R_{47})-$, le groupe 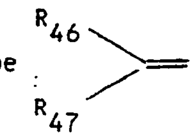 le groupe $R_{47}NSO_2-$ ($R_{48}$), le groupe $R_{46}SO_2-$, le groupe $R_{47}OCO-$, le groupe $R_{47}NCON-$ ($R_{48}$, $R_{49}$), le groupe 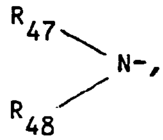 un groupe tel que défini pour $R_{46}$, le groupe 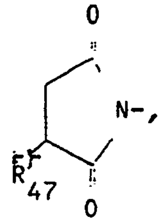 le groupe $R_{46}COO-$, le groupe $R_{47}OSO_2-$, un groupe cyano ou un groupe nitro, dans lesquels $R_{46}$ représente un groupe aliphatique, un groupe aromatique groupe hétérocyclique ; et $R_{47}$, $R_{48}$ et $R_{49}$ représentent chacun un atome d'hydrogène, un groupe aliph un groupe aromatique ou un groupe hétérocyclique.

22. Un matériau photographique à l'halogénure d'argent selon la revendication 20, dans lequel $R_{51}$ sente un groupe aliphatique ou un groupe aromatique ; $R_{52}$, $R_{53}$ et $R_{55}$ représentent chacun un groupe tique ; $R_{54}$ représente le groupe $R_{41}$ CONH – ou le groupe

$$R_{41}-N(R_{43})- \;;$$

$R_{56}$ et $R_{57}$ représentent chacun un groupe aliphatique, le groupe $R_{41}O$ – ou le groupe $R_{41}S$ – ;

$R_{58}$ représente un groupe aliphatique ou un groupe aromatique ;

$R_{59}$ dans la formule générale (Cp-6) représente un atome de chlore, un groupe aliphatique ou le $R_{41}CONH$ – ;

p dans la formule générale (Cp-6) représente 1 ou 2 ;

$R_{60}$ représente un groupe aromatique ;

$R_{59}$ dans la formule générale (Cp-7) représente le groupe $R_{41}CONH$ – ;

p dans la formule générale (Cp-7) représente 1 ;

$R_{61}$ représente un groupe aliphatique ou un groupe aromatique ;

h dans la formule générale (Cp-8) représente 0 ou 1 ; et

$R_{62}$ représente le groupe $R_{41}OCONH$ –, le groupe $R_{41}CONH$ – ou le groupe $R_{41}SO_2NH$ –.

23. Matériau photographique à l'halogénure d'argent selon la revendication 20, dans laquelle $LVG_1$ sente le groupe $R_{65}O$ –, un groupe imido qui est relié par son atome d'azote a la position de copulat groupe hétérocyclique azoté insaturé qui est relié par son atome d'azote à la position de copulation ou le $R_{66}S$ –, dans lesquels $R_{65}$ représente un groupe aromatique ou un groupe hétérocyclique et $R_{66}$ représe groupe aliphatique, un groupe aromatique ou un groupe hétérocyclique.

24. Un matériau photographique à l'halogénure d'argent selon la revendication 20, dans lequel $LVG_2$ sente le groupe $R_{66}S$ –, un groupe hétérocyclique azoté insaturé qui est relié par son atome d'azote à la p de copulation, le groupe $R_{65}O$ – ou un atome d'hydrogène, dans lesquels $R_{65}$ représente un groupe arom ou un groupe hétérocyclique et $R_{66}$ représente un groupe aliphatique, un groupe aromatique ou un group rocyclique.

25. Un matériau photographique à l'halogénure d'argent selon la revendication 20, dans lequel $LVG_3$ sente un atome d'halogène, le groupe $R_{66}S$ – ou un groupe hétérocyclique azoté insaturé qui est relié p atome d'azote à la position de copulation.